# FASCICULE DE BREVET EUROPEEN

(11) **EP 1 001 942 B1**
(45) Date de publication et mention de la délivrance du brevet: **25.06.2003**
(21) Numéro de dépôt: 98939677.5
(22) Date de dépôt: 15.07.1998
(51) Int. Cl.: C07D 241/12, A61K 31/495, C07D 405/14

(54) **POLYHYDROXYBUTYLPYRAZINES, LEUR PREPARATION ET MEDICAMENTS LES CONTENANT**
POLYHYDROXYBUTYLPYRAZINEN, DEREN HERSTELLUNG UND DIESE ENTHALTENDE ARZNEIMITTELN
POLYHYDROXYBUTYLPYRAZINES, THEIR PREPARATION AND MEDICINES CONTAINING THEM

(30) Priorité: 17.07.1997 FR 9709059
(43) Date de publication de la demande: 24.05.2000
(73) Titulaire: Aventis Pharma S.A., 92160 Antony (FR)
(72) Inventeur: BASHIARDES, Georges, F-94320 Thiais (FR); CARRY, Jean-Christophe, F-92190 Meudon (FR); EVERS, Michel, F-94510 La Queue-en-Brie (FR); FILOCHE, Bruno, F-94000 Créteil (FR); MIGNANI, Serge, F-92290 Châtenay-Malabry (FR)
(86) Numéro de dépôt international: FR9801543
(87) Numéro de publication internationale: WO99003841

(56) Documents cités:
- WO-A-97/28813
- CHEMICAL ABSTRACTS, vol. 121, no. 1, 1994 Columbus, Ohio, US; abstract no. 9331h, AN RONG LEE: "SYNTHESIS A. PHARMACOL. EFFECTS OF TETRAMETHYLPYRAZINE DERIVATIVES." page 990; colonne 1; XP002060215 & YIXUE YANJIU, vol. 13, no. 1, 1992, pages 41-50, TAIWAN
- CHEMICAL ABSTRACTS, vol. 90, no. 11, 1979 Columbus, Ohio, US; abstract no. 19276z, page 329; XP002060269 & JP 07 890401 A (JAPAN TOBACCO)
- S.EITELMAN: "DECOMPOSITION REACTIONS OF AMINO SUGARS" CARBOHYDRATE RESEARCH., vol. 77, 1979, pages 205-211, XP002082944 AMSTERDAM NL cité dans la demande

## Description

La présente invention concerne les médicaments contenant en tant que principe actif au moins un composé de formule : 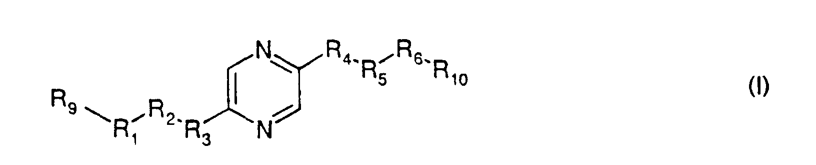 ou un de ses stéréoisomères ou un de ses sels, les nouveaux composés de formule (I), leurs stéréoisomères et leurs sels et leur procédé de préparation.

Dans la formule (I)
R₉ et R₁₀ représentent chacun un radical -CH₂OH, et soit
a) R₃ représente un radical méthylène, R₄ représente un radical -CHOH- et l'un des radicaux R₁, R₂, R₅ et R₆ représente un radical carbonyle, méthylène, -CHF-, -CH(NHR₇)- ou -CH(OR₈) et les autres représentent chacun un radical -CHOH-,
b) R₂ et R₅ représentent chacun un radical -CHOH-, R₃ représente un radical méthylène et R₁, R₄ et R₆ sont identiques et représentent chacun un radical carbonyle, méthylène, -CHF-, -CH(NHR₇)- ou -CH(OR₈),
c) R₁ et R₆ représentent chacun un radical -CHOH-, R₃ représente un radical méthylène et R₂, R₄ et R₅ sont identiques et représentent chacun un radical carbonyle, méthylène, -CHF-, -CH(NHR₇)- ou -CH(OR₈),
d) R₁, R₄, R₅ et R₆ représentent chacun un radical -CHOH- et -R₂-R₃-représente un radical -CH=CH-,
R₇ représente un atome d'hydrogène ou un radical alkyle, -CO-alk, -CO-Ar ou -CO-Het,
R₈ représente un radical alkyle, -alk-COOH ou -alk-OH,
alk représente un radical alkyle,
Ar représente un radical phényle ou phényle substitué par un ou plusieurs substituants choisis parmi un atome d'halogène, un radical alkyle, alcoxy, alcoxycarbonyle, amino, monoalkylamino ou dialkylamino,
Het représente un hétérocycle mono, di ou tricyclique saturé ou insaturé contenant 1 à 9 atomes de carbone et un ou plusieurs hétéroatomes choisis parmi oxygène, soufre et azote.

Dans les définitions précédentes et celles qui suivent, les radicaux et portions alkyle et alcoxy contiennent 1 à 6 atomes de carbone en chaîne droite ou ramifiée et les atomes d'halogène sont les atomes de chlore, fluor, iode et brome.

Les composés de formule (I) comportant plusieurs carbones asymétriques, présentent des formes stéréoisomères. Ces différents stéréoisomères font partie de l'invention. En outre les composés pour lesquels -R₂-R₃- représente un radical -CH=CH- peuvent se présenter sous forme cis ou trans; ces formes font également partie de l'invention.

De préférence Het représente un hétérocycle choisi parmi les cycles 2-, 3-, ou 4-pyridyle, imidazolyle, thiazolyle et oxazolyle.

Autrement dit, les composés de formule (I) répondent aux formules suivantes: 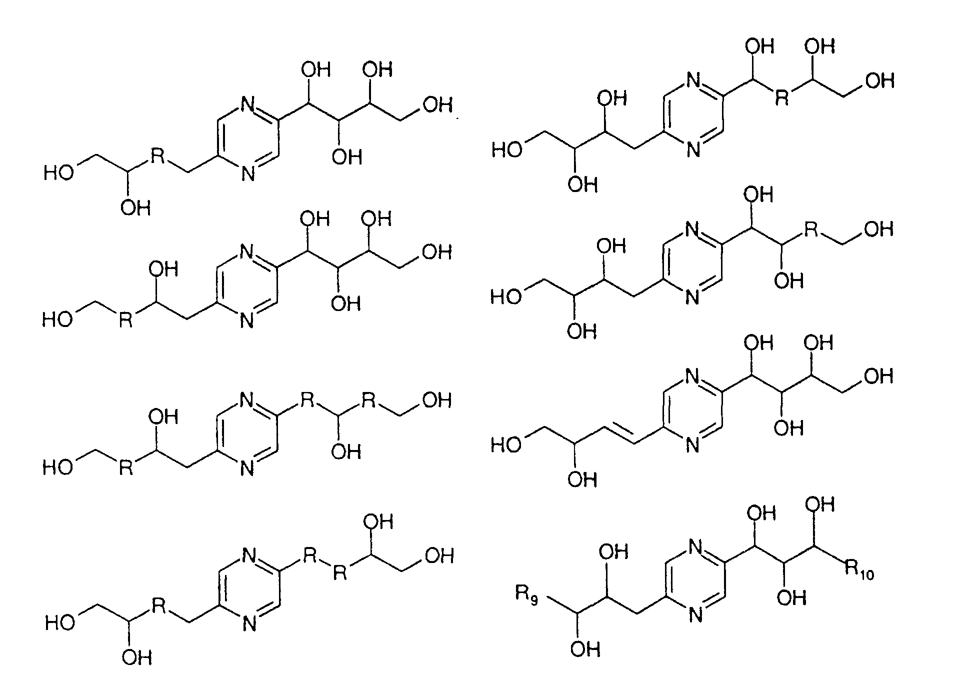 dans lesquelles R représente représente un radical carbonyle, méthylène, -CHF-, -CH(NHR₇)- ou -CH(OR₈), R₇, R₈, R₉ et R₁₀ ayant les mêmes significations que précédemment, leurs formes stéréoisomères et les formes cis et trans des composés comportant une chaîne CH=CH.

Les médicaments préférés sont ceux qui contiennent en tant que principe actif au moins un composé de formule (I) choisi parmi les composés suivants:
1-[5-(3R,4-Dihydroxy-2-oxo-butyl)-pyrazin-2-yl]-butane-1R,2S,3R,4-tétraol,
1-[5-(3S,4-Dihydroxy-butyl)-pyrazin-2-yl]-butane-1R,2S,3R,4-tétraol,
1-[5-(2S-Fluoro-3R,4-dihydroxy-butyl)-pyrazin-2-yl]-butane-1R,2S,3R,4-tétraol,
1-[5-(2R-Fluoro-3R,4-dihydroxy-butyl)-pyrazin-2-yl]-butane-1R,2S,3R,4-tétraol,
1-[5-(2S-Amino-3S,4-dihydroxy-butyl)-pyrazin-2-yl]-butane-1R,2S,3R,4-tétraol,
1-[5-(2R-Amino-3S,4-dihydroxy-butyl)-pyrazin-2-yl]-butane-1R,2S,3R,4-tétraol,
1-[5-(2S-(N-Méthyl)amino-3S,4-dihydroxy-butyl)-pyrazin-2-yl]-butane-1R,2S,3R,4-tétraol,
1-[5-(2R-(N-Méthyl)amino-3S,4-dihydroxy-butyl)-pyrazin-2-yl]-butane-1R,2S,3R,4-tétraol,
1-[5-(2S-(N-Ethyl)amino-3S,4-dihydroxy-butyl)-pyrazin-2-yl]-butane-1R,2S,3R,4-tétraol,
1-[5-(2R-(N-Ethyl)amino-3S,4-dihydroxy-butyl)-pyrazin-2-yl]-butane-1R,2S,3R,4-tétraol,
1-[5-(2S-(N-n-Butyl)amino-3S,4-dihydroxy-butyl)-pyrazin-2-yl]-butane-1R,2S,3R,4-tétraol,
1-[5-(2R-(N-n-Butyl)amino-3S,4-dihydroxy-butyl)-pyrazin-2-yl]-butane-1R,2S,3R,4-tétraol,
1-[5-(2S-(N-Benzyl)amino-3S,4-dihydroxy-butyl)-pyrazin-2-yl]-butane-1R,2S,3R,4-tétraol,
1-[5-(2R-(N-Benzyl)amino-3S,4-dihydroxy-butyl)-pyrazin-2-yl]-butane-1R,2S,3R,4-tétraol,
1-[5-(2S-(N-Acétyl)amino-3S,4-dihydroxy-butyl)-pyrazin-2-yl]-butane-1R,2S,3R,4-tétraol,
1-[5-(2R-(N-Acétyl)amino-3S,4-dihydroxy-butyl)-pyrazin-2-yl]-butane-1R,2S,3R,4-tétraol,
1-[5-(2S-(N-Butanoyl)amino-3S,4-dihydroxy-butyl)-pyrazin-2-yl]-butane-1R,2S,3R,4-tétraol,
1-[5-(2R-(N-Butanoyl)amino-3S,4-dihydroxy-butyl)-pyrazin-2-yl]-butane-1R,2S,3R,4-tétraol,
1-[5-(2S-(N-Benzoyl)amino-3S,4-dihydroxy-butyl)-pyrazin-2-yl]-butane-1R,2S,3R,4-tétraol,
1-[5-(2R-(N-Benzoyl)amino-3S,4-dihydroxy-butyl)-pyrazin-2-yl]-butane-1R,2S,3R,4-tétraol,
1-[5-(2S-Méthoxy-3R,4-dihydroxy-butyl)-pyrazin-2-yl]-butane-1R,2S,3R,4-tétraol,
1-[5-(2R-Méthoxy-3R,4-dihydroxy-butyl)-pyrazin-2-yl]-butane-1R,2S,3R,4-tétraol,
1-[5-(2S-Ethoxy-3R,4-dihydroxy-butyl)-pyrazin-2-yl]-butane-1R,2S,3R,4-tétraol,
1-[5-(2R-Ethoxy-3R,4-dihydroxy-butyl)-pyrazin-2-yl]-butane-1R,2S,3R,4-tétraol,
7-[5-(2S-n-Butoxy-3S,4-dihydroxy-butyl)-pyrazin-2-yl]-butane-1R,2S,3R,4-tétraol,
1-[5-(2R-n-Butoxy-3R,4-dihydroxy-butyl)-pyrazin-2-yl]-butane-1R,2S,3R,4-tétraol,
1-[5-(2S-(2-Hydroxyéthyl)oxy-3R,4-dihydroxy-butyl)-pyrazin-2-yl]-butane-1R,2S,3R,4-tétraol,
1-[5-(2R-(2-Hydroxyéthyl)oxy-3R,4-dihydroxy-butyl)-pyrazin-2-yl]-butane-1R,2S,3R,4-tétraol,
1-[5-(2S-(3-Hydroxy-n-propyl)oxy-3R,4-dihydroxy-butyl)-pyrazin-2-yl]-butane-1R,2S,3R,4-tétraol,
1-[5-(2R-(3-Hydroxy-n-propyl)oxy-3R,4-dihydroxy-butyl)-pyrazin-2-yl]-butane-1R,2S,3R,4-tétraol,
1-[5-(2S-(Carboxyméthyl)oxy-3R,4-dihydroxy-butyl)-pyrazin-2-yl]-butane-1R,2S,3R,4-tétraol,
1-[5-(2R-(Carboxyméthyl)oxy-3R,4-dihydroxy-butyl)-pyrazin-2-yl]-butane-1R,2S,3R,4-tétraol,
1-[5-(2S-(3-Carboxy-n-propyl)oxy-3R,4-dihydroxy-butyl)-pyrazin-2-yl]-butane-1R,2S,3R,4-tétraol,
1-[5-(2R-(3-Carboxy-n-propyl)oxy-3R,4-dihydroxy-butyl)-pyrazin-2-yl]-butane-1R,2S,3R,4-tétraol,
1-[5-(2S,4-Dihydroxy-3-oxo-butyl)-pyrazin-2-yl]-butane-1R,2S,3R,4-tétraol,
1-[5-(2S,4-Dihydroxy-butyl)-pyrazin-2-yl]-butane-1R,2S,3R,4-tétraol,
1-[5-(3R-Fluoro-2S,4-dihydroxy-butyl)-pyrazin-2-yl]-butane-1R,2S,3R,4-tétraol,
1-[5-(3S-Fluoro-2S,4-dihydroxy-butyl)-pyrazin-2-yl]-butane-1R,2S,3R,4-tétraol,
1-[5-(3R-Amino-2R,4-dihydroxy-butyl)-pyrazin-2-yl]-butane-1R,2S,3R,4-tétraol,
1-[5-(3S-Amino-2R,4-dihydroxy-butyl)-pyrazin-2-yl]-butane-1R,2S,3R,4-tétraol,
1-[5-(3R-(N-Méthyl)amino-2R,4-dihydroxy-butyl)-pyrazin-2-yl]-butane-1R,2S,3R,4-tétraol,
1-[5-(3S-(N-Méthyl)amino-2R,4-dihydroxy-butyl)-pyrazin-2-yl]-butane-1R,2S,3R,4-tétraol,
1-[5-(3R-(N-Ethyl)amino-2R,4-dihydroxy-butyl)-pyrazin-2-yl]-butane-1R,2S,3R,4-tétraol,
1-[5-(3S-(N-Ethyl)amino-2R,4-dihydroxy-butyl)-pyrazin-2-yl]-butane-1R,2S,3R,4-tétraol,
1-[5-(3R-(N-n-Butyl)amino-2R,4-dihydroxy-butyl)-pyrazin-2-yl]-butane-1R,2S,3R,4-tétraol,
1-[5-(3S-(N-n-Butyl)amino-2R,4-dihydroxy-butyl)-pyrazin-2-yl]-butane-1R,2S,3R,4-tétraol,
1-[5-(3R-(N-Benzyl)amino-2R,4-dihydroxy-butyl)-pyrazin-2-yl]-butane-1R,2S,3R,4-tétraol,
1-[5-(3S-(N-Benzyl)amino-2R,4-dihydroxy-butyl)-pyrazin-2-yl]-butane-1R,2S,3R,4-tétraol,
1-[5-(3R-(N-Acétyl)amino-2R,4-dihydroxy-butyl)-pyrazin-2-yl]-butane-1R,2S,3R,4-tétraol,
1-[5-(3S-(N-Acétyl)amino-2R,4-dihydroxy-butyl)-pyrazin-2-yl]-butane-1R,2S,3R,4-tétraol,
1-[5-(3R-(N-Butanoyl)amino-2R,4-dihydroxy-butyl)-pyrazin-2-yl]-butane-1R,2S,3R,4-tétraol,
1-[5-(3S-(N-Butanoyl)amino-2R,4-dihydroxy-butyl)-pyrazin-2-yl]-butane-1R,2S,3R,4-tétraol,
1-[5-(3R-(N-Benzoyl)amino-2R,4-dihydroxy-butyl)-pyrazin-2-yl]-butane-1R,2S,3R,4-tétraol,
1-[5-(3S-(N-Benzoyl)amino-2R,4-dihydroxy-butyl)-pyrazin-2-yl]-butane-1R,2S,3R,4-tétraol,
1-[5-(3R-Méthoxy-2S,4-dihydroxy-butyl)-pyrazin-2-yl]-butane-1R,2S,3R,4-tétraol,
1-[5-(3S-Méthoxy-2S,4-dihydroxy-butyl)-pyrazin-2-yl]-butane-1R,2S,3R,4-tétraol,
1-[5-(3R-Ethoxy-2S,4-dihydroxy-butyl)-pyrazin-2-yl]-butane-1R,2S,3R,4-tétraol,
1-[5-(3S-Ethoxy-2S,4-dihydroxy-butyl)-pyrazin-2-yl]-butane-1R,2S,3R,4-tétraol,
1-[5-(3R-n-Butoxy-2S,4-dihydroxy-butyl)-pyrazin-2-yl]-butane-1R,2S,3R,4-tétraol,
1-[5-(3S-n-Butoxy-2S,4-dihydroxy-butyl)-pyrazin-2-yl]-butane-1R,2S,3R,4-tétraol,
1-[5-(3R-(2-Hydroxyéthyl)oxy-2S,4-dihydroxy-butyl)-pyrazin-2-yl]-butane-1R,2S,3R,4-tétraol,
1-[5-(3S-(2-Hydroxyéthyl)oxy-2S,4-dihydroxy-butyl)-pyrazin-2-yl]-butane-1R,2S,3R,4-tétraol,
1-[5-(3R-(3-Hydroxy-n-propyl)oxy-2S,4-dihydroxy-butyl)-pyrazin-2-yl]-butane-1R,2S,3R,4-tétraol,
1-[5-(3S-(3-Hydroxy-n-propyl)oxy-2S,4-dihydroxy-butyl)-pyrazin-2-yl]-butane-1R,2S,3R,4-tétraol,
1-[5-(3R-(Carboxyméthyl)oxy-2S,4-dihydroxy-butyl)-pyrazin-2-yl]-butane-1R,2S,3R,4-tétraol,
1-[5-(3S-(Carboxyméthyl)oxy-2S,4-dihydroxy-butyl)-pyrazin-2-yl]-butane-1R,2S,3R,4-tétraol,
1-[5-(3R-(3-Carboxy-n-propyl)oxy-2S,4-dihydroxy-butyl)-pyrazin-2-yl]-butane-1R,2S,3R,4-tétraol,
1-[5-(3S-(3-Carboxy-n-propyl)oxy-2S,4-dihydroxy-butyl)-pyrazin-2-yl]-butane-1R,2S,3R,4-tétraol,
4-[5-(2S,3R,4-Trihydroxy-butyl)-pyrazin-2-yl]-1,3S,4R-trihydroxy-butane-2-one,
1-[5-(2S,3R,4-Trihydroxy-butyl)-pyrazin-2-yl]-butane-1R,2R,4-triol,
1-[5-(2S,3R,4-Trihydroxy-butyl)-pyrazin-2-yl]-3R-fluoro-butane-1R,2S,4-triol,
1-[5-(2S,3R,4-Trihydroxy-butys)-pyrazin-2-yl]-3S-fluoro-butane-1R,2S,4-triol,
1-[5-(2S,3R,4-Trihydroxy-butyl)-pyrazin-2-yl]-3R-amino-butane-1R,2R,4-triol,
1-[5-(2S,3R,4-Trihydroxy-butyl)-pyrazin-2-yl]-3S-amino-butane-1R,2R,4-triol,
1-[5-(2S,3R,4-Trihydroxy-butyl)-pyrazin-2-yl]-3R-(N-méthyl)amino-butane-1R,2R,4-triol,
1-[5-(2S,3R,4-Trihydroxy-butyl)-pyrazin-2-yl]-3S-(N-méthyl)amino-butane-1R,2R,4-triol,
1-[5-(2S,3R,4-Trihydroxy-butyl)-pyrazin-2-yl]-3R-(N-éthyl)amino-butane-1R,2R,4-triol,
1-[5-(2S,3R,4-Trihydroxy-butyl)-pyrazin-2-yl]-3S-(N-éthyl)amino-butane-1R,2R,4-triol,
1-[5-(2S,3R,4-Trihydroxy-butyl)-pyrazin-2-yl]-3R-(N-n-butyl)amino-butane-1R,2R,4-triol,
1-[5-(2S,3R,4-Trihydroxy-butyl)-pyrazin-2-yl]-3S-(N-n-butyl)amino-butane-1R,2R,4-triol,
1-[5-(2S,3R,4-Trihydroxy-butyl)-pyrazin-2-yl]-3R-(N-benzyl)amino-butane-1R,2R,4-triol,
1-[5-(2S,3R,4-Trihydroxy-butyl)-pyrazin-2-yl]-3S-(N-benzyl)amino-butane-1R,2R,4-triol,
1-[5-(2S,3R,4-Trihydroxy-butyl)-pyrazin-2-yl]-3R-(N-acétyl)amino-butane-1R,2R,4-triol,
1-[5-(2S,3R,4-Trihydroxy-butyl)-pyrazin-2-yl]-3S-(N-acétyl)amino-butane-1R,2R,4-triol,
1-[5-(2S,3R,4-Trihydroxy-butyl)-pyrazin-2-yl]-3R-(N-Butanoyl)amino-butane-1R,2R,4-triol,
1-[5-(2S,3R,4-Trihydroxy-butyl)-pyrazin-2-yl]-3S-(N-Butanoyl)amino-butane-1R,2R,4-triol,
1-[5-(2S,3R,4-Trihydroxy-butyl)-pyrazin-2-yl]-3R-(N-Benzoyl)amino-butane-1R,2R,4-triol,
1-[5-(2S,3R,4-Trihydroxy-butyl)-pyrazin-2-yl]-3S-(N-Benzoyl)amino-butane-1R,2R,4-triol,
1-[5-(2S,3R,4-Trihydroxy-butyl)-pyrazin-2-yl]-3R-méthoxy-butane-1R,2S,4-triol,
1-[5-(2S,3R,4-Trihydroxy-butyl)-pyrazin-2-yl]-3S-méthoxy-butane-1R,2S,4-triol,
1-[5-(2S,3R,4-Trihydroxy-butyl)-pyrazin-2-yl]-3R-éthoxy-butane-1R,2S,4-triol,
1-[5-(2S,3R,4-Trihydroxy-butyl)-pyrazin-2-yl]-3S-éthoxy-butane-1R,2S,4-triol,
1-[5-(2S,3R,4-Trihydroxy-butyl)-pyrazin-2-yl]-3R-n-butoxy-butane-1R,2S,4-triol,
1-[5-(2S,3R,4-Trihydroxy-butyl)-pyrazin-2-yl]-3S-n-butoxy-butane-1R,2S,4-triol,
1-[5-(2S,3R,4-Trihydroxy-butyl)-pyrazin-2-yl]-3R-(2-hydroxyéthyl)oxy-butane-1R,2S,4-triol,
1-[5-(2S,3R,4-Trihydroxy-butyl)-pyrazin-2-yl]-3S-(2-hydroxyéthyl)oxy-butane-1R,2S,4-triol,
1-[5-(2S,3R,4-Trihydroxy-butyl)-pyrazin-2-yl]-3R-(3-hydroxy-n-propyl)oxy-butane-1R,2S,4-triol,
1-[5-(2S,3R,4-Trihydroxy-butyl)-pyrazin-2-yl]-3S-(3-hydroxy-n-propyl)oxy-butane-1 R,2S,4-triol,
1-[5-(2S,3R,4-Trihydroxy-butyl)-pyrazin-2-yl]-3R-(carboxyméthyl)oxy-butane-1R,2S,4-triol,
1-[5-(2S,3R,4-Trihydroxy-butyl)-pyrazin-2-yl]-3S-(carboxyméthyl)oxy-butane-1R,2S,4-triol,
1-[5-(2S,3R,4-Trihydroxy-butyl)-pyrazin-2-yl]-3R-(3-carboxy-n-propyl)oxy-butane-1R,2S,4-triol,
1-[5-(2S,3R,4-Trihydroxy-butyl)-pyrazin-2-yl]-3S-(3-carboxy-n-propyl)oxy-butane-1R,2S,4-triol,
1-[5-(2S,3R,4-Trihydroxy-butyl)-pyrazin-2-yl]-1R,3R,4-trihydroxy-butane-2-one,
1-[5-(2S,3R,4-Trihydroxy-butyl)-pyrazin-2-yl]-butane-1R,3R,4-triol,
1-[5-(2S,3R,4-Trihydroxy-butyl)-pyrazin-2-yl]-2R-fluoro-butane-1R,3R,4-triol,
1-[5-(2S,3R,4-Trihydroxy-butyl)-pyrazin-2-yl]-2S-fluoro-butane-1R,3R,4-triol,
1-[5-(2S,3R,4-Trihydroxy-butyl)-pyrazin-2-yl]-2R-amino-butane-1S,3S,4-triol,
1-[5-(2S,3R,4-Trihydroxy-butyl)-pyrazin-2-yl]-2S-amino-butane-1S,3S,4-triol,
1-[5-(2S,3R,4-Trihydroxy-butyl)-pyrazin-2-yl]-2R-(N-méthyl)amino-butane-1S,3S,4-triol,
1-[5-(2S,3R,4-Trihydroxy-butyl)-pyrazin-2-yl]-2S-(N-méthyl)amino-butane-1S,3S,4-triol,
1-[5-(2S,3R,4-Trihydroxy-butyl)-pyrazin-2-yl]-2R-(N-éthyl)amino-butane-1S,3S,4-triol,
1-[5-(2S,3R,4-Trihydroxy-butyl)-pyrazin-2-yl]-2S-(N-éthyl)amino-butane-1S,3S,4-triol,
1-[5-(2S,3R,4-Trihydroxy-butyl)-pyrazin-2-yl]-2R-(N-n-butyl)amino-butane-1S,3S,4-triol,
1-[5-(2S,3R,4-Trihydroxy-butyl)-pyrazin-2-yl]-2S-(N-n-butyl)amino-butane-1S,3S,4-triol,
1-[5-(2S,3R,4-Trihydroxy-butyl)-pyrazin-2-yl]-2R-(N-benzyl)amino-butane-1S,3S,4-triol,
1-[5-(2S,3R,4-Trihydroxy-butyl)-pyrazin-2-yl]-2S-(N-benzyl)amino-butane-1S,3S,4-triol,
1-[5-(2S,3R,4-Trihydroxy-butyl)-pyrazin-2-yl]-2R-(N-acétyl)amino-butane-1S,3S,4-triol,
1-[5-(2S,3R,4-Trihydroxy-butyl)-pyrazin-2-yl]-2S-(N-acétyl)amino-butane-1S,3S,4-triol,
1-[5-(2S,3R,4-Trihydroxy-butyl)-pyrazin-2-yl]-2R-(N-Butanoyl)amino-butane-1S,3S,4-triol,
1-[5-(2S,3R,4-Trihydroxy-butyl)-pyrazin-2-yl]-2S-(N-Butanoyl)amino-butane-1S,3S,4-triol,
1-[5-(2S,3R,4-Trihydroxy-butyl)-pyrazin-2-yl]-2R-(N-Benzoyl)amino-butane-1S,3S,4-triol,
1-[5-(2S,3R,4-Trihydroxy-butyl)-pyrazin-2-yl]-2S-(N-Benzoyl)amino-butane-1S,3S,4-triol,
1-[5-(2S,3R,4-Trihydroxy-butyl)-pyrazin-2-yl]-2R-méthoxy-butane-1R,3R,4-triol,
1-[5-(2S,3R,4-Trihydroxy-butyl)-pyrazin-2-yl]-2S-méthoxy-butane-1R,3R,4-triol,
1-[5-(2S,3R,4-Trihydroxy-butyl)-pyrazin-2-yl]-2R-éthoxy-butane-1R,3R,4-triol,
1-[5-(2S,3R,4-Trihydroxy-butyl)-pyrazin-2-yl]-2S-éthoxy-butane-1R,3R,4-triol,
1-[5-(2S,3R,4-Trihydroxy-butyl)-pyrazin-2-yl]-2R-n-butoxy-butane-1R,3R,4-triol,
1-[5-(2S,3R,4-Trihydroxy-butyl)-pyrazin-2-yl]-2S-n-butoxy-butane-1R,3R,4-triol,
1-[5-(2S,3R,4-Trihydroxy-butyl)-pyrazin-2-yl]-2R-(2-hydroxyéthyl)oxy-butane-1R,3R,4-triol,
1-[5-(2S,3R,4-Trihydroxy-butyl)-pyrazin-2-yl]-2S-(2-hydroxyéthyl)oxy-butane-1R,3R,4-triol,
1-[5-(2S,3R,4-Trihydroxy-butyl)-pyrazin-2-yl]-2R-(3-hydroxy-n-propyl)oxy-butane-1R,3R,4-triol,
1-[5-(2S,3R,4-Trihydroxy-butyl)-pyrazin-2-yl]-2S-(3-hydroxy-n-propyl)oxy-butane-1R,3R,4-triol,
1-[5-(2S,3R,4-Trihydroxy-butyl)-pyrazin-2-yl]-2R-(carboxyméthyl)oxy-butane-1R,3R,4-triol,
1-[5-(2S,3R,4-Trihydroxy-butyl)-pyrazin-2-yl]-2S-(carboxyméthyl)oxy-butane-1R,3R,4-triol,
1-[5-(2S,3R,4-Trihydroxy-butyl)-pyrazin-2-yl]-2R-(3-carboxy-n-propyl)oxy-butane-1R,3R,4-triol,
1-[5-(2S,3R,4-Trihydroxy-butyl)-pyrazin-2-yl]-2S-(3-carboxy-n-propyl)oxy-butane-1R,3R,4-triol,
1-[5-(2S,4-Dihydroxy-3-oxo-butyl)-pyrazin-2-yl]-2S,4-dihydroxy-butane-1,3-dione,
4-[5-(2S,4-Dihydroxy-butyl)-pyrazin-2-yl]-butane-1,3S-diol,
4-[5-(3R-Fluoro-2S,4-dihydroxy-butyl)-pyrazin-2-yl]-2R,4R-difluoro-butane-1,3S-diol,
4-[5-(3S-Fluoro-2S,4-dihydroxy-butyl)-pyrazin-2-yl]-2S,4S-difluoro-butane-1,3S-diol,
1-[5-(3R-Amino-2S,4-dihydroxy-butyl)-pyrazin-2-yl]-2R,4R-diamino-butane-1,3S-diol,
1-[5-(3S-Amino-2S,4-dihydroxy-butyl)-pyrazin-2-yl]-2S,4S-diamino-butane-1,3S-diol,
1-[5-(3R-(N-Méthyl)amino-2S,4-dihydroxy-butyl)-pyrazin-2-yl]-2R,4R-di-(N-Méthyl)amino-butane-1,3S-diol,
1-[5-(3S-(N-Méthyl)amino-2S,4-dihydroxy-butyl)-pyrazin-2-yl]-2S,4S-di-(N-Méthyl)amino-butane-1,3S-diol,
1-[5-(3R-(N-Ethyl)amino-2S,4-dihydroxy-butyl)-pyrazin-2-yl]-2R,4R-di-(N-éthyl)amino-butane-1,3S-diol,
1-[5-(3S-(N-Ethyl)amino-2S,4-dihydroxy-butyl)-pyrazin-2-yl]-2S,4S-di-(N-éthyl)amino-butane-1,38-diol,
1-[5-(3R-(N-n-Butyl)amino-2S,4-dihydroxy-butyl)-pyrazin-2-yl]-2R,4R-di-(N-n-Butyl)amino-butane-1,3S-diol,
1-[5-(3S-(N-n-Butyl)amino-2S,4-dihydroxy-butyl)-pyrazin-2-yl]-2S,4S-di-(N-n-Butyl)amino-butane-1,3S-diol,
1-[5-(3R-(N-Benzyl)amino-2S,4-dihydroxy-butyl)-pyrazin-2-yl]-2R,4R-di-(N-Benzyl)amino-butane-1,3S-diol,
1-[5-(3S-(N-Benzyl)amino-2S,4-dihydroxy-butyl)-pyrazin-2-yl]-2S,4S-di-(N-Benzyl)amino-butane-1,3S-diol,
1-[5-(3R-(N-Acétyl)amino-2S,4-dihydroxy-butyl)-pyrazin-2-yl]-2R,4R-di-(N-Acétyl)amino-butane-1,3S-diol,
1-[5-(3S-(N-Acétyl)amino-2S,4-dihydroxy-butyl)-pyrazin-2-yl]-2S,4S-di-(N-Acétyl)amino-butane-1,3S-diol,
1-[5-(3R-(N-Butanoyl)amino-2S,4-dihydroxy-butyl)-pyrazin-2-yl]-2R,4R-di-(N-Butanoyl)amino-butane-1,3S-diol,
1-[5-(3S-(N-Butanoyl)amino-2S,4-dihydroxy-butyl)-pyrazin-2-yl]-2S,4S-di-(N-Butanoyl)amino-butane-1,3S-diol,
1-[5-(3R-(N-Benzoyl)amino-2S,4-dihydroxy-butyl)-pyrazin-2-yl]-2R,4R-di-(N-Benzoyl)amino-butane-1,3S-diol,
1-[5-(3S-(N-Benzoyl)amino-2S,4-dihydroxy-butyl)-pyrazin-2-yl]-2S,4S-di-(N-Benzoyl)amino-butane-1,3S-diol,
1-[5-(3R-Méthoxy-2S,4-dihydroxy-butyl)-pyrazin-2-yl]-2R,4R-di-méthoxy-butane-1,3S-diol,
1-[5-(3S-Méthoxy-2S,4-dihydroxy-butyl)-pyrazin-2-yl]-2S,4S-di-méthoxy-butane-1,3S-diol,
1-[5-(3R-Ethoxy-2S,4-dihydroxy-butyl)-pyrazin-2-yl]-2R,4R-di-éthoxy-butane-1,3S-diol,
1-[5-(3S-Ethoxy-2S,4-dihydroxy-butyl)-pyrazin-2-yl]-2S,4S-di-éthoxy-butane-1,3S-diol,
1-[5-(3R-n-Butoxy-2S,4-dihydroxy-butyl)-pyrazin-2-yl]-2R,4R-di-n-butoxy-butane-1,3S-diol,
1-[5-(3S-n-Butoxy-2S,4-dihydroxy-butyl)-pyrazin-2-yl]-2S,4S-di-n-butoxy-butane-1,3S-diol,
1-[5-(3R-(2-Hydroxyéthyl)oxy-2S,4-dihydroxy-butyl)-pyrazin-2-yl]-2R,4R-di-(2-hydroxyéthyl)oxy-butane-1,3S-diol,
1-[5-(3S-(2-Hydroxyéthyl)oxy-2S,4-dihydroxy-butyl)-pyrazin-2-yl]-2S,4S-di-(2-hydroxyéthyl)oxy-butane-1,3S-diol,
1-[5-(3R-(3-Hydroxy-n-propyl)oxy-2S,4-dihydroxy-butyl)-pyrazin-2-yl]-2R,4R-di-(3-hydroxy-n-propyl)oxy-butane-1,3S-diol,
1-[5-(3S-(3-Hydroxy-n-propyl)oxy-2S,4-dihydroxy-butyl)-pyrazin-2-yl]-2S,4S-di-(3-hydroxy-n-propyl)oxy-butane-1,3S-diol,
1-[5-(3R-(Carboxyméthyl)oxy-2S,4-dihydroxy-butyl)-pyrazin-2-yl]-2R,4R-di-(carboxyméthyl)oxy-butane-1,3S-diol,
1-[5-(3S-(Carboxyméthyl)oxy-2S,4-dihydroxy-butyl)-pyrazin-2-yl]-2S,4S-di-(carboxyméthyl)oxy-butane-1,3S-diol,
1-[5-(3R-(3-Carboxy-n-propyl)oxy-2S,4-dihydroxy-butyl)-pyrazin-2-yl]-2R,4R-di-(3-carboxy-n-propyl)oxy-butane-1,3S-diol,
1-[5-(3S-(3-Carboxy-n-propyl)oxy-2S,4-dihydroxy-butyl)-pyrazin-2-yl]-2S,4S-di-(3-carboxy-n-propyl)oxy-butane-1,3S-diol,
1-[5-(3R,4-Dihydroxy-2-oxo-butyl)-pyrazin-2-yl]-3R,4-dihydroxy-butane-1,2-dione,
4-[5-(2S,4-Dihydroxy-butyl)-pyrazin-2-yl]-butane-1,3S-diol,
4-[5-(3R-Fluoro-2S,4-dihydroxy-butyl)-pyrazin-2-yl]-2R,4R-difluoro-butane-1,3S-diol,
4-[5-(3S-Fluoro-2S,4-dihydroxy-butyl)-pyrazin-2-yl]-2S,4S-difluoro-butane-1,3S-diol,
4-[5-(3R-Amino-2S,4-dihydroxy-butyl)-pyrazin-2-yl]-2R,4R-diamino-butane-1,3S-diol,
4-[5-(3S-Amino-2S,4-dihydroxy-butyl)-pyrazin-2-yl]-2S,4S-diamino-butane-1,3S-diol,
4-[5-(3R-(N-Méthyl)amino-2S,4-dihydroxy-butyl)-pyrazin-2-yl]-2R,4R-di-(N-méthyl)amino-butane-1,3S-diol,
4-[5-(3S-(N-Méthyl)amino-2S,4-dihydroxy-butyl)-pyrazin-2-yl]-2S,4S-di-(N-méthyl)amino-butane-1,3S-diol,
4-[5-(3R-(N-Ethyl)amino-2S,4-dihydroxy-butyl)-pyrazin-2-yl]-2R,4R-di-(N-éthyl)amino-butane-1,3S-diol,
4-[5-(3S-(N-Ethyl)amino-2S,4-dihydroxy-butyl)-pyrazin-2-yl]-2S,4S-di-(N-éthyl)amino-butane-1,3S-diol,
4-[5-(3R-(N-n-Butyl)amino-2S,4-dihydroxy-butyl)-pyrazin-2-yl]-2R,4R-di-(N-n-butyl)amino-butane-1,3S-diol,
4-[5-(3S-(N-n-Butyl)amino-2S,4-dihydroxy-butyl)-pyrazin-2-yl]-2S,4S-di-(N-n-butyl)amino-butane-1,3S-diol,
4-[5-(3R-(N-Benzyl)amine-2S,4-dihydroxy-butyl)-pyrazin-2-yl]-2R,4R-di-(N-benzyl)amino-butane-1,3S-diol,
4-[5-(3S-(N-Benzyl)amino-2S,4-dihydroxy-butyl)-pyrazin-2-yl]-2S,4S-di-(N-benzyl)amino-butane-1,3S-diol,
4-[5-(3R-(N-Acétyl)amino-2S,4-dihydroxy-butyl)-pyrazin-2-yl]-2R,4R-di-(N-acétyl)amino-butane-1,3S-diol,
4-[5-(3S-(N-Acétyl)amino-2S,4-dihydroxy-butyl)-pyrazin-2-yl]-2S,4S-di-(N-acétyl)amino-butane-1,3S-diol,
4-[5-(3R-(N-Butanoyl)amino-2S,4-dihydroxy-butyl)-pyrazin-2-yl]-2R,4R-di-(N-butanoyl)amino-butane-1,3S-diol,
4-[5-(3S-(N-Butanoyl)amino-2S,4-dihydroxy-butyl)-pyrazin-2-yl]-2S,4S-di-(N-butanoyl)amino-butane-1,3S-diol,
4-[5-(3R-(N-Benzoyl)amino-2S,4-dihydroxy-butyl)-pyrazin-2-yl]-2R,4R-di-(N-benzoyl)amine-butane-1,3S-diol,
4-[5-(3S-(N-Benzoyl)amino-2S,4-dihydroxy-butyl)-pyrazin-2-yl]-2S,4S-di-(N-benzoyl)amino-butane-1,3S-diol,
4-[5-(3R-Méthoxy-2S,4-dihydroxy-butyl)-pyrazin-2-yl]-2R,4R-diméthoxy-butane-1,3S-diol,
4-[5-(3S-Méthoxy-2S,4-dihydroxy-butyl)-pyrazin-2-yl]-2S,4S-diméthoxy-butane-1,3S-diol,
4-[5-(3R-Ethoxy-2S,4-dihydroxy-butyl)-pyrazin-2-yl]-2R,4R-diéthoxy-butane-1,3S-diol,
4-[5-(3S-Ethoxy-2S,4-dihydroxy-butyl)-pyrazin-2-yl]-2S,4S-diéthoxy-butane-1,3S-diol,
4-[5-(3R-n-Butoxy-2S,4-dihydroxy-butyl)-pyrazin-2-yl]-2R,4R-di-n-Butoxy-butane-1,3S-diol,
4-[5-(3S-n-Butoxy-2S,4-dihydroxy-butyl)-pyrazin-2-yl]-2S,4S-di-n-Butoxy-butane-1,3S-diol,
4-[5-(3R-(2-Hydroxyéthyl)oxy-2S,4-dihydroxy-butyl)-pyrazin-2-yl]-2R,4R-di-(2-hydroxyéthyl)oxy-butane-1,3S-diol,
4-[5-(3S-(2-Hydroxyéthyl)oxy-2S,4-dihydroxy-butyl)-pyrazin-2-yl]-2S,4S-di-(2-hydroxyéthyl)oxy-butane-1,3S-diol,
4-[5-(3R-(3-Hydroxy-n-propyl)oxy-2S,4-dihydroxy-butyl)-pyrazin-2-yl]-2R,4R-di-(3-hydroxy-n-propyl)oxy-butane-1,3S-diol,
4-[5-(3S-(3-Hydroxy-n-propyl)oxy-2S,4-dihydroxy-butyl)-pyrazin-2-yl]-2S,4S-di-(3-hydroxy-n-propyl)oxy-butane-1,3S-diol,
4-[5-(3R-(Carboxyméthyl)oxy-2S,4-dihydroxy-butyl)-pyrazin-2-yl]-2R,4R-di-(carboxyméthyl)oxy-butane-1,3S-diol,
4-[5-(3S-(Carboxyméthyl)oxy-2S,4-dihydroxy-butyl)-pyrazin-2-yl]-2S,4S-di-(carboxyméthyl)oxy-butane-1,3S-diol,
4-[5-(3R-(3-Carboxy-n-propyl)oxy-2S,4-dihydroxy-butyl)-pyrazin-2-yl]-2R,4R-di-(3-carboxy-n-propyl)oxy-butane-1,3S-diol,
4-[5-(3S-(3-Carboxy-n-propyl)oxy-2S,4-dihydroxy-butyl)-pyrazin-2-yl]-2S,4S-di-(3-carboxy-n-propyl)oxy-butane-1,3S-diol,
1-[5-(3S,4-Dihydroxy-1E-butényl)-pyrazin-2-yl]-butane-1R,2S,3R,4-tétraol,
et leurs sels avec un acide minéral ou organique pharmaceutiquement acceptable.

Les médicaments particulièrement préférés sont ceux qui contiennent en tant que pricipe actif au moins un composé de formule (I) pour laquelle :
R₉ et R₁₀ représentent chacun un radical -CH₂OH, et soit
   a) R₃ représente un radical méthylène, R₄ représente un radical -CHOH- et l'un des radicaux R₁, R₂, R₅ et R₆ représente un radical -CHFou -CH(OR₈) et les autres représentent chacun un radical -CHOH-,
   b) R₂ et R₅ représentent chacun un radical -CHOH-, R₃ représente un radical méthylène et R₁, R₄ et R₆ sont identiques et représentent chacun un radical -CH(OR₈),
   c) R₁ et R₆ représentent chacun un radical -CHOH-, R₃ représente un radical méthylène et R₂, R₄ et R₅ sont identiques et représentent chacun un radical -CH(OR₈),
   d) R₁, R₄, R₅ et R₆ représentent chacun un radical -CHOH- et -R₂-R₃-représente un radical -CH=CH-,
R₈ représente un radical alkyle,
leurs stéréoisomères, les formes cis et trans des composés pour lesquels -R₂-R₃- représente une chaîne -CH=CH- et leurs sels avec un acide minéral ou organique pharmaceutiquement acceptable.

Les médicaments encore plus préférés sont ceux qui contiennent en tant que principe actif au moins un composé de formule (I) choisi parmi les composés suivants :
1-[5-(3S,4-Dihydroxy-butyl)-pyrazin-2-yl]-butane-1R,2S,3R,4-tétraol
1-[5-(3S,4-Dihydroxy-1E-butényl)-pyrazin-2-yl]-butane-1R,2S,3R,4-tétraol
1-[5-(2S-Méthoxy-3R,4-dihydroxy-butyl)-pyrazin-2-yl]-butane-1R,2S,3R,4-tétraol
1-[5-(2R-fluoro-3R,4-dihydroxy-butyl)-pyrazin-2-yl]-butane-1R,2S,3R,4-tétraol
1-[5-(2S,4-dihydroxy-3R-méthoxy-butyl)-pyrazin-2-yl]-butane-1R,2S,3R,4-tétraol
4-[5-(3R,4-dihydroxy-2S-méthoxy-butyl)]-pyrazin-2-yl]-3R,4R-diméthoxy-butane-1,2-diol
et leurs sels avec un acide minéral ou organique pharmaceutiquement acceptable.

Le composé de formule : 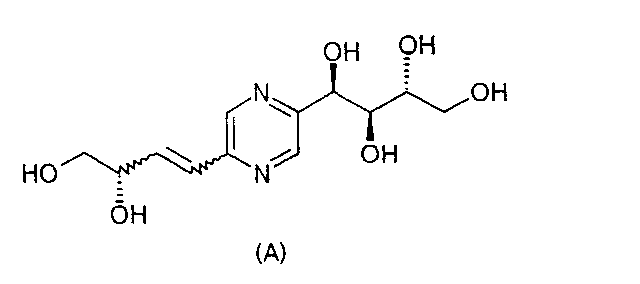 est connu (Carbohydr. Res., 77, 205 (1979)) mais aucune propriété pharmacologique n'est décrite pour celui-ci.

Les autres composés de formule (I), leurs stéréoisoméres et leurs sels sont nouveaux et font partie de l'invention en tant que tels.

Les composés de formule (I) préférés sont les composés mentionnés dans les listes des médicaments préférés à l'exception du composé de formule (A).

Les composés de formule (I) pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical carbonyle et les autres représentent chacun un radical -CHOH-, soit R₁ et R₆ représentent chacun un radical -CHOH-, R₃ représente un radical méthylène et R₂, R₄ et R₅ sont identiques et représentent chacun un radical carbonyle, soit R₂ et R₅ représentent chacun un radical -CHOH-, R₃ représente un radical méthylène et R₁, R₄ et R₆ sont identiques et représentent chacun un radical carbonyle peuvent être préparés par oxydation d'un dérivé choisi parmi les formules : 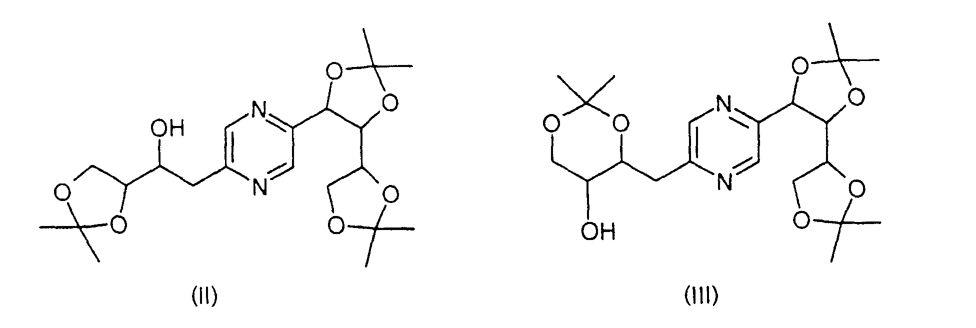 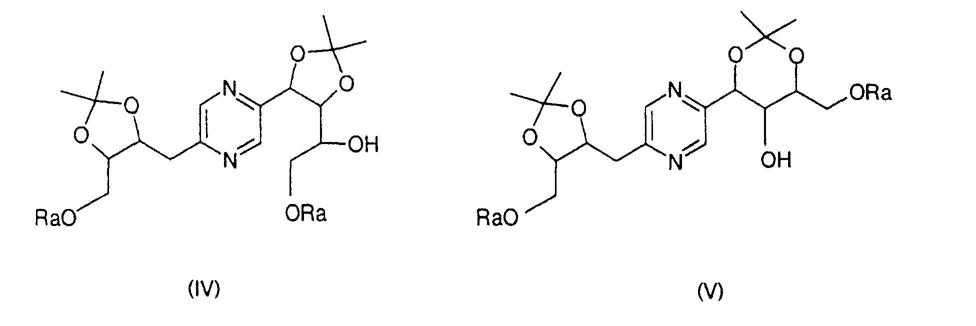 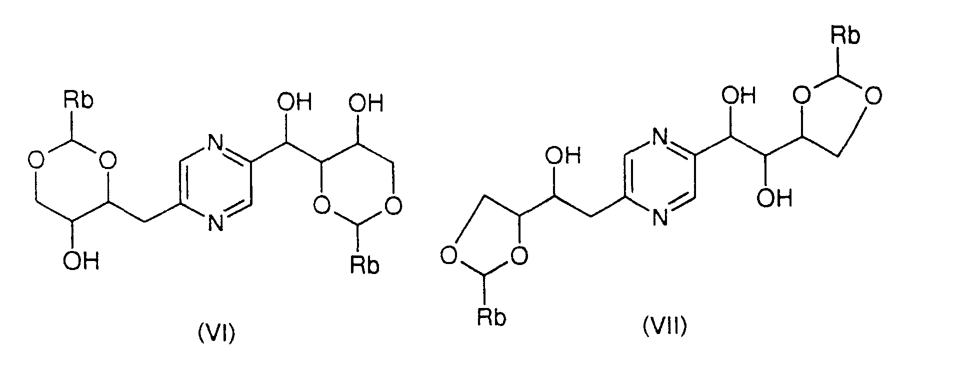 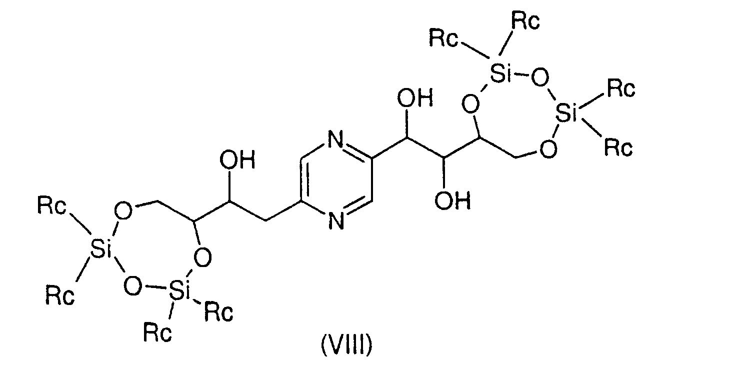 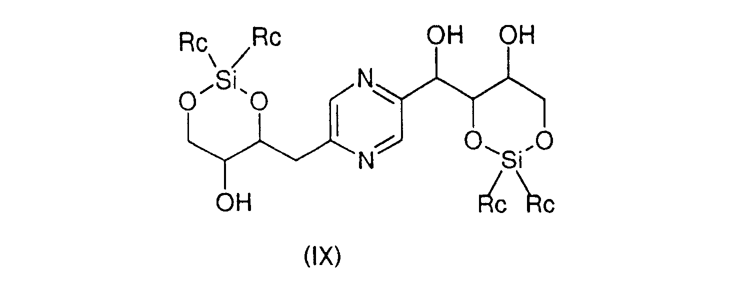 dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle ou un stéréoisomère d'un tel dérivé, suivie d'une déprotection des hydroxyles.

Les radicaux trialkylsilyle, alkyldiphénylsilyle ou dialkylphénylsilyle préférés sont les radicaux triméthylsilyle, tert-butyldiphénylsilyle et diméthylphénylsilyle.

La réaction d'oxydation s'effectue par toute méthode connue d'oxydation des fonctions alcool et notamment celles décrites par D. SWERN et coll., Synthesis, 165 (1981) et T.T. TIDWELL, Synthesis, 857 (1990). De préférence, cette oxydation est effectuée dans le diméthylsulfoxyde, en présence de chlorure d'oxalyle et de triéthylamine, à une température comprise entre -78°C et 0°C.

La déprotection des hydroxyles s'effectue par toute méthode connue de déprotection et notamment celles décrites par T.W. GREENE, Protective Groups in Organic Synthesis, J. Wiley-Interscience Publication (1991) ou par S.V. LEY et coll., Tetrahedron, 46, 4995 (1990). De préférence, on utilise l'acide trifluoroacétique, à une température comprise entre 0°C et 100°C ou le fluorure de tétra(n-butyl)ammonium, au sein du tétrahydrofuranne, à une température voisine de 25°C.

Les dérivés de formules (II) et (III) peuvent être obtenus par action du 2,2-diméthoxypropane sur la 2-(1,2,3,4-tétrahydroxybutyl)-5-(2,3,4-trihydroxybutyl)-pyrazine ou un de ses stéréoisomères et séparation des dérivés (II) et (III).

Cette réaction s'effectue généralement selon les conditions réactionnelles décrites par T.W. GREENE, Protective Groups in Organic Synthesis, J. Wiley-Interscience Publication (1991). De préférence, on opère en présence d'un acide tel que l'acide paratoluènesulfonique, au sein d'un solvant inerte tel que le diméthylformamide, à une température comprise entre 0°C et 100°C.

Les dérivés de formule (IV) et (V) peuvent être obtenus par action du 2,2-diméthoxypropane sur la 2-(1,2,3,4-tétrahydroxybutyl)-5-(2,3,4-trihydroxybutyl)pyrazine dont les hydroxyles en bout de chaîne ont été préalablement bloqués au moyen de chlorure de trialkylsilyle ou de chlorure d'alkyldiphénylsilyle ou de chlorure de dialkyphénylsilyle ou un stéréoisomère d'un tel dérivé, puis séparation des dérivés (IV) et (V).

Cette réaction s'effectue généralement selon les conditions réactionnelles décrites par T.W. GREENE, Protective Groups in Organic Synthesis, J. Wiley-Interscience Publication (1991). De préférence, on opère en présence d'un acide tel que l'acide paratoluènesulfonique, au sein d'un solvant inerte tel que le diméthylformamide, à une température comprise entre 0°C et 100°C.

La protection des hydroxyles en bout de chaîne, s'effectue généralement selon les conditions réactionnelles décrites par T.W. GREENE, Protective Groups in Organic Synthesis, J. Wiley-Interscience Publication (1991). De préférence, on opère au sein de la pyridine, à une température comprise entre 0°C et 30°C.

Les dérivés de formule (VI) et (VII) peuvent être obtenus par action d'un benzaldéhyde dont le phényle est éventuellement substitué par au moins un radical alcoxy ou un dérivé dialkylacétal du benzaldéhyde dont le phényle est éventuellement substitué par au moins un radical alcoxy (benzaldéhydediméthylacétal par exemple) sur la 2-(1,2,3,4-tétrahydroxybutyl)-5-(2,3,4-trihydroxybutyl)pyrazine ou un de ses stéréoisomères.

Cette réaction s'effectue généralement selon les conditions réactionnelles décrites par R.S. COLEMAN et coll., J. Org. Chem., 57, 3732 (1992). De préférence, on opère en présence d'un acide tel que l'acide D-camphosulfonique, au sein d'un solvant inerte tel que le diméthylformamide, à une température comprise entre 0°C et 100°C.

Les dérivés de formule (VIII) et (IX) peuvent être respectivement obtenus par action de 1,3-dichloro-1,1,3,3-tétraalkyldisiloxane ou de 1,3-dichloro-1,1,3,3-tétraphényldisiloxane d'une part, ou de bis(chlorure) de dialkylsilyle ou de bis(trifluorométhanesulfonate) de dialkylsilyle ou de bis(chlorure) de diphénylsilyle ou de bis(trifluorométhanesulfonate) de diphénylsilyle d'autre part, sur la 2-(1,2,3,4-tétrahydroxybutyl)-5-(2,3,4-trihydroxybutyl)pyrazine ou un de ses stéréoisomères généralement selon les conditions réactionnelles décrites par T.W. GREENE, Protective Groups in Organic Synthesis, J. Wiley-Interscience Publication (1991). De préférence, on opère au sein de la pyridine, en présence respectivement de 1,3-dichloro-1,1,3,3-tétraisopropyldisiloxane ou de bis(trifluorométhanesulfonate) de diisopropylsilyle à une température comprise entre 0°C et 30°C.

La 2-(1,2,3,4-tétrahydroxybutyl)-5-(2,3,4-trihydroxybutyl)pyrazine et ses stéréoisomères peuvent être obtenus soit à partir d'un ou deux amino-aldoses OHC-CH(NH₂)-(CHOH)₃-CH₂OH ou un de ses stéréoisomères, en milieu acide et plus particulièrement en mileu acide acétique et en opérant de préférence à une température comprise entre 15°C et 100°C; soit à partir d'un ou deux cétoses HOCH₂-CO-(CHOH)₃-CH₂OH ou un de ses stéréoisomères, par action du formiate d'ammonium et en opérant préférentiellement à une température comprise entre 15°C et 100°C, et de préférence en milieu aqueux.

L'amino-aldose OHC-CH(NH₂)-(CHOH)₃-CH₂OH et ses stéréoisomères sont commercialisés ou peuvent être préparés par application ou adaptation des méthodes décrites par exemple dans :
(a) Methods Carbohydr. Chem., 7, 29 (1976) qui consistent à transformer la fonction aldéhyde de l'aldose correspondant en un groupement nitroéthylénique à l'aide du nitrométhane en milieu basique (éthylate de sodium par exemple) puis à traiter le produit obtenu successivement par une solution saturée d'ammoniaque, à une température comprise entre 20°C et 30°C, par du Ba(OH)₂ en solution aqueuse, à une température comprise entre 20°C et 30°C et enfin de l'acide sulfurique dilué (10 à 15%), à une température comprise entre 20°C et 30°C,
(b) «The Amino Sugar» , éditeur: R. W. Jeanloz, Academic Press, New-York, 1969, page 1 ou «The Carbohydrates», éditeurs: W. Pigman et D. Horton, Academic Press, New-York, Volume IB, 1980, page 664 qui consistent à transformer la fonction aldéhyde de l'aldose correspondant en un groupement imino à partir d'une amine primaire aromatique (aniline par exemple), de faire ensuite successivement réagir l'acide cyanhydrique, à une température comprise entre 0°C et 20°C et de l'hydrogène en présence de palladium dans un solvant tel qu'un éther (tétrahydrofuranne par exemple) ou un alcool aliphatique (l'éthanol ou le méthanol par exemple), à une température comprise entre 20°C et 50°C.

Le cétose HOCH₂-CO-(CHOH)₃-CH₂OH et ses stéréoisomères sont commercialisés ou peuvent être préparés par application ou adaptation des méthodes décrites par exemple dans :
a) Adv. Carbohydr. Chem., 13, 63 (1958) qui consistent à faire réagir l'aldose correspondant soit avec une base telle l'hydroxyde de calcium, la soude, la pyridine, la quinoléine, soit avec un acide tel que l'acide sulfurique en solution aqueuse ou en phase pure, à une température comprise entre 20 et 50°C
b) Tetrahedron Asymmetry, 7(8), 2185, (1996), J. Am. Chem; Soc., 118(33), 7653 (1996), J. Org. Chem., 60(13), 4294 (1995), Tetrahedron Lett., 33(36), 5157 (1992), J. Am. Chem. Soc., 113(17), 6678 (1991), Angew. Chem., 100(5), 737, (1988), J. Org. Chem., 57, 5899 (1992) qui consistent par exemple à condenser soit l'hydroxypyruvaldéhyde, la 1,3-dihydroxyacétone, le 1,3-dihydroxyacétone monophosphate ou l'acide hydroxypyruvique sur un 2-hydroxy-acétaldéhyde substitué en position 2, éventuellement optiquement pur, en présence éventuellement d'une enzyme telle qu'une transcétolase. Cette réaction s'éffectue généralement en solution aqueuse, à une température comprise entre 20 et 50°C, éventuellement en présence d'une base (la soude par exemple), de chlorure de barium, de chlorure de magnésium ou de chlorure de zinc. Les dérivés possédant un groupement 2-hydroxy-acétaldéhyde sont commercialisés ou peuvent être préparés à partir d'aldoses par application ou adaptation des méthodes décrites dans P. Collins, R. Ferrier, Monosaccharides, Their Chemistry and their roles in Natural Products, éditeur J. Wiley (1995), M. Bols, Carbohydrate Building Bloks, éditeur J. Wiley (1996).

Les aldoses correspondants et leurs stéréoisomères sont commercialisés ou peuvent être obtenus à partir :
a) d'aldoses commercialement disponibles :
   - par des réactions d'épimérisation par application ou adaptation des méthodes décrites dans Adv. Carbohydr. Chem., 13, 63, (1958) notamment en milieu basique au moyen d'une solution aqueuse diluée de soude (0,03 à 0.05%), à une température comprise entre 20 et 40°C,
   - par des réactions d'allongement de chaîne par application ou adaptation des méthodes décrites dans «The Carbohydrates», éditeurs: W. Pigman et D. Horton, Academic Press, New-York, Volume IA, 133 (1972) et notamment en formant la cyanhydrine de l'aldose de départ (par exemple par action du cyanure de sodium en solution aqueuse, à une température comprise entre 10 et 30°C et en présence de soude, à un pH voisin de 9) puis hydrolyse de la fonction nitrile ainsi formée en acide correspondant par application ou adaptation des méthodes décrites dans Organic Synthesis volume I page 436 et volume III page 85 (par exemple à l'aide d'acide chlorhydrique ou d'acide sulfurique concentré, en solution aqueuse, à une température comprise entre 20°C et la température d'ébullition du milieu réactionnel), puis réduction de la fonction acide carboxylique en aldéhyde correspondant par application ou adaptation des méthodes décrites dans J. Am. Chem. Soc. 71, 122 (1949) notamment à l'aide d'un borohydrure d'un métal alcalin (le borohydrure de sodium par exemple), en solution aqueuse à une température comprise entre 20°C et la température d'ébullition du milieu réactionnel,
   - par des réactions de racourcissement de chaînes par application ou adaptation des méthodes décrites dans «The Carbohydrates», éditeurs: W. Pigman et D. Horton, Academic Press, New-York, Volume IB, 1980, page 929 ou Chem. Ber., 83, 559 (1950) et notamment en transformant la fonction aldéhyde de l'aldose en hydroxylamine correspondant par application ou adaptation des méthodes décrites dans Organic Synthesis volume II page 314 (par exemple à l'aide de chlorhydrate d'hydroxylamine, en solution aqueuse et en présence d'une base telle que le carbonate de sodium à une température comprise entre 20 et 50°C), puis action du 3,4-dinitro-fluorobenzéne en présence de dioxyde de carbone et d'une base telle le d'hydrogénocarbonate de sodium en solution aqueuse et d'un alcool aliphatique (alcool isopropylique par exemple), à une température comprise entre 50 et 80°C,
b) d'alcools allyliques correspondants par application ou adaptation des méthodes décrites dans Science, 220, 949 (1983) et notamment à l'aide d'hydroperoxyde de terbutyle en présence d'un complexe de titane (IV) tel que le complexe isopropylate de titane (IV) et tartrate de dialkyle optiquement pur (le tartrate de diéthyle par exemple), suivi de l'action successive de thiophénolate de sodium, d'acide para-chloroperbenzoïque dans l'anhydride acétique et d'hydrure de diisopropylaluminium.

Les composés de formule (I) pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical méthylène et les autres représentent chacun un radical -CHOH-, soit R₁ et R₆ représentent chacun un radical -CHOH-, R₃ représente un radical méthylène et R₂, R₄ et R₅ sont identiques et représentent chacun un radical méthylène, soit R₂ et R₅ représentent chacun un radical -CHOH- et R₁, R₃, R₄ et R₆ représentent chacun un radical méthylène peuvent être préparés par condensation d'un chlorothionocarbonate d'alkyle ou de phényle sur un dérivé de formule (II), (III), (IV), (V), (VI), (VII), (VIII) ou (IX) dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle ou un stéréoisomère d'un tel dérivé puis réduction du produit obtenu et déprotection des hydroxyles.

La condensation et la réduction s'effectuent généralement selon les conditions réactionnelles décrites par D.H.R. BARTON, J. Chem. Soc. Perkin I, 1574 (1975) et H. PAULSEN et coll., Liebigs Ann. Chem., 735 (1992). De préférence, pour la condensation, on opère au sein d'un solvant inerte tel qu'un solvant chloré (dichlorométhane par exemple), un éther (diéthyléther, tétrahydrofuranne, dioxanne par exemple), en présence d'un accepteur d'acide tel qu'une base organique comme la pyridine ou la 4-diméthylaminopyridine, à une température voisine de 20°C et, pour la réduction, on opère au moyen d'hydrure de tributylétain et d'azobis(2-méthylpropionitrile), au sein d'un solvant inerte tel que un solvant aromatique (benzène, toluène par exemple), à une température comprise entre 80°C et 110°C. La déprotection s'effectue comme mentionné précédemment.

Les composés de formule (I) pour lesquels (A) R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical -CHF- et les autres représentent chacun un radical -CHOH-, soit R₁ et R₆ représentent chacun un radical -CHOH-, R₃ représente un radical méthylène et R₂, R₄ et R₅ sont identiques et représentent chacun un radical -CHF-, soit R₂ et R₅ représentent chacun un radical -CHOH-, R₃ représente un radical méthylène et R₁, R₄ et R₆ sont identiques et représentent chacun un radical -CHF- soit (B) R₁, R₂, R₄, R₅ et R₆ représentent chacun un radical -CHOH-, R₃ représente un radical méthylène, R₉ représente un radical -CH₂F ou -CH₂OH, R₁₀ représente un radical -CH₂F ou -CH₂OH, R₉ et R₁₀ n'étant pas tous les deux un radical -CH₂OH peuvent être préparés par fluoration d'un dérivé de formule (II), (III), (IV), (V), (VI), (VII), (VIII) ou (IX) dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle ou un stéréoisomère d'un tel dérivé, suivie d'une déprotection des hydroxyles. Les composés pour lesquels R₁, R₂, R₄, R₅ et R₆ représentent chacun un radical -CHOH-, R₃ représente un radical méthylène, R₉ représente un radical -CH₂F ou -CH₂OH, R₁₀ représente un radical -CH₂F ou -CH₂OH mais R₉ et R₁₀ n'étant pas tous les deux un radical -CH₂OH sont obtenus en mélange avec les composés préparés à partir de l'intermédiaire (IV).

Cette fluoration s'effectue généralement selon les conditions opératoires décrites par W.J. MIDDLETON, J. Org. Chem., 40, 574 (1975). De préférence, la fluoration s'effectue au moyen d'un trifluorure de dialkylaminosulfure (trifluorure de diéthylaminosulfure par exemple), au sein d'un solvant inerte tel qu'un solvant chloré (dichlorométhane par exemple), ou un éther (tétrahydrofuranne par exemple), à une température comprise entre -78°C et 20°C. La déprotection s'effectue comme mentionné précédemment.

Les composés de formule (I) pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical -CH(NHR₇)- et les autres représentent chacun un radical -CHOH-, soit R₁ et R₆ représentent chacun un radical -CHOH-, R₃ représente un radical méthylène et R₂, R₄ et R₅ sont identiques et représentent chacun un radical -CH(NHR₇)-, soit R₂ et R₅ représentent chacun un radical -CHOH-, R₃ représente un radical méthylène et R₁, R₄ et R₆ sont identiques et représentent chacun un radical -CH(NHR₇)- peuvent être préparés par réduction d'un dérivé choisi parmi les formules : 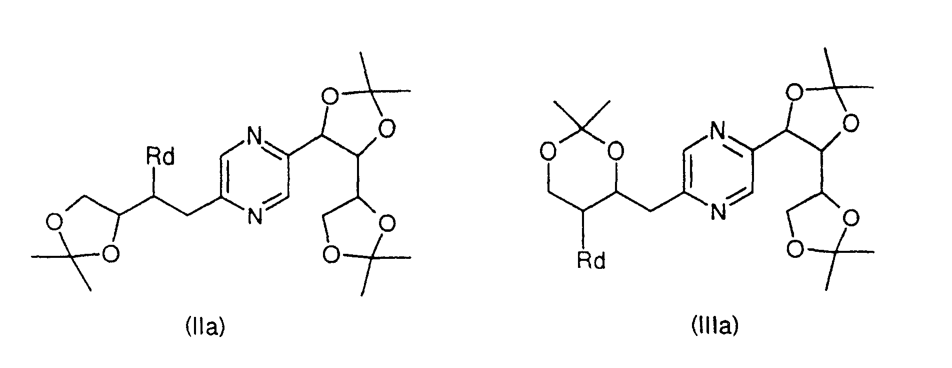 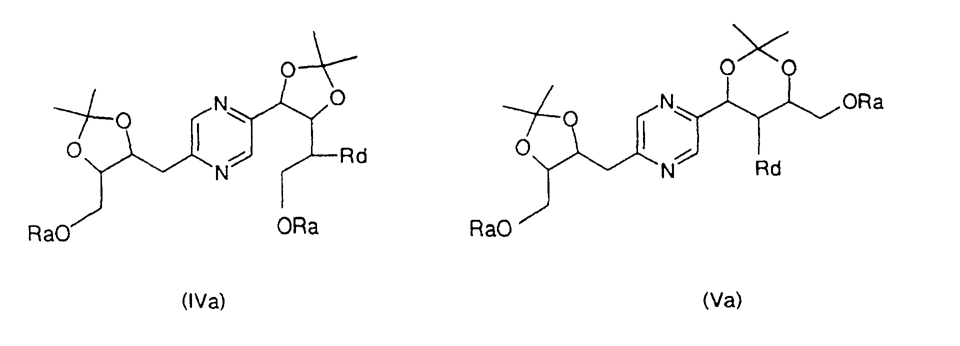 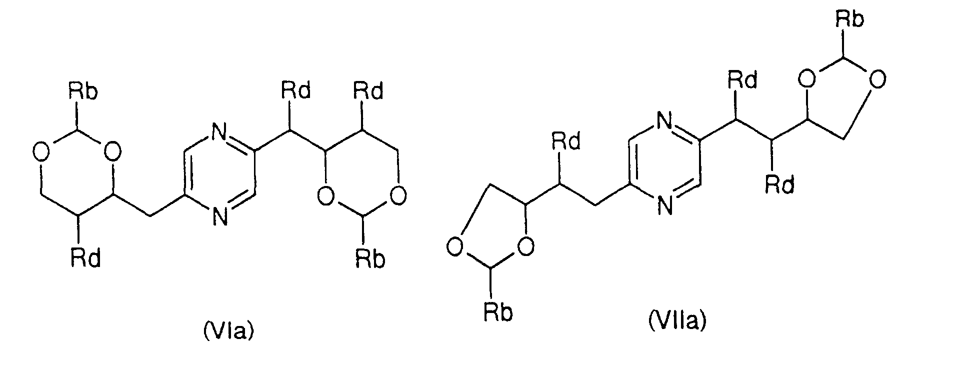 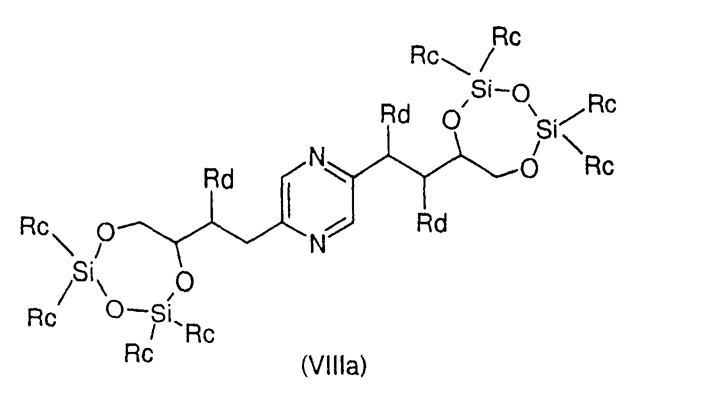 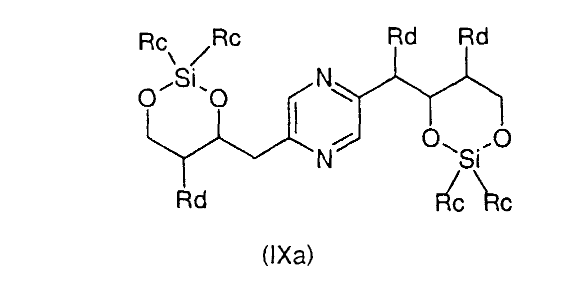 dans lesquelles Ra représente un radical trialkylsilyle, alkyldiphénylsilyle ou dialkylphénylsilyle, Rb représente un radical phényle éventuellement substitué par au moins un radical alcoxy, Rc représente un radical alkyle ou phényle et Rd représente un radical azido ou un stéréoisomère d'un tel dérivé, suivie éventuellement de la réaction d'un dérivé de formule HalR₇ pour lequel R₇ a les mêmes significations que dans la formule (I) sauf hydrogène et Hal représente un atome d'halogène et suivie d'une déprotection des hydroxyles.

La réduction s'effectue généralement selon les conditions réactionnelles décrites par R.C. LAROCK, Comprehensive Organic Transformations, VCH Publication (1989). De préférence, on opère soit au moyen d'hydrogène, en présence d'un catalyseur tel que le palladium, au sein d'un solvant inerte tel qu'un alcool aliphatique 1-4C (méthanol par exemple), à une température voisine de 20°C ou bien au moyen d'un hydrure de métal alcalin (borohydrure de métal alcalin comme le borohydrure de sodium), l'aluminohydrure de lithium, au sein d'un solvant inerte tel qu'un éther (tétrahydrofuranne, diéthyléther par exemple), à une température comprise entre -78°C et 100°C.

La réaction avec le dérivé HalR₇ s'effectue généralement selon les conditions réactionnelles décrites par R.C. LAROCK, Comprehensive Organic Transformations, VCH Publication (1989). De préférence, on opère en présence d'une base organique comme une amine (trialkylamine telle que triéthylamine, pyridine) ou une base organométallique comme un dialkylamidure de métal alcalin (sodium, lithium par exemple) ou un hydrure de métal alcalin (hydrure de sodium par exemple) ou d'une base minérale telle qu'un hydroxyde de métal alcalin (soude, potasse par exemple), au sein d'un solvant inerte tel qu'un éther (diéthyléther, tétrahydrofuranne, dioxanne par exemple), un alcool aliphatique 1-4C (méthanol, éthanol par exemple), un solvant chloré (dichlorométhane par exemple), le diméthylformamide ou le diméthylsulfoxyde, à une température comprise entre 0°C et la température d'ébullition du milieu réactionnel.

La déprotection s'effectue comme mentionné précédemment.

Les dérivés de formules (IIa), (IIIa), (IVa), (Va), (VIa), (VIIa), (VIIIa) et (IXa) dans lesquelles Ra représente un radical trialkylsilyle, alkyldiphénylsilyle ou dialkylphénylsilyle, Rb représente un radical phényle éventuellement substitué par au moins un radical alcoxy, Rc représente un radical alkyle ou phényle et Rd représente un radical azido et leurs stéréoisomères peuvent être obtenus par action d'un azoture de métal alcalin (sodium de préférence) sur un dérivé de formule (IIa), (IIIa), (IVa), (Va), (VIa), (VIIa), (VIIIa) ou (IXa) dans laquelle Ra représente un radical trialkylsilyle, alkyldiphénylsilyle ou dialkylphénylsilyle, Rb représente un radical phényle éventuellement substitué par au moins un radical alcoxy, Rc représente un radical alkyle ou phényle et Rd représente un radical -OSO₂-Re pour lequel Re représente un radical méthyle, trifluorométhyle ou 4-méthylphényle ou un stéréoisomère d'un tel dérivé.

Cette réaction s'effectue généralement selon les conditions opératoires décrites par A.C. RICHARDSON, Methods Carbohydr. Chem., 6, 218 (1972). De préférence, on opère au sein d'un solvant inerte tel que le diméthylformamide, à une température comprise entre 0°C et 100°C.

Les dérivés de formules (IIa), (IIIa), (IVa), (Va), (VIa), (VIIa), (VIIIa) et (IXa) pour lesquels Ra représente un radical trialkylsilyle, alkyldiphénylsilyle ou dialkylphénylsilyle, Rb représente un radical phényle éventuellement substitué par au moins un radical alcoxy, Rc représente un radical alkyle ou phényle et Rd représente un radical -OSO₂-Re et leurs stéréoisomères peuvent être préparés par action d'un dérivé ClSO₂Re ou (ReSO₂)₂O pour lesquels Re a les mêmes significations que précédemment sur un dérivé de formule (II), (III), (IV), (V), (VI), (VII), (VIII) ou (IX) dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle ou un stéréoisomère d'un tel dérivé.

Cette réaction s'effectue généralement selon les conditions opératoires décrites par A.C. RICHARDSON, Methods Carbohydr. Chem., 6, 218, (1972) et H. PAULSEN, Liebigs Ann. Chem., 735 (1992). De préférence, on opère au sein d'un solvant inerte tel qu'un solvant chloré (dichlorométhane par exemple), en présence d'une base organique telle que la pyridine, à une température comprise entre -20°C et 20°C.

Les composés de formule (I) pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical -CH(OR₈) et les autres représentent chacun un radical -CHOH-, soit R₁ et R₆ représentent chacun un radical -CHOH-, R₃ représente un radical méthylène et R₂, R₄ et R₅ sont identiques et représentent chacun un radical -CH(OR₈), soit R₂ et R₅ représentent chacun un radical -CHOH-, R₃ représente un radical méthylène et R₁, R₄ et R₆ sont identiques et représentent chacun un radical -CH(OR₈) peuvent être préparés par action d'un dérivé de formule (II), (III), (IV), (V), (VI), (VII), (VIII) ou (IX) ou un stéréoisomère d'un tel dérivé et d'un dérivé HalR₈ pour lequel R₈ a les mêmes significations que dans la formule (I).

Cette réaction s'effectue généralement selon les conditions réactionnelles décrites R.C. LAROCK, Comprehensive Organic Transformations, VCH Publications (1989). De préférence, on opère en présence d'une base organique telle qu'une amine (trialkylamine telle que triéthylamine, pyridine), une base organométallique telle qu'un dialkylamidure de métal alcalin (diisopropylamidure de lithium par exemple) ou un hydrure de métal alcalin (hydrure de sodium par exemple) ou une base minérale (hydroxyde de métal alcalin (soude ou potasse par exemple)), au sein d'un solvant inerte tel qu'un éther (tétrahydrofuranne, diéthyléther par exemple), un alcool aliphatique (méthanol, éthanol par exemple), un solvant chloré (dichlorométhane par exemple), le diméthylformamide ou le diméthylsulfoxyde, à une température comprise entre 0°C et la température d'ébullition du milieu réactionnel.

La déprotection s'effectue comme mentionné précédemment.

Les composés de formule (I) pour lesquels R₁, R₄, R₅ et R₆ représentent chacun un radical -CHOH- et -R₂-R₃- représente un radical -CH=CH- peuvent être préparés par déshydratation d'un dérivé de formule (IIa) dans laquelle Rd représente un radical -OH ou -OSO₂-Re dans lequel Re représente un radical méthyle, trifluorométhyle ou 4-méthylphényle ou un stéréoisomère d'un tel dérivé, puis déprotection des hydroxyles.

Cette réaction s'effectue généralement selon les conditions réactionnelles décrites R.C. LAROCK, Comprehensive Organic Transformations, VCH Publications (1989). De préférence, on opère au moyen d'une base organique telle qu'une amine (trialkylamine telle que triéthylamine ou la 1,8-diazabicyclo[5.4.0]undéc-7-ène), une base organométallique (alcoolate de métal alcalin tel que éthanolate de sodium) ou un dialkylamidure d'un métal alcalin ou alcalinoterreux (diisopropylamidure de lithium par exemple) ou d'une base minérale telle qu'un hydroxyde de métal alcalin (soude ou potasse par exemple), au sein d'un solvant inerte tel qu'un alcool aliphatique 1-4C (méthanol, éthanol par exemple), un éther (diéthyléther, tétrahydrofuranne par exemple), un solvant chloré (dichlorométhane par exemple) ou le diméthylformamide, à une température comprise entre 0°C et la température d'ébullition du milieu réactionnel. Cette réaction peut également être effectuée selon les conditions réactionnelles décrites par O. MITSUNOBU, Synthesis, p1 (1981). De préférence, on opère en milieu organique au sein d'un solvant inerte tel qu'un éther (diéthyléther, tétrahydrofuranne par exemple), en présence de trialkylphosphine (triphénylphosphine par exemple) et de dialkyl azodicarboxylate (diéthylazocarboxylate par exemple), à une température comprise entre 0°C et la température d'ébullition du milieu réactionnel.Les différents stéréoisomères des composés de formule (I) sont obtenus à partir des stéréoisomères correspondants des différents intermédiaires (II), (III), (IV), (V), (VI), (VII), (VIII)ou (IX).Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. Comme exemples de groupes protecteurs de la fonction hydroxy, on peut citer les trialkylsilyle (triéthylsilyle par exemple), benzyle. Comme groupes protecteurs des fonctions carboxy, on peut citer les esters (méthoxyméthylester, tétrahydropyranylester, benzylester par exemple), les oxazoles et les 2-alkyl-1,3-oxazolines. D'autres groupes protecteurs utilisables dans ces procédés sont également décrits par W. GREENE et coll., Protective Groups in Organic Synthesis, second édition, 1991, Jonh Wiley & Sons et P.J. KOCIENSKI, Protecting groups, éditeur Thieme Verlag (1994).

Les mélanges réactionnels obtenus par les divers procédés décrits précédemment sont traités selon des méthodes classiques physiques (évaporation, extraction, distillation, chromatographie, cristallisation par exemple) ou chimiques (formation de sels par exemple).

Les composés de formule (I) pêuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les exemples suivants illustrent l'invention :

### EXEMPLE 1

A 349 mg de 2-[2,2-diméthyl-[1,3]dioxolan-4S-yléthyl]-5-[2,2,2',2'-tétraméthyl-[4R,4'R]bi[[1,3]dioxolanyl]-5S-yl]-pyrazine on ajoute 50 cm³ d'une solution aqueuse d'acide trifluoroacétique à 80%. Le mélange réactionnel est agité à une température d'environ 25°C pendant 4 heures. Après concentration sous pression réduite (2,7 kPa) à une température de 50°C, l'huile résiduelle est reprise dans 10 cm³ de toluène et reconcentrée dans les mêmes conditions. L'huile obtenue est reprise dans 2 cm³ d'éthanol absolu et placée dans un bain de glace pendant 2 heures. Le précipité obtenu est filtré sur verre fritté, essoré puis séché dans un dessicateur sous pression réduite (2,7 kPa) à une température de 25°C. On obtient un solide beige qui est recristallisé dans un mélange eau/éthanol absolu (1:8 en volumes). Les cristaux sont filtrés sur verre fritté, lavés avec 0,2 cm³ d'éthanol absolu, essorés puis séchés sous pression réduite (2,7 kPa) à une température de 40°C. On isole ainsi 158 mg de 1-[5-(3S,4-Dihydroxy-butyl)-pyrazin-2-yl]-butane-1R,2S,3R,4-tétraol sous forme d'une poudre cristalline de couleur ivoire fondant à 171°C [Spectre de R.M.N. ¹H (400 MHz, (CD₃)₂SO d6, δ en ppm) : 1,63 et 1,88 (2 mts, 1H chacun : CH₂ 5β); 2,79 et 2,86 (2 mts, 1H chacun : CH₂ 5α); de 3,20 à 3,70 (mt, 7H : CH 2β - CH 2γ - CH 5γ - CH₂ 2δ et CH₂O 5δ); 4,38 et 4;51 (respectivement t large et t, J = 6 Hz, 1H chacun : OH en 2δ et OH en 5δ); 4,43 (d, J = 7,5 Hz, 1H : OH); 4,58 (d, J = 5 Hz, 1H : OH); 4,65 (d, J = 5 Hz, 1H : OH); 4,95 (d large, J = 6 Hz, 1H : CH 2α); 5,31 (d, J = 6 Hz, 1H : OH en 2α); 8,43 (s, 1H : =CH en 6); 8,63 (s, 1H : =CH en 3); α_{D}²⁰=-55,9° ±1,5 (c=0,36/ eau)].

La 2-[2,2-diméthyl-[1,3]dioxolan-4R-yléthyl]-5-[2,2,2',2'-tétraméthyl-[4R,4'R]bi[[1,3]dioxolanyl]-5S-yl]-pyrazine est préparée selon la méthode suivante : A une solution de 0,72 g de phénylthionocarbonate de 1S-2,2-Di-méthyl-[1,3]dioxolan-4R-yl)-2-[5-(2,2,2',2'-tétraméthyl-[4R,4'R]bi[[1,3]dioxola nyl]-5S-yl)-pyrazin-2-yl]-éthyle dans 56 cm³ de toluène sous argon est ajouté une solution de 1,1 cm³ d'hydrure de tributylétain et 13 mg de 2,2'-azobis(2-méthyl-propionitrile) dans 14 cm³ de toluène. Le mélange réactionnel est chauffé à une température de 80°C pendant 45 minutes puis à reflux à une température d'environ 110°C pendant 70 heures. Après filtration sur filtre en papier, le mélange est concentré sous pression réduite (2,7 kPa) à une température de 40°C. L'huile résiduelle est chromatographiée sur une colonne de silice (0,020-0,045 mm) éluée avec un mélange acétate d'éthyle/cyclohexane (1:4 en volumes) à une pression d'environ 1,5x10⁵ Pa. Les fractions contenant le produit attendu sont réunies et concentrées sous pression réduite (2,7 kPa) à une température de 40°C. On obtient ainsi 0,36 g de 2-[2,2-diméthyl-[1,3]dioxolan-4S-yléthyl]-5-[2,2,2',2'-tétraméthyl-[4R,4'R]bi[[1,3]dioxolanyl]-5S-yl]-pyrazine sous forme d'une huile jaune (Rf=0,5; chromatographie sur couche mince de gel de silice; éluant : mélange acétate d'éthyle/cyclohexane (1:1 en volumes)).

Le phénylthionocarbonate de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthyle est préparé selon la méthode suivante : A une solution de 0,72 g de 1S-(2,2-Diméthyl-[1,3]dioxolan-4R-yl)-2-[5-(2,2,2',2'-tétraméthyl-[4R,4'R]bi[[1,3]dioxo lanyl]-5S-yl)-pyrazin-2-yl]-éthanol dans 50 cm³ de dichlorométhane sont ajoutés successivement 2 cm³ de pyridine, 0,021 g de 4-diméthylaminopyridine et 0,35 cm³ de phénylchlorothionocarbonate. Le mélange réactionnel est agité à une température d'environ 25°C pendant 1 jour, puis il est dilué avec un mélange de 20 cm³ d'eau et de 20 cm³ de dichlorométhane. Après décantation, la phase organique est lavée 3 fois avec 20 cm³ d'eau, séchée sur sulfate de magnésium, puis concentrée sous pression réduite (2,7 kPa) à une température voisine de 45°C. L'huile résiduelle est chromatographiée sur une colonne de silice (0,020-0,045 mm) éluée avec un mélange acétate d'éthyle/cyclohexane (1:4 en volumes) à une pression d'environ 1.5x10⁵ Pa. Les fractions contenant le produit attendu sont réunies et concentrées sous pression réduite (2,7 kPa) à une température de 40°C. On obtient ainsi 0,74 g de phénylthionocarbonate de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthyle sous forme d'une huile jaune (Rf=0,2; chromatographie sur couche mince de gel de silice; éluant mélange acétate d'éthyle/cyclohexane (1:4 en volumes)).

Le 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthanol est préparé selon la méthode suivante : A une solution de 10 g de désoxyfructosazine dans 250 cm³ de diméthylformamide sous agitation on ajoute 81 cm³ de 2,2-diméthoxypropane puis 0,3 g d'acide para-toluènesulfonique. Le mélange réactionnel est agité à une température d'environ 25°C pendant 20 heures, puis 10 cm³ de 2,2-diméthoxypropane sont rajoutés et l'agitation est poursuivie pendant 3 heures. Le mélange est alors chauffé à une température de 50°C pendant 21 heures. Après concentration sous pression réduite (2,7 kPa) à une température de 60°C, l'huile résiduelle est dissoute dans 300 cm³ de dichlorométhane et lavée 2 fois avec 100 cm³ d'une solution aqueuse de bicarbonate de sodium à 5% puis 2 fois avec 200 cm³ d'eau. La phase organique est séchée sur sulfate de magnésium puis concentrée sous pression réduite (2,7 kPa) à une température de 40°C. L'huile obtenue est chromatographiée sur une colonne de silice (0,020-0,045 mm) éluée avec un mélange acétate d'éthyle/cyclohexane (1:1 en volumes) à une pression d'environ 1,5x10⁵ Pa. Les fractions contenant les produits attendus sont réunies et concentrées sous pression réduite (2,7 kPa) à une température de 40°C. On obtient ainsi 5,2 g de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthanol sous forme d'un solide blanc ainsi que 2,4 g du même produit impur. Ce dernier est recristallisé dans un mélange eau/éthanol absolu (5:1 en volumes). On isole ainsi 0,6 g de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthanol sous forme de cristaux blancs fondant à 74°C [Spectre de R.M.N. ¹H (400 MHz, (CD₃)₂SO d6, δ en ppm) : 1,04 - 1,20 - 1,28 - 1,33 et 1,44 (5 s, respectivement 3H - 3H - 3H - 3H et 6H : les 6 CH₃); 2,79 (dd, J = 13 et 9 Hz, 1H : 1H du CH₂ 5α); 3,06 (dd, J = 13 et 2,5 Hz, 1H : l'autre H du CH₂ 5α); 3,79 (mf, 1H : CH 5β); de 3,80 à 3,90 (mt, 2H : 1H du CH₂ 2δ et 1H du CH₂ 5δ); 3,91 (mt, 1H : CH 5γ); 4,00 (t, J = 7 Hz, 1H : l'autre H du CH₂ 5δ); 4,06 (t, J = 7,5 Hz, 1H : l'autre H du CH₂2δ); 4,28 (mt, 1H : CH 2γ); 4,33 (t, J = 7 Hz, 1H CH 2β); 4,99 (d, J = 7,5 Hz, 1H : CH 2α); 5,07 (d large, J = 5 Hz, 1H: OH en 5β); 8,54 (s, 1H : =CH en 6); 8,66 ( s, 1H : =CH en 3); **α**_{D}²⁰=+6,7° ± 1,1 (c=0,5/ dichlorométhane); (Rf=0,36; chromatographie sur couche mince de gel de silice; éluant mélange acétate d'éthyle/cyclohexane 1:1 en volumes)].

On isole aussi de cette colonne 1,4 g de 2,2-Diméthyl-4S-[5-(2,2,2',2'-tétraméthyl-[4R,4'R]bi[[1,3]dioxolanyl]-5S-yl)-pyrazin-2-ylméthyl]-[1,3]dioxan-5R-ol sous forme d'une huile jaune [Spectre de R.M.N. ¹H (400 MHz, (CD₃)₂SO d6, δ en ppm) : 0,95 - 1,18 - 1,28 et 1,44 (4 s, respectivement 3H - 6H - 3H et 6H : les 6 CH₃); 2,83 (dd, J = 13 et 9 Hz, 1H : 1H du CH₂ 5α); 3,29 (dd, J = 13 et 2,5 Hz, 1H : l'autre H du CH₂ 5α); de 3,30 à 3,40 (mt, 1H : CH 5γ); 3,53 (t, J = 11 Hz, 1H : 1H du CH₂ 5δ); 3,74 (dd, J = 11 et 5,5 Hz, 1H : l'autre H du CH₂ 5δ); 3,84 (dd, J = 9 et 4 Hz, 1H : 1 H du CH₂ 2δ); de 3,95 à 4,15 (mt, 2H : l'autre H du CH₂ 2δ et CH 5β); de 4,20 à 4,35 (mt, 2H : CH 2β et CH 2γ); 4,98 (d, J = 7 Hz, 1H : CH 2α); 5,20 (d, J = 6 Hz, 1H : OH en 5γ); 8,53 (s, 1H : =CH en 6); 8,66 ( s, 1H : =CH en 3); α_{D}²⁰= -23,8° ± 1,1 (c=0,5/ dichlorométhane); (Rf=0,28; chromatographie sur couche mince de gel de silice; éluant mélange acétate d'éthylelcyclohexane 1:1 en volumes)].

La désoxyfructosazine peut être préparée selon la méthode décrite par K. Sumoto et al. dans Chem. Pharm. Bull., *39*, 792 (1991).

### EXEMPLE 2

A 0,72 g de 2-(2,2,2',2'-tétraméthyl-[4R,4'R]bi[[1,3]dioxolanyl]-5S-yl)-5-(2-[2,2-diméthyl-[1,3]dioxolan-4S-yl]-E-éthényl)-pyrazine on ajoute 100 cm³ d'une solution aqueuse d'acide trifluoroacétique à 80%. Le mélange réactionnel est agité à une température d'environ 25°C pendant 2,5 heures. Après concentration sous pression réduite (2,7 kPa) à une température de 50°C, la pâte marron résiduelle est reprise dans un mélange de 10 cm³ d'éthanol et 1 cm³ d'eau et recristallisée. Le précipité obtenu est filtré sur verre fritté, rincé avec le même mélange froid, essoré puis séché dans un dessicateur sous pression réduite (2,7 kPa) à une température voisine de 60°C. On isole ainsi 200 mg de 1-[5-(3S,4-Dihydroxy-1E-butényl)-pyrazin-2-yl]-butane-1R,2S,3R,4-tétraol sous forme de cristaux de couleur blanc cassé fondant à 192°C [Spectre de R.M.N. ¹ H (400 MHz, (CD₃)₂SO d6, δ en ppm) : de 3,20 à 3,50 (mt, 3H : 1 H du CH₂O 2δ et CH₂O 5δ); de 3,55 à 3,70 (mt, 3H : l'autre H du CH₂O 2δ - CH 2β et CH 2γ); 4,23 (mt, 1H : CH 5γ); 4,39 (t, J = 6 Hz, 1H : OH en 2δ); 4,46 (d, J = 7 Hz, 1H : OH); 4,66 (d, J = 4 Hz, 1H : OH); 4,75 (t, J = 6 Hz, 1H : OH en 5δ); 4,96 (d large, J = 6,5 Hz, 1H : CH 2α); 5,12 (d, J = 5 Hz, 1H : OH en 5γ); 5,34 (d, J = 6,5 Hz, 1H : OH en 2α); 6,74 (d large, J = 16 Hz, 1H : =CH 5α); 6,91 (dd, J = 16 et 4,5 Hz, 1H : =CH 5β); 8,58 (s, 1H : =CH en 6); 8,66 (s, 1H : =CH en 3); α_{D}²⁰ = -29,7°+/-1,0 (c=0,5/ eau)]

La 2-(2,2,2',2'-tétraméthyl-[4R,4'R]bi[[1,3]dioxolanyl]-5S-yl)-5-(2-[2,2-diméthyl-[1,3]dioxolan-4S-yl]-E-éthenyl)-pyrazine est préparée selon la méthode suivante : A une solution de 0,5 g de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthanol dans 9,6 cm³ de tétrahydrofuranne sont successivement ajoutés 132 mg de succinimide et 340 mg de triphénylphosphine. Une solution de diéthyl azodicarboxylate à 40% dans le toluène (0,62 cm³) est ensuite ajoutée goutte à goutte et le mélange réactionnel est agité à une température voisine de 25°C pendant 4 heures à l'issue desquelles 396 mg de succinimide, 1,02 g de triphénylphosphine et 1,87 cm³ de solution de diéthyl azodicarboxylate à 40% dans le toluène sont rajoutés. Après 48 heures d'agitation, le mélange réactionnel est concentré sous pression réduite (2,7 kPa) à une température voisine de 45°C. Le résidu obtenu est chromatographié sur une colonne de silice (0,020-0,045 mm) éluée avec un mélange acétate d'éthyle/cyclohexane (1:4 puis 1:2,3 en volumes) à une pression d'environ 1,5x10⁵ Pa. Les fractions contenant le produit attendu sont réunies et concentrées sous pression réduite (2,7 kPa) à une température de 45°C. On obtient ainsi 340 mg de 2-(2,2,2',2'-tétraméthyl-[4R,4'R]bi[[1,3]dioxolanyl]-5S-yl)-5-(2-[2,2-diméthyl-[1,3]dioxolan-4S-yl]-E-éthenyl)-pyrazine sous forme d'une huile visqueuse blanchâtre (Rf=0,7; chromatographie sur couche mince de gel de silice; éluant acétate d'éthyle).

### EXEMPLE 3

A 0,45 g de 2-(2,2,2',2'-tétraméthyl-[4R,4'R]bi[[1,3]dioxolanyl]-5S-yl)-5-(2S-[2,2-diméthyl-[1,3]dioxolan-4R-yl]-2-méthoxyéthyl)-pyrazine on ajoute 62 cm³ d'une solution aqueuse d'acide trifluoroacétique à 80%. Le mélange réactionnel est agité à une température d'environ 25°C pendant 2 heures. Après concentration sous pression réduite (2,7 kPa) à une température de 65°C, la laque marron résiduelle est reprise 3 fois dans l'éther puis reconcentrée sous pression réduite (2,7 kPa) à une température de 45°C. La pâte résiduelle est reprise dans 4 cm³ d'éthanol et recristallisée. Le précipité obtenu est filtré sur verre fritté, lavé à l'éthanol, essoré puis séché sous pression réduite (2,7 kPa) à une température de 40°C. On isole ainsi 100 mg de 1-[5-(2S-Méthoxy-3R,4-dihydroxy-butyl)-pyrazin-2-yl]-butane-1R,2S,3R,4-tétraol sous forme d'une poudre cristalline beige fondant à 144°C [Spectre de R.M.N. ¹H (400 MHz, (CD₃)₂SO d6, δ en ppm) : 2,92 et 3,02 (2 dd, respectivement J = 14 et 8 Hz et J = 14 et 4 Hz, 1H chacun : CH₂ 5α); 3,14 (s, 3H : OCH₃ en 5β); de 3,30 à 3,50 et de 3,50 à 3,60 (2 mts, respectivement 2H et 6H : CH 2β - CH 2γ - CH₂O 2δ - CH 5β - CH 5γ et CH₂O 5δ); 4,39 et 4,53 (2 t, J = 5,5 Hz, 1H chacun : OH en 2δ et OH en 5δ); 4,45 (d, J = 7,5 Hz, 1H : OH); 4,65 (d large, J = 5 Hz, 1H : OH); 4,70 (d, J = 5 Hz, 1H : OH); 4,95 (d, J = 6,5 Hz, 1H : CH 2α); 5,31 (d, J = 6,5 Hz, 1H : OH en 2α); 8,43 (s large, 1H : =CH en 6); 8,65 (s large, 1H : =CH en 3)].

La 2-(2,2,2',2'-tétraméthyl-[4R,4'R]bi[[1,3]dioxolanyl]-5S-yl)-5-(2S-[2,2-diméthyl-[1,3]dioxolan-4R-yl]-2-méthoxyéthyl)-pyrazine est préparée selon la méthode suivante : A une solution de 1,0 g de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthanol dans 5 cm³ de diméthylformamide est ajoutée une suspension de 104 mg d'hydrure de sodium (à 60% dans l'huile) dans 5 cm³ de diméthylformamide. Le mélange réactionnel est agité à une température voisine de 25°C pendant 30 minutes, puis 0,15 cm³ d'iodure de méthyle est ajouté. Après 48 heures d'agitation, le mélange réactionnel est traité avec 25 cm³ d'eau et 25 cm³ d'acétate d'éthyle puis décanté. La phase aqueuse est extraite 2 fois avec 25 cm³ d'acétate d'éthyle et les extraits organiques sont réunis, séchés sur sulfate de magnésium, puis concentrés sous pression réduite (2,7 kPa) à une température voisine de 45°C. Le résidu obtenu est chromatographié sur sur une colonne de silice (0,020-0,045 mm) éluée avec un mélange acétate d'éthyle/cyclohexane (1:2,3 en volumes). Les fractions contenant le produit attendu sont réunies et concentrées sous pression réduite (2,7 kPa) à une température voisine de 50°C. On obtient ainsi 0,45 g de 2-(2,2,2',2'-tétraméthyl-[4R,4'R]bi[[1,3]dioxolanyl]-5S-yl)-5-(2S-[2,2-diméthyl-[1,3]dioxolan-4R-yl]-2-méthoxyéthyl)-pyrazine sous forme d'un solide beige (Rf=0,6; chromatographie sur couche mince de gel de silice; éluant mélange acétate d'éthyle/cyclohexane (1:1 en volumes)).

### EXEMPLE 4

A 0,58 g de 2-(2,2,2',2'-tétraméthyl-[4R,4'R]bi[[1,3]dioxolanyl]-5S-yl)-5-(2R-[2,2-diméthyl-[1,3]dioxolan-4R-yl]-2-fluoroéthyl)-pyrazine on ajoute 100 cm³ d'une solution aqueuse d'acide trifluoroacétique à 80%. Le mélange réactionnel est agité à une température d'environ 25°C pendant 16 heures. Après concentration sous pression réduite (2,7 kPa) à une température de 50°C, l'huile résiduelle est chromatographiée sur une colonne de silice (0,020-0,045 mm) éluée avec un mélange éthanol/n-butanol/solution aqueuse d'ammoniac (8/1/1 en volumes). Les fractions contenant les produits attendus sont réunies et concentrées sous pression réduite (2,7 kPa) à une température de 50°C. On obtient ainsi le 1-[5-(2R-fluoro-3R,4-dihydroxybutyl)-pyrazin-2-yl]-butane-1R,2S,3R,4-tétraol sous forme d'un solide couleur sable qui est repris dans un mélange de 1,2 cm³ d'éthanol et 0,2 cm³ d'eau et recristallisé. Le précipité obtenu est filtré sur verre fritté, lavé à l'éther diéthylique, essoré puis séché sous pression réduite (2,7 kPa) à une température de 60°C. On isole ainsi 62 mg de 1-[5-(2R-fluoro-3R,4-dihydroxy-butyl)-pyrazin-2-yl]-butane-1R,2S,3R,4-tétraol sous forme de cristaux blancs fondant à 172°C.[Spectre de R.M.N. ¹H (400 MHz, (CD₃)₂SO d6, à une température de 303 K, δ en ppm) : de 3,00 à 3,40 (mt, 2H : CH₂ 5α); de 3,40 à 3,70 (mt, 7H : CH 2β - CH 2γ - CH₂O 2δ - CH 5γ et CH₂O 5δ); 4,38 et 4,73 (2 t larges, J = 5 Hz, 1H chacun : OH en 2δ et OH en 5δ); 4,46 (d, J = 7 Hz, 1H: OH); 4,66 (d, J = 5 Hz, 1H : OH); 4,96 (d large, J = 5,5 Hz, 1H : CH 2α); 5,02 (d démultiplié, J_{HF} = 44 Hz, 1H : CH 5β); 5,35 (d, J = 5,5 Hz, 1H : OH en 2α); 8,47 (s large, 1H : =CH en 6); 8,69 (s large, 1H : =CH en 3)].

La 2-(2,2,2',2'-tétraméthyl-[4R,4'R]bi[[1,3]dioxolanyl]-5S-yl)-5-(2R-[2,2-diméthyl-[1,3]dioxolan-4R-yl]-2-fluoroéthyl)-pyrazine est préparée selon la méthode suivante : A une solution de 2,0 g de 1S-(2,2-Diméthyl-[1,3]dioxolan-4R-yl)-2-[5-(2,2,2',2'-tétraméthyl-[4R,4'R]bi[[1,3]dioxolanyl]-5S-yl)-pyrazin-2-yl]-éthanol dans 40 cm³ de tétrahydrofuranne refroidie à une température voisine de -78°C et sous azote, sont ajoutés au goutte à goutte 1,25 cm³ de trifluorure de diéthylaminosulfure. Le mélange réactionnel est laissé se réchauffer à une température d'environ -10°C. Après 4 heures à cette température de -10°C, le mélange réactionnel est traité avec 60 cm³ de méthanol puis agité pendant 30 minutes et concentré sous pression réduite (2,7 kPa) à une température voisine de 40°C. L'huile marron résiduelle est chromatographiée sur une colonne de silice (0,020-0,045 mm) éluée avec un mélange acétate d'éthyle/cyclohexane (1:3 en volumes) Les fractions contenant les produits attendus sont réunies et concentrées sous pression réduite (2,7 kPa) à une température voisine de 40°C. On obtient ainsi 0,67 g d'un mélange de 2-(2,2,2',2'-tétraméthyl-[4R,4'R]bi[[1,3]dioxolanyl]-5S-yl)-5-(2R-[2,2-diméthyl-[1,3]dioxolan-4R-yl]-2-fluoroéthyl)-pyrazine et de 2-(2,2,2',2'-tétraméthyl-[4R,4'R]bi[[1,3]dioxolanyl]-5S-yl)-5-{2S-[2,2-diméthyl-[1,3]dioxolan-4R-yl]-2-(4-fluorobutoxy)éthyl}-pyrazine dans les proportions approximatives 1:1 sous forme d'une huile incolore (Rf=0,3 pour les 2 produits; chromatographie sur couche mince de gel de silice; éluant mélange acétate d'éthyle/cyclohexane (1:1 en volumes)).

### EXEMPLE 5

A 0,36 g de 2-(2,2,2',2'-tétraméthyl-[4R,4'R]bi[[1,3]dioxolanyl]-5S-yl)-5-([5R-méthoxy-2,2-diméthyl-[1,3]dioxan-4S-yl]méthyl)-pyrazine on ajoute 51 cm³ d'une solution aqueuse d'acide trifluoroacétique à 80%. Le mélange réactionnel est agité à une température d'environ 25°C pendant 18 heures. Après concentration sous pression réduite (2,7 kPa) à une température voisine de 60°C, l'huile orange résiduelle est reprise dans 4 cm³ d'éthanol et recristallisée. Le précipité obtenu est filtré sur verre fritté, lavé à l'éthanol, essoré puis séché sous pression réduite (2,7 kPa) à une température voisine de 40°C. On isole ainsi 88 mg de 1-[5-(2S,4-dihydroxy-3R-méthoxy-butyl)-pyrazin-2-yl]-butane-1R,2S,3R,4-tétraol sous forme d'une poudre cristalline beige fondant à 96°C [Spectre de R.M.N. ¹H (400 MHz, (CD₃)₂SO d6, avec ajout de quelques gouttes de CD₃COOD d4, δ en ppm) : 2,79 et 2,97 (2 dd, respectivement J = 14 et 9 Hz et J = 14 et 3 Hz, 1H chacun : CH₂ 5α); 3,10 (mt, 1H : CH 5γ); 3,38 (s, 3H : OCH₃ en 5γ); de 3,40 à 3,70 (mt, 4H : CH 2β - CH 2γ et CH₂O 2δ); 3,50 et 3,64 (respectivement dd, J = 5 et 12 Hz et mt, 1H chacun : CH₂O 5δ); 3,93 (mt, 1H : CH 5β); 4,95 (s large, 1H : CH 2α); 8,40 (s, 1H : =CH en 6); 8,64 (s,1H : =CH en 3)].

La 2-(2,2,2',2'-tétraméthyl-[4R,4'R]bi[[1,3]dioxolanyl]-5S-yl)-5-([5R-méthoxy-2,2-diméthyl-[1,3]dioxan-4S-yl]méthyl)-pyrazine est préparée selon la méthode suivante : A une suspension de 0,14 g d'hydrure de sodium (à 60% dans l'huile) dans 10 cm³ de diméthylformamide refroidie à une température voisine de 0°C et sous azote, est ajoutée une solution de 1,24 g de 2,2-Diméthyl-4S-[5-(2,2,2',2'-tétraméthyl-[4R,4'R]bi[[1,3]dioxolanyl]-5S-yl)-pyrazin-2-ylméthyl]-[1,3]dioxan-5R-ol dans 15 cm³ de diméthylformamide. Le mélange réactionnel est agité à une température voisine de 0°C pendant 30 minutes, puis 0,19 cm³ d'iodure de méthyle est ajouté. Le mélange réactionnel est laissé se réchauffer à une température voisine de 25°C. Après 16 heures d'agitation, le mélange réactionnel est traité avec 25 cm³ d'eau et 25 cm³ d'acétate d'éthyle puis décanté. La phase aqueuse est extraite 2 fois avec 25 cm³ d'acétate d'éthyle et les extraits organiques sont réunis, séchés sur sulfate de magnésium, puis concentrés sous pression réduite (2,7 kPa) à une température de 50°C. Le résidu obtenu est chromatographié sur une colonne de silice (0,020-0,045 mm) éluée avec un mélange acétate d'éthyle/cyclohexane (1:2,3 en volumes). Les fractions contenant le produit attendu sont réunies et concentrées sous pression réduite (2,7 kPa) à une température de 50°C. On obtient ainsi 0,36 g de 2-(2,2,2',2'-tétraméthyl-[4R,4'R]bi[[1,3]dioxolanyl]-5S-yl)-5-([5R-méthoxy-2,2-diméthyl-[1,3]dioxan-4S-yl]méthyl)-pyrazine sous forme d'une huile incolore (Rf=0,5; chromatographie sur couche mince de gel de silice; éluant mélange acétate d'éthyle/cyclohexane (1:1 en volumes)).

### EXEMPLE 6

I- A 573 mg de 2-(1R,2S)-2-[(2R)-1,4-dioxaspiro[4.5]déc-2-yl]-1,2-diméthoxyéthyl-5-(2S)-2-[(2R)-1,4-dioxaspiro[4.5]déc-2-yl]-2-méthoxyéthyl-pyrazine on ajoute 12,5 cm³ d'une solution aqueuse d'acide trifluoroacétique à 80%. Le mélange réactionnel est agité à une température d'environ 25°C pendant 5,5 heures. Après concentration sous pression réduite (2,7 kPa) à une température de 45°C, l'huile résiduelle est reprise dans 10 cm³ de toluène et reconcentrée dans les mêmes conditions. L'huile obtenue est chromatographiée sur une colonne de silice (0,040-0,063 mm) éluée avec un mélange dichlorométhane/méthanol (95/5 en volumes), puis avec un mélange éthanol/n-butanol/solution aqueuse d'ammoniac (8/2/1 en volumes) à une pression de 1,6x10⁵ Pa. Les fractions contenant le produit attendu sont réunies et concentrées sous pression réduite (2,7 kPa) à une température de 50°C. L'huile ainsi obtenue est reprise dans l'éthanol, le solvant évaporé, puis le résidu est trituré plusieurs fois dans le dichlorométhane, puis repris dans le toluène. Le solvant est évaporé et le précipité est filtré pour donner un solide jaunâtre, qui est de nouveau trituré dans le dichlorométhane. On obtient ainsi 135 mg de 4-[5-(3R,4-dihydroxy-2S-méthoxy-butyl)]-pyrazin-2-yl]-3R,4R-diméthoxy-butane-1,2-diol sous forme d'un solide jaune pâle qui fond à 84°C [Spectre de R.M.N. ¹H (400 MHz, (CD₃)₂SO d6, δ en ppm) : 2,82 - 3,08 et 3,33 (3 s, 3H chacun : OCH₃); 2,93 et 3,05 (2 dd, respectivement J = 14 et 9 Hz et J = 14 et 4 Hz, 1H chacun : CH₂ 5α); de 3,30 à 3,50 (mt : les 3H correspondant aux CH₂O 5δ et CH 2β); de 3,50 à 3,70 (mt, 5H : CH 2γ - CH₂O 2δ - CH 5β et CH 5γ); 4,46 (t, J = 5,5 Hz, 1H : OH en 2δ); 4,55 (t, J = 5,5 Hz, 1H : OH en 5δ); 4,62 (d, J = 2,5 Hz, 1H : CH 2α); 4,81 (mt, 2H : OH en 2γ et OH en 5γ); 8,52 (s, 1H : =CH en 6); 8,56 (s,1H : =CH en 3)].
   La 2-(1R,2S)-2-[(2R)-1,4-dioxaspiro[4.5]déc-2-yl]-1,2-diméthoxyéthyl-5-(2S)-2-[(2R)-1,4-dioxaspiro[4.5]déc-2-yl]-2-méthoxyéthyl-pyrazine peut être préparée de la manière suivante : A une suspension de 150 mg d'hydrure de sodium dans 5 cm³ de diméthylformamide sous argon et à 0°C on ajoute une solution de 0,5 g de 2-(1R,2S)-2-[(2R)-1,4-dioxaspiro[4.5]déc-2-yl]-1,2-dihydroxyéthyl-5-(2S)-2-[(2R)-1,4-dioxaspiro[4.5]déc-2-yl]-2-dihydroxyéthyl pyrazine dans 7 cm³ de diméthylformamide. La suspension jaunâtre est maintenue ainsi sous agitation pendant 0,5 heure puis on ajoute lentement 0,21 cm³ d'iodure de méthyle. La température est ensuite remontée jusqu'à 15°C et on ajoute goutte à goutte 25 cm³ d'eau. Le mélange réactionnel est extrait par 3 fois 25 cm³ d'acétate d'éthyle. La phase organique est séchée sur sulfate de magnésium, filtrée puis concentrée sous pression réduite (2,7 kPa) à une température de 45°C. On obtient ainsi 0,58 g de 2-(1R,2S)-2-[(2R)-1,4-dioxaspiro[4.5]dec-2-yl]-1,2-diméthoxyéthyl-5-(2S)-2-[(2R)-1,4-dioxaspiro[4.5]dec-2-yl]-2-méthoxyéthyl-pyrazine sous forme d'une huile jaune, utilisée telle quelle dans l'étape suivante.
   La 2-(1R,2S)-2-[(2R)-1,4-dioxaspiro[4.5]déc-2-yl]-1,2-dihydroxyéthyl-5-(2S)-2-[(2R)-1,4-dioxaspiro[4.5]déc-2-yl]-2-dihydroxyéthyl pyrazine peut être obtenue de la manière suivante : à 500 mg de 2-[(1R,2S,3R) (1,2,3,4-tétrahydroxybutyl)]-5-[(2'S,3'R)-(2',3',4'-trihydroxybutyl)]-pyrazine en suspension dans 12 cm³ de diméthylformamide, sont successivement additionnés à une température voisine de 20°C, 2,54 cm³ de cyclohexanone et 31 mg d'acide para-toluènesulfonique monohydrate. La solution obtenue après 15 mn d'agitation à une température voisine de 20°C est de nouveau agitée pendant 2 heures 30 minutes à une température voisine de 20°C. On additionne ensuite du sulfate de magnésium et le milieu réactionnel est agité pendant 16 heures supplémentaires à une température voisine de 20°C. Le milieu est ensuite chauffé à une température voisine de 60°C pendant quelques minutes et devient blanc, laiteux. Le milieu réactionnel est laissé revenir à une température voisine de 20°C et est dilué avec un mélange de 10 cm³ d'eau distillée et 10 cm³ d'acétate d'éthyle. La phase organique, après décantation, est lavée avec deux fois 10 cm³ d'eau distillée. Les phases aqueuses sont réunies et extraite avec une fois 10 cm³ d'acétate d'éthyle. Les phases organiques réunies sont séchées sur sulfate de magnésium, filtrées sur verre fritté puis concentrées à sec sous pression réduite (0,27 kPa) à une température voisine de 30°C. On obtient ainsi 910 mg d'une meringue jaune pâle qui est reprise par 10 cm³ d'éther éthylique. Après 10 minutes d'agitation à une température voisine de 20°C, l'insoluble est filtré sur verre fritté, rincé avec 5 cm³ d'éther éthylique pour donner un produit blanc floconneux qui est séché à une température voisine de 40°C sous presion réduite (0,27 kPa). On obtient ainsi 417 mg de 2-(1R,2S)-2-[(2R)-1,4-dioxaspiro[4.5]déc-2-yl]-1,2-dihydroxyéthyl-5-(2S)-2-[(2R)-1,4-dioxaspiro[4.5]déc-2-yl]-2-dihydroxyéthyl pyrazine sous forme d'un solide blanc [Spectre de R.M.N. ¹H (400 MHz, (CD₃)₂SO d6, δ en ppm) : de 1,25 à 1,65 (mf, 20H : les 10 CH₂ des 2 cyclohexyles); 2,76 (dd, J = 14 et 9 Hz, 1H : 1H du CH₂ 5α); 3,04 (dd, J = 14 et 3,5 Hz, 1H : l'autre H du CH₂ 5α); 3,61 (dt, J = 7 et 1,5 Hz, 1H : CH 2β); 3,77 (mt, 1H : CH 5β); de 3,80 à 3,95 et de 3,95 à 4,10 (2 mts, respectivement 3H et 2H : CH 5γ - CH₂O 2d et CH₂O 5δ); 4,18 (mt, 1H : CH 2γ); 4,80 (mt, 2H : CH 2α et OH en 2β); 5,02 (d, J = 7 Hz, 1H : OH en 5β); 5,54 (d, J = 6,5 Hz, 1H : OH en 2α); 8,43 (s, 1H : =CH en 6); 8,65 (s, 1H : =CH en 3).II - La 4-[5-(3R,4-dihydroxy-2S-méthoxy-butyl)]-pyrazin-2-yl]-3R,4R-diméthoxy-butane-1,2-diol peut également être préparée de façon similaire mais à partir du 1-{5-[2(S)-Hydroxy-2-(2,2,4,4-tétraisopropyl-[1,3,5,2,4]trioxadisilepan-6(R)-yl)-éthyl]-pyrazin-2-yl}-2-(2,2,4,4-tétraisopropyl-[1,3,5,2,4]trioxadisilepan-6(R)-yl)-éthane-1(R),2(S)-diol/
   Le 1-{5-[2(S)-Hydroxy-2-(2,2,4,4-tétraisopropyl-[1,3,5,2,4]trioxadisilepan-6(R)-yl)-éthyl]-pyrazin-2-yl}-2-(2,2,4,4-tétraisopropyl-[1,3,5,2,4]trioxadisile pan-6(R)-yl)-éthane-1(R),2(S)-diol peut être obtenu de la manière suivante : A une solution de 1 g de désoxyfructosazine dans 27 cm³ de pyridine sous agitation et sous azote on ajoute 2,3 cm³ de 1,3-dichloro-1,1,3,3-tétraisopropyldisiloxane. Le mélange réactionnel est agité à une température d'environ 25°C pendant 41 heures. Après concentration sous pression réduite (2,7 kPa) à une température voisine de 60°C, l'huile résiduelle est reprise 3 fois dans 30 cm³ de pentane puis reconcentrée dans les mêmes conditions. Le résidu solide obtenu est dissous dans 50 cm³ de dichlorométhane et lavé successivement 2 fois avec 30 cm³ d'une solution aqueuse d'acide chlorhydrique 1N, 2 fois avec 30 cm³ d'eau, puis 2 fois avec 30 cm³ d'une solution aqueuse de chlorure de sodium saturée. La phase organique est séchée sur sulfate de magnésium puis concentrée sous pression réduite (2,7 kPa) à une température de 40°C. L'huile obtenue est chromatographiée sur une colonne de silice (0,020-0,045 mm) éluée avec un mélange acétate d'éthyle/cyclohexane (en gradient de 1:4 à 1:2 en volumes) à une pression d'environ 1,5x10⁵ Pa. Les fractions contenant le produit attendu sont réunies et concentrées sous pression réduite (2,7 kPa) à une température de 40°C. On obtient ainsi 1,5 g de 1-{5-[2(S)-Hydroxy-2-(2,2,4,4-tétraisopropyl-[1,3,5,2,4]trioxadisilepan-6(R)-yl)-éthyl]-pyrazin-2-yl}-2-(2,2,4,4-tétraisopropyl-[1,3,5,2,4]trioxadisile pan-6(R)-yl)-éthane-1(R),2(S)-diol sous forme d'une meringue blanche fondant à 87°C [Spectre de R.M.N. ¹H (250 MHz, (CD₃)₂SO d6, δ en ppm) : de 0,80 à 1,30 (mt, 56H : 8 CH(CH₃)₂); 2,78 (dd, J = 14 et 9 Hz, 1H : 1H du CH₂ 5α); 3,17 (dd, J = 14 et 3,5 Hz, 1H : l'autre H du CH₂ 5α); 3,53 (t large, J = 9 Hz, 1H : CH 2β); de 3,60 à 3,70 (mt, 4H : CH 5β - CH 5γ - 1H du CH₂O 2δ et 1 H du CH₂O 5δ); de 4,05 à 4,30 (mt, 3H : CH 2γ - l'autre H du CH₂O 2δ et l'autre H du CH₂O 5δ); 4,82 (d, J = 8 Hz, 1H : OH en 2β); de 4,90 à 5,05 (mt, 2H : CH 2α et OH 5β); 5,46 (d, J = 6 Hz, 1H : OH 2α); 8,43 (s, 1H : =CH en 6); 8,66 (s, 1H : =CH en 3); α_{D}²⁰ = - 11,1° ± 0,5 (c=0,5/ dichlorométhane)].
III - Le 4-[5-(3R,4-dihydroxy-2S-méthoxy-butyl)]-pyrazin-2-yl]-3R,4R-diméthoxy-butane-1,2-diol peut aussi être préparé d'une manière similaire à partir du 1-[5-(3,4-O-benzylidène-2S,3R,4-trihydroxy-butyl)-pyrazin-2-yl]-butane-3,4-O-benzylidène-1R,2S,3R,4-tétraol.

Le 1-[5-(3,4-O-benzylidène-2S,3R,4-trihydroxy-butyl)-pyrazin-2-yl]-butane-3,4-O-benzylidène-1R,2S,3R,4-tétraol peut être obtenu de la manière suivante : A une solution de 2,0 g de désoxyfructosazine dans 24 cm³ de diméthylformamide à une température voisine de 20°C sous argon sont ajoutés 20 mg d'acide camphorsulfonique-D. La suspension blanche est chauffée à 50°C pour donner une solution incolore, puis 5 cm³ de benzaldéhyde diméthylacétal sont ajoutés. La solution jaune est maintenue à 50°C sous agitation pendant 20 heures, puis laissée refroidir à température ambiante et 60 cm³ d'eau sont ajoutés. Le mélange est extrait par de l'acétate d'éthyle (2 fois) et lavé avec 20 cm³ d'une solution saturée d'hydrogénocarbonate de sodium, puis séché sur du sulfate de magnésium et filtré. Les phases organiques sont concentrées sous pression réduite (2,7 kPa) à une température de 40°C pour donner un résidu huileux orangé qui est chromatographié sur une colonne de silice (0,040-0,063 mm) en éluant avec un mélange dichlorométhane/méthanol (95/5 en volumes). Les fractions contenant le produit attendu sont réunies et concentrées sous pression réduite (2,7 kPa) à une température de 40°C pour donner 1,2 g de 1-[5-(3,4-O-benzylidène-2S,3R,4-trihydroxy-butyl)-pyrazin-2-yl]-butane-3,4-O-benzylidène-1R,2S,3R,4-tétraol pur en mélange diastéréoisomérique 4/6 sous forme d'une huile jaune pâle. Cette dernière donne un produit solide blanc qui fond à 70°C après trituration dans l'éther diéthylique [Spectre de R.M.N. ¹H (400 MHz, (CD₃)₂SO d6, δ en ppm). Nous observons le mélange de deux diastéréoisoméres dans des proportions approximatives 40/60 : 2,34 (dd large, J = 14 et 10 Hz, 1H : 1H du CH₂ 5α); 3,09 (d large, J = 14 Hz, 1H : l'autre H du CH₂ 5α); de 3,70 à 4,50 (mts, 8H : CH 2β - CH 2γ -CH₂O 2δ - CH 5β - CH 5γ et CH₂O 5δ); 4,83 (s large, 1H : CH 2α); 5,00 (d large, J = 5 Hz, 1H : OH en 2γ); 5,21 (d large, J = 3 Hz, 1H : OH en 5β); 5,69 (mt, 1H : OH en 2α); 5,77 - 5,79 et 5,90 (3 s, 2H en totalité : les 2 OCHO); 8,46 et 8,49 (2 s, 1H en totalité : =CH en 6); 8,70 (s, 1H : =CH en 3)].

Une autre série de fractions de la chromatographie donne le 1-[5-(2,4-O-benzylidène-2S,3R,4-trihydroxy-butyl)-pyrazin-2-yl]-butane-2,4-O-benzylidène-1R,2S,3R,4-tétraol.

### EXEMPLE 7

En opérant de la même manière que dans les exemples précédents, mais à partir de (2,2-diisopropyl-[1,3,2]dioxasilinan-5R-ol-4S-yl)-{5-[(2,2-diisopropyl-[1,3,2]dioxasilinan-5R-ol-4S-yl)méthyl]-pyrazin-2-yl}-méthane-1 R-ol, on obtient le 4-[5-(3R-Méthoxy-2S,4-dihydroxy-butyl)pyrazin-2-yl]-2R,4R-diméthoxy-butane-1,3S-diol.

Le (2,2-diisopropyl-[1,3,2]dioxasilinan-5R-ol-4S-yl)-{5-[(2,2-diisopropyl-[1,3,2]dioxasilinan-5R-ol-4S-yl)méthyl]-pyrazin-2-yl}-méthane-1R-ol peut être obtenu de la manière suivante : A une solution de 1 g de désoxyfructosazine dans 27 cm³ de pyridine sous agitation et sous azote on ajoute 2,1 cm³ de bis(trifluorométhanesulfonate) de diisopropylsilyle. Le mélange réactionnel est agité à une température d'environ 25°C pendant 71 heures. Après concentration sous pression réduite (2,7 kPa) à une température de 50°C, l'huile résiduelle est reprise 3 fois dans 30 cm³ de pentane puis reconcentrée dans les mêmes conditions. L'huile résiduelle obtenue est dissoute dans 50 cm³ de dichlorométhane et lavée successivement 2 fois avec 30 cm³ d'une solution aqueuse d'acide chlorhydrique 1N, 2 fois avec 30 cm³ d'eau, puis 2 fois avec 30 cm³ d'une solution aqueuse de chlorure de sodium saturée. La phase organique est séchée sur sulfate de magnésium puis concentrée sous pression réduite (2,7 kPa) à une température de 40°C. La meringue obtenue est chromatographiée sur une colonne de silice (0,020-0,045 mm) éluée avec un mélange acétate d'éthyle/cyclohexane (1:1 en volumes) à une pression d'environ 1,5x10⁵ Pa. Les fractions contenant le produit attendu sont réunies et concentrées sous pression réduite (2,7 kPa) à une température de 40°C. La meringue obtenue est chromatographiée sur une colonne de silice (0,020-0,045 mm) éluée avec un mélange acétate d'éthyle/cyclohexane (1:1 en volumes) à une pression d'environ 1,5x10⁵ Pa. Les fractions contenant le produit attendu sont réunies et concentrées sous pression réduite (2,7 kPa) à une température de 40°C. On isole ainsi 60 mg de (2,2-diisopropyl-[1,3,2]dioxasilinan-5R-ol-4S-yl)-{5-[(2,2-diisopropyl-[1,3,2]dioxasilinan-5R-ol-4S-yl)méthyl]-pyrazin-2-yl}-méthane-1R-ol sous forme d'une meringue blanche fondant à 54 °C [Spectre de R.M.N. ¹H (400 MHz, (CD₃)₂SO d6, δ en ppm) : de 0,65 à 1,15 (mt, 28H : 4 CH(CH₃)₂); 2,80 (dd, J = 14 et 9 Hz, 1H : 1H du CH₂ 5a); 3,28 (mt, l'autre H du CH₂ 5α); 3,40 (mt, 1H : CH 5γ); 3,68 (mt, 2H : 1H du CH₂O 2d et 1H du CH₂O 5δ); 3,83 (mt, 1H : CH 2γ); de 3,90 à 4,05 (mt, 3H : CH 2β - l'autre H du CH₂O 2δ et l'autre H du CH₂O 5δ); 4,13 (t large, J = 9,5 Hz, 1H : CH 5β); 4,99 (d large, J = 6,5 Hz, 1H : CH 2α); 5,22 (d, J = 6,5 Hz, 1H : OH en 2γ); 5,34 (d, J = 6 Hz, 1H : OH 5γ); 5,39 (d, J = 6,5 Hz, 1H : OH en 2α); 8,41 (s, 1H : =CH en 6); 8,70 (s, 1H : =CH en 3); α_{D}²⁰= -38,3° ± 0,8 (c=0,5/ dichlorométhane)].

### EXEMPLE 8

A 54 mg de 2-fluoro-1S-[5R-(5-{[5R-(hydroxyméthyl)-2,2-diméthyl-[1,3]dioxolan-4S-yl]méthyl}-pyrazin-2-yl)-2,2-diméthyl-[1,3]dioxolan-4R-yl]-éthanol on ajoute 8 cm³ d'une solution aqueuse d'acide trifluoroacétique à 80%. Le mélange réactionnel est agité à une température d'environ 25°C pendant 1,5 heure. Après concentration sous pression réduite (2,7 kPa) à une température de 45°C, le résidu est repris dans le toluène puis reconcentré dans les mêmes conditions (opération répétée une fois). L'huile brune résiduelle est dissoute dans 5 cm³ de méthanol, filtrée sur papier, puis reconcentrée dans les mêmes conditions. L'huile jaune résiduelle est reprise dans 0,4 cm³ d'éthanol et recristallisée. Le précipité obtenu est filtré sur verre fritté, lavé avec 0,1 cm³ d'éthanol, essoré puis séché sous pression réduite (2,7 kPa) à une température de 25°C. On isole ainsi 31 mg de 4-Fluoro-1-[5-(2S,3R,4-trihydroxy-butyl)-pyrazin-2-yl]-butane-1 R,2S,3R-triol sous forme d'une poudre cristalline beige fondant à 141°C. Spectre de R.M.N. ¹ H (400 MHz, (CD₃)₂SO d6, δ en ppm) : 2,76 et 3,08 (2 dd, respectivement J = 14 et 9 Hz et J = 14 et 2 Hz, 1H chacun : CH₂ 5α); de 3,30 à 3,45 (mt, 2H : 1H du CH₂O 5δ et CH 5γ); de 3,50 à 3,70 (mt, 1H : l'autre H du CH₂O 5δ); 3,64 (d large, J = 9 Hz, 1H : CH 2β); 3,80 (mt, 1H : CH 5β); de 3,70 à 3,90 (mt, 1H : CH 2γ); de 4,35 à 4,70 (mt, J_{HF} = 48 Hz, 2H : CH₂F 2δ); 4,97 (s large, 1H : CH en 2α); 8,44 (s large, 1H : =CH en 6); 8,67 (s large, 1H : =CH en 3).

Le 2-fluoro-1S-[5R-(5-{[5R-(hydroxyméthyl)-2,2-diméthyl-[1,3]dioxolan-4S-yl]méthyl}-pyrazin-2-yl)-2,2-diméthyl-[1,3]dioxolan-4R-yl]-éthanol est préparé selon la méthode suivante: A une solution de 210 mg de 2-fluoro-1S-[5R-(5-{[5R-(tert-butyl-diphényl-silanyloxyméthyl)-2,2-diméthyl-[1,3]dioxolan-4S-yl]méthyl}-pyrazin-2-yl)-2,2-diméthyl-[1,3]dioxolan-4R-yl]-éthanol dans 20 cm³ de tétrahydrofuranne sous argon, on ajoute au goutte à goutte 0,84 cm³ d'une solution 1,0 M de fluorure de tétra(n-butyl)ammonium dans le tétrahydrofuranne. Le mélange réactionnel est agité à une température voisine de 25°C pendant 1,5 heure. Après concentration sous pression réduite (2,7 kPa) à une température voisine de 40°C, l'huile ambrée résiduelle est chromatographiée sur une colonne de silice (0,040-0,063 mm) éluée avec un mélange acétate d'éthyle/cyclohexane 9:1 en volumes à une pression d'environ 1,5x10⁵ Pa. Les fractions contenant le produit attendu sont réunies et concentrées sous pression réduite (2,7 kPa) à une température de 40°C. On isole ainsi 54 mg de 2-fluoro-1S-[5R-(5-{[5R-(hydroxyméthyl)-2,2-diméthyl-[1,3]dioxolan-4S-yl]méthyl}-pyrazin-2-yl)-2,2-diméthyl-[1,3]dioxolan-4R-yl]-éthanol sous forme d'une huile jaune; (Rf=0,2; chromatographie sur couche mince de gel de silice; éluant mélange acétate d'éthyle/cyclohexane 9:1 en volumes).

Le 2-fluoro-1S-[5R-(5-{[5R-(tert-butyl-diphényl-silanyloxyméthyl)-2,2-diméthyl-[1,3]dioxolan-4S-yl]méthyl}-pyrazin-2-yl)-2,2-diméthyl-[1,3]dioxolan-4R-yl]-éthanol est préparé selon la méthode suivante : A une solution de 1,0 g de 2-(tert-Butyl-diphényl-silanyloxy)-1R-(5R-{5-[5R-(tert-butyl-diphényl-silanyloxyméthyl)-2,2-diméthyl-[1,3]dioxolan-4S-ylméthyl]-pyrazin-2-yl}-2,2-diméthyl-[1,3]dioxolan-4S-yl)-éthanol dans 6 cm³ de tétrahydrofuranne refroidie à une température de -45°C et sous argon, est ajouté au goutte à goutte 0,32 cm³ de trifluorure de diéthylaminosulfure. Le mélange réactionnel est agité à une température de -45°C pendant 2 heures, puis il est traité au goutte à goutte avec 0.2 cm³ de méthanol. Il est ensuite laissé se réchauffer à une température de 25°C puis concentré sous pression réduite (2,7 kPa) à une température de 40°C. L'huile brune résiduelle est dissoute dans 50 cm³ de dichlorométhane et extraite 3 fois avec 5 cm³ d'eau. La phase organique est séchée sur sulfate de magnésium puis concentré sous pression réduite (2,7 kPa) à une température de 40°C. L'huile brune résiduelle est chromatographiée sur une colonne de silice (0,020-0,045 mm) éluée avec un mélange acétate d'éthyle/cyclohexane 1:9 en volumes à une pression d'environ 1,5x10⁵ Pa. Les fractions contenant le produit attendu sont réunies et concentrées sous pression réduite (2,7 kPa) à une température de 40°C. On obtient ainsi 0,17 g de 2-fluoro-1S-[5R-(5-{[5R-(tert-butyl-diphényl-silanyloxyméthyl)-2,2-diméthyl-[1,3]dioxolan-4S-yl]méthyl}-pyrazin-2-yl)-2,2-diméthyl-[1,3]dioxolan-4R-yl]-éthanol sous forme d'une huile visqueuse jaune; (Rf=0,3; chromatographie sur couche mince de gel de silice; éluant mélange acétate d'éthyle/cyclohexane 1:2,3 en volumes).

Le 2-(tert-Butyl-diphényl-silanyloxy)-1R-(5R-{5-[5R-(tert-butyl-diphényl-silanyloxyméthyl)-2,2-diméthyl-[1,3]dioxolan-4S-ylméthyl]-pyrazin-2-yl}-2,2-diméthyl-[1,3]dioxolan-4S-yl)-éthanol est préparé selon la méthode suivante : A une solution de 1 g de 1-[5-(4-(tert-butyl-diphényl-silanyloxy)-2S,3R-dihydroxy-butyl)-pyrazin-2-yl]-4-[tert-butyl-diphényl-silanyloxy]-butane-1R,2S,3R-triol dans 50 cm³ de diméthylformamide sous agitation on ajoute 5 cm³ de 2,2-diméthoxypropane puis 0,01 g d'acide para-toluènesulfonique. Le mélange réactionnel est agité à une température de 60°C pendant 42 heures. Après concentration sous pression réduite (2,7 kPa) à une température de 60°C, l'huile résiduelle est dissoute dans 60 cm³ de dichlorométhane et lavée 2 fois avec 20 cm³ d'une solution aqueuse de bicarbonate de sodium à 5% puis 2 fois avec 20 cm³ d'eau. La phase organique est séchée sur sulfate de magnésium puis concentrée sous pression réduite (2,7 kPa) à une température de 40°C. L'huile obtenue est chromatographiée sur une colonne de silice (0,020-0,045 mm) éluée avec un mélange acétate d'éthyle/cyclohexane (1:9 en volumes) à une pression d'environ 1,5x10⁵ Pa. Les fractions contenant les produits attendus sont réunies et concentrées sous pression réduite (2,7 kPa) à une température de 40°C. On obtient ainsi 0,3 g de 2-(tert-Butyl-diphényl-silanyloxy)-1R-(5R-{5-[5R-(tert-butyl-diphényl-silanyloxyméthyl)-2,2-diméthyl-[1,3]dioxolan-4S-ylméthyl]-pyrazin-2-yl}-2,2-diméthyl-[1,3]dioxolan-4S-yl)-éthanol sous forme d'une huile jaune visqueuse [Spectre de R.M.N. ¹H (400 MHz, (CD₃)₂SO d6, δ en ppm) : 0,90 et 1,01 (2 s, 9H chacun : les 2 C(CH₃)₃); 1,22 - 1,35 - 1,44 et 1,46 (4 s, 3H chacun : les 4 CH₃); 3,08 (dd, J = 14 et 9 Hz, 1H : 1H du CH₂ 5α); 3,15 (dd, J = 14 et 3,5 Hz, 1H : l'autre H du CH₂ 5α); 3,60 (AB limite, 2H : CH₂O 2δ); 3,71 (dd, J = 11 et 5 Hz, 1H : 1H du CH₂O 5δ); de 3,80 à 3,95 (mt, 2H : l'autre H du CH₂ 5δ et CH 2γ); 4,32 (q, J = 5 Hz, 1H : CH 5γ); 4,48 (dd, J = 7 et 5 Hz, 1H : CH 2β); 4,70 (mt, 1H: CH 5β); 5,14 (d, J = 7 Hz, 1H : CH 2α); 5,19 (d, J = 5 Hz, 1H : OH en 2γ); de 7,35 à 7,80 (mt, 20H : H aromatiques des 4 phényles); 8,53 (s, 1H : =CH en 6); 8,68 (s, 1H : =CH en 3); α_{D}²⁰= -32,6° ± 0,8 (c=0,5/dichlorométhane); (Rf=0,30; chromatographie sur couche mince de gel de silice; éluant mélange acétate d'éthyle/cyclohexane 1:4 en volumes)].

On isole aussi de cette colonne 0,2 g de 4R-(tert-Butyl-diphényl-silanyloxyméthyl)-6R-{5-[5R-(tert-butyl-diphényl-silanyloxyméthyl)-2,2-diméthyl-[1,3]dioxolan-4S-ylméthyl]-pyrazin-2-yl}-2,2-diméthyl-[1,3]dioxan-5S-ol sous forme d'une huile jaune [Spectre de R.M.N. ¹H (400 MHz, (CD₃)₂SO d6, δ en ppm) : 1,01 et 1,05 (2s, 9H chacun : les 2 C(CH₃)₃); 1,24 - 1,36 et 1,50 (3 s, respectivement 3H - 6H et 3H : les 4 CH₃); 3,03 (dd, J = 14 et 9 Hz, 1H : 1H du CH₂ 5α); 3,12 (dd, J = 14 et 3,5 Hz, 1H : l'autre H du CH₂ 5α); 3,70 (dd, J = 11 et 5,5 Hz, 1H : 1H du CH₂O 5δ); de 3,75 à 3,95 (mt, 4H : CH 2γ - CH₂O 2δ et l'autre H du CH₂O 5δ); 4,03 (mt, 1H : CH 2β); 4,32 (mt, 1H : CH 5γ); 4,70 (mt, 1H : CH 5β); de 4,95 à 5,05 (mt, 2H : CH 2α et OH 2β); de 7,35 à 7,55 et de 7,60 à 7,80 (2 mts, respectivement 12H et 8H : H aromatiques des 4 phényles); 8,46 (s, 1H : =CH en 6); 8,53 (s, 1H : =CH en 3); α_{D}²⁰= -47,5° ±1,5 (c=0,5/ dichlorométhane); (Rf=0,24; chromatographie sur couche mince de gel de silice; éluant mélange acétate d'éthyle/cyclohexane (1:4 en volumes)).

Le 1-[5-(4-(tert-butyl-diphényl-silanyloxy)-2S,3R-dihydroxy-butyl)-pyrazin-2-yl]-4-[tert-butyl-diphényl-silanyloxy]-butane-1R,2S,3R-triol est préparé selon la méthode suivante : A une solution de 2 g de désoxyfructosazine dans 55 cm³ de pyridine sous agitation on ajoute 4,3 cm³ de tertbutyldiphénylchlorosilane. Le mélange réactionnel est agité à une température d'environ 25°C pendant 68 heures, puis 0,5 cm³ de terbutyldiphénylchlorosilane est ajouté et l'agitation est poursuivie pendant 8 jours. Après concentration sous pression réduite (2,7 kPa) à une température de 60°C, l'huile résiduelle est dissoute dans 100 cm³ de dichlorométhane et lavée 3 fois avec 30 cm³ d'eau. La phase organique est séchée sur sulfate de magnésium puis concentrée sous pression réduite (2,7 kPa) à une température de 40°C. L'huile obtenue est chromatographiée sur une colonne de silice (0,020-0,045 mm) éluée avec un mélange méthanol/dichlorométhane (1:49 en volumes) à une pression d'environ 1,5x10⁵ Pa. Les fractions contenant le produit attendu sont réunies et concentrées sous pression réduite (2,7 kPa) à une température de 40°C. On obtient ainsi 4 g de 1-[5-(4-(tert-butyl-diphényl-silanyloxy)-2(S),3(R)-dihydroxy-butyl)-pyrazin-2-yl]-4-[tert-butyl-diphényl-silanyloxy]-butane-1(R),2(S),3(R)-triol sous forme d'une meringue jaune (Rf=0,13; chromatographie sur couche mince de gel de silice; éluant mélange méthanol/dichlorométhane (1:19 en volumes)).

Les composés de formule (I) présentent des propriétés pharmacologiques intéressantes. Ce sont des hypoglycémiants.

L'activité hypoglycémiante des composés de formule (I) a été déterminée sur la réponse hyperglycémique à l'administration de glucose par la voie orale chez la souris normoglycémique, selon le protocole suivant : Des souris Swiss albinos pesant entre 22 et 26 g sont laissées à jeun pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des composés selon l'invention.

Les souris ainsi choisies sont réparties en groupes d'au moins 10 animaux. Des groupes distincts reçoivent une solution de 3 à 50 mg/kg du produit à tester dans un véhicule tel que l'eau ou un mélange de méthylcellulose/tween et eau ou du véhicule une fois par jour par tubage gastrique. Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les composés selon l'invention présentent un pourcentage d'inhibition de la glycémie supérieur ou égal à 10%.

Les composés de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type Il (NID diabète), du diabète de l'obèse, du diabète de la cinquantaine, du diabète métapléthorique, du diabète du sujet âgé et du diabète léger. Ils peuvent être utilisés en complément de l'insulinothérapie dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt artérioveineux, dilatation veineuse, hémorragies ponctiformes et maculaires, exudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcéres des extrémités inférieures et du pied), la néphropathie diabétique dans ses deux formes diffuse et nodulaire, l'athéromatose (élévation des HDL lipoproteines favorisant l'élimination du cholestérol à partir des plaques d'athérome, baisse des LDL lipoproteines, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéines A et B), de la cataracte, de l'hypertension artérielle et ses conséquences.

Les médicaments selon l'invention sont constitués par un composé selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

L'invention concerne également l'utilisation des composés de formule générale (I) pour la préparation de compositions pharmaceutiques utiles pour le traitement ou la prévention du diabète et les complications du diabète.

## Revendications

1. Médicaments contenant en tant que principe actif au moins un composé de formule : 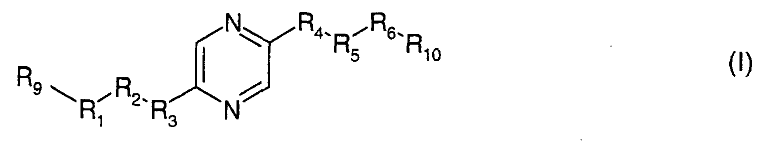 dans laquelle
R₉ et R₁₀ sont identiques et représentent un radical -CH₂OH, et soit
a) R₃ représenté un radical méthylène, R₄ représente un radical -CHOH- et l'un des radicaux R₁, R₂, R₅ et R₆ représente un radical carbonyle, méthylène, -CHF-, -CH(NHR₇)- ou -CH(OR₈) et les autres représentent chacun un radical -CHOH-,
b) R₂ et R₅ représentent chacun un radical -CHOH-, R₃ représente un radical méthylène et R₁, R₄ et R₆ sont identiques et représentent chacun un radical carbonyle, méthylène, -CHF-, -CH(NHR₇)- ou -CH(OR₈),
c) R₁ et R₆ représentent chacun un radical -CHOH-, R₃ représente un radical méthylène et R₂, R₄ et R₅ sont identiques et représentent chacun un radical carbonyle, méthylène, -CHF-, -CH(NHR₇)- ou -CH(OR₈),
d) R₁, R₄, R₅ et R₆ représentent chacun un radical -CHOH- et -R₂-R₃-représente un radical -CH=CH-,
R₇ représente un atome d'hydrogène ou un radical alkyle, -CO-alk, -CO-Ar ou -CO-Het,
R₈ représente un radical alkyle, -alk-COOH ou -alk-OH,
alk représente un radical alkyle,
Ar représente un radical phényle ou phényle substitué par un ou plusieurs substituants choisis parmi un atome d'halogène, un radical alkyle, alcoxy, alcoxycarbonyle, amino, monoalkylamino ou dialkylamino,
Het représente un hétérocycle mono, di ou tricyclique saturé ou insaturé contenant 1 à 9 atomes de carbone et un ou plusieurs hétéroatomes choisis parmi oxygène, soufre et azote,
les radicaux et portions alkyle et alcoxy contenant 1 à 6 atomes de carbone en chaîne droite ou ramifiée,
ou un de ses stéréoisomères ou pour le composé pour lequel -R₂-R₃-représente un radical -CH=CH- ses formes cis ou trans ou un de ses sels avec un acide minéral ou organiques pharmaceutiquement acceptable.

2. Médicaments selon la revendication 1 pour lesquels dans les composés de formule (I) Het représente un hétérocycle choisi parmi les cycles 2-, 3-, ou 4-pyridyle, imidazolyle, thiazolyle et oxazolyle.

3. Médicaments selon la revendication 1 pour lesquels le composé de formule (I) est choisi parmi les composés suivants :
1-[5-(3R,4-Dihydroxy-2-oxo-butyl)-pyrazin-2-yl]-butane-1R,2S,3R,4-tétraol,
1-[5-(3S,4-Dihydroxy-butyl)-pyrazin-2-yl]-butane-1R,2S,3R,4-tétraol,
1-[5-(2S-Fluoro-3R,4-dihydroxy-butyl)-pyrazin-2-yl]-butane-1R,2S,3R,4-tétraol,
1-[5-(2R-Fluoro-3R,4-dihydroxy-butyl)-pyrazin-2-yl]-butane-1R,2S,3R,4-tétraol,
1-[5-(2S-Amino-3S,4-dihydroxy-butyl)-pyrazin-2-yl]-butane-1R,2S,3R,4-tétraol,
1-[5-(2R-Amino-3S,4-dihydroxy-butyl)-pyrazin-2-yl]-butane-1R,2S,3R,4-tétraol,
1-[5-(2S-(N-Méthyl)amino-3S,4-dihydroxy-butyl)-pyrazin-2-yl]-butane-1R,2S,3R,4-tétraol,
1-[5-(2R-(N-Méthyl)amino-3S,4-dihydroxy-butyl)-pyrazin-2-yl]-butane-1R,2S,3R,4-tétraol,
1-[5-(2S-(N-Ethyl)amino-3S,4-dihydroxy-butyl)-pyrazin-2-yl]-butane-1R,2S,3R,4-tétraol,
1-[5-(2R-(N-Ethyl)amino-3S,4-dihydroxy-butyl)-pyrazin-2-yl]-butane-1R,2S,3R,4-tétraol,
1-[5-(2S-(N-n-Butyl)amino-3S,4-dihydroxy-butyl)-pyrazin-2-yl]-butane-1R,2S,3R,4-tétraol,
1-[5-(2R-(N-n-Butyl)amino-3S,4-dihydroxy-butyl)-pyrazin-2-yl]-butane-1R,2S,3R,4-tétraol,
1-[5-(2S-(N-Benzyl)amino-3S,4-dihydroxy-butyl)-pyrazin-2-yl]-butane-1R,2S,3R,4-tétraol,
1-[5-(2R-(N-Benzyl)amino-3S,4-dihydroxy-butyl)-pyrazin-2-yl]-butane-1R,2S,3R,4-tétraol,
1-[5-(2S-(N-Acétyl)amino-3S,4-dihydroxy-butyl)-pyrazin-2-yl]-butane-1R,2S,3R,4-tétraol,
1-[5-(2R-(N-Acétyl)amino-3S,4-dihydroxy-butyl)-pyrazin-2-yl]-butane-1R,2S,3R,4-tétraol,
1-[5-(2S-(N-Butanoyl)amino-3S,4-dihydroxy-butyl)-pyrazin-2-yl]-butane-1R,2S,3R,4-tétraol,
1-[5-(2R-(N-Butanoyl)amino-3S,4-dihydroxy-butyl)-pyrazin-2-yl]-butane-1R,2S,3R,4-tétraol,
1-[5-(2S-(N-Benzoyl)amino-3S,4-dihydroxy-butyl)-pyrazin-2-yl]-butane-1R,2S,3R,4-tétraol,
1-[5-(2R-(N-Benzoyl)amino-3S,4-dihydroxy-butyl)-pyrazin-2-yl]-butane-1R,2S,3R,4-tétraol,
1-[5-(2S-Méthoxy-3R,4-dihydroxy-butyl)-pyrazin-2-yl]-butane-1R,2S,3R,4-tétraol,
1-[5-(2R-Méthoxy-3R,4-dihydroxy-butyl)-pyrazin-2-yl]-butane-1R,2S,3R,4-tétraol,
1-[5-(2S-Ethoxy-3R,4-dihydroxy-butyl)-pyrazin-2-yl]-butane-1R,2S,3R,4-tétraol,
1-[5-(2R-Ethoxy-3R,4-dihydroxy-butyl)-pyrazin-2-yl]-butane-1R,2S,3R,4-tétraol,
1-[5-(2S-n-Butoxy-3S,4-dihydroxy-butyl)-pyrazin-2-yl]-butane-1R,2S,3R,4-tétraol,
1-[5-(2R-n-Butoxy-3R,4-dihydroxy-butyl)-pyrazin-2-yl]-butane-1R,2S,3R,4-tétraol,
1-[5-(2S-(2-Hydroxyéthyl)oxy-3R,4-dihydroxy-butyl)-pyrazin-2-yl]-butane-1R,2S,3R,4-tétraol,
1-[5-(2R-(2-Hydroxyéthyl)oxy-3R,4-dihydroxy-butyl)-pyrazin-2-yl]-butane-1R,2S,3R,4-tétraol,
1-[5-(2S-(3-Hydroxy-n-propyl)oxy-3R,4-dihydroxy-butyl)-pyrazin-2-yl]-butane-1R,2S,3R,4-tétraol,
1-[5-(2R-(3-Hydroxy-n-propyl)oxy-3R,4-dihydroxy-butyl)-pyrazin-2-yl]-butane-1R,2S,3R,4-tétraol,
1-[5-(2S-(Carboxyméthyl)oxy-3R,4-dihydroxy-butyl)-pyrazin-2-yl]-butane-1R,2S,3R,4-tétraol,
1-[5-(2R-(Carboxyméthyl)oxy-3R,4-dihydroxy-butyl)-pyrazin-2-yl]-butane-1R,2S,3R,4-tétraol,
1-[5-(2S-(3-Carboxy-n-propyl)oxy-3R,4-dihydroxy-butyl)-pyrazin-2-yl]-butane-1R,2S,3R,4-tétraol,
1-[5-(2R-(3-Carboxy-n-propyl)oxy-3R,4-dihydroxy-butyl)-pyrazin-2-yl]-butane-1R,2S,3R,4-tétraol,
1-[5-(2S,4-Dihydroxy-3-oxo-butyl)-pyrazin-2-yl]-butane-1R,2S,3R,4-tétraol,
1-[5-(2S,4-Dihydroxy-butyl)-pyrazin-2-yl]-butane-1R,2S,3R,4-tétraol,
1-[5-(3R-Fluoro-2S,4-dihydroxy-butyl)-pyrazin-2-yl]-butane-1R,2S,3R,4-tétraol,
1-[5-(3S-Fluoro-2S,4-dihydroxy-butyl)-pyrazin-2-yl]-butane-1R,2S,3R,4-tétraol,
1-[5-(3R-Amino-2R,4-dihydroxy-butyl)-pyrazin-2-yl]-butane-1R,2S,3R,4-tétraol,
1-[5-(3S-Amino-2R,4-dihydroxy-butyl)-pyrazin-2-yl]-butane-1R,2S,3R,4-tétraol,
1-[5-(3R-(N-Méthyl)amino-2R,4-dihydroxy-butyl)-pyrazin-2-yl]-butane-1R,2S,3R,4-tétraol,
1-[5-(3S-(N-Méthyl)amino-2R,4-dihydroxy-butyl)-pyrazin-2-yl]-butane-1R,2S,3R,4-tétraol,
1-[5-(3R-(N-Ethyl)amino-2R,4-dihydroxy-butyl)-pyrazin-2-yl]-butane-1R,2S,3R,4-tétraol,
1-[5-(3S-(N-Ethyl)amino-2R,4-dihydroxy-butyl)-pyrazin-2-yl]-butane-1R,2S,3R,4-tétraol,
1-[5-(3R-(N-n-Butyl)amino-2R,4-dihydroxy-butyl)-pyrazin-2-yl]-butane-1R,2S,3R,4-tétraol,
1-[5-(3S-(N-n-Butyl)amino-2R,4-dihydroxy-butyl)-pyrazin-2-yl]-butane-1R,2S,3R,4-tétraol,
1-[5-(3R-(N-Benzyl)amino-2R,4-dihydroxy-butyl)-pyrazin-2-yl]-butane-1R,2S,3R,4-tétraol,
1-[5-(3S-(N-Benzyl)amino-2R,4-dihydroxy-butyl)-pyrazin-2-yl]-butane-1R,2S,3R,4-tétraol,
1-[5-(3R-(N-Acétyl)amino-2R,4-dihydroxy-butyl)-pyrazin-2-yl]-butane-1R,2S,3R,4-tétraol,
1-[5-(3S-(N-Acétyl)amino-2R,4-dihydroxy-butyl)-pyrazin-2-yl]-butane-1R,2S,3R,4-tétraol,
1-[5-(3R-(N-Butanoyl)amino-2R,4-dihydroxy-butyl)-pyrazin-2-yl]-butane-1R,2S,3R,4-tétraol,
1-[5-(3S-(N-Butanoyl)amino-2R,4-dihydroxy-butyl)-pyrazin-2-yl]-butane-1R,2S,3R,4-tétraol,
1-[5-(3R-(N-Benzoyl)amino-2R,4-dihydroxy-butyl)-pyrazin-2-yl]-butane-1R,2S,3R,4-tétraol,
1-[5-(3S-(N-Benzoyl)amino-2R,4-dihydroxy-butyl)-pyrazin-2-yl]-butane-1R,2S,3R,4-tétraol,
1-[5-(3R-Méthoxy-2S,4-dihydroxy-butyl)-pyrazin-2-yl]-butane-1R,2S,3R,4-tétraol,
1-[5-(3S-Méthoxy-2S,4-dihydroxy-butyl)-pyrazin-2-yl]-butane-1R,2S,3R,4-tétraol,
1-[5-(3R-Ethoxy-2S,4-dihydroxy-butyl)-pyrazin-2-yl]-butane-1R,2S,3R,4-tétraol,
1-[5-(3S-Ethoxy-2S,4-dihydroxy-butyl)-pyrazin-2-yl]-butane-1R,2S,3R,4-tétraol,
1-[5-(3R-n-Butoxy-2S,4-dihydroxy-butyl)-pyrazin-2-yl]-butane-1R,2S,3R,4-tétraol,
1-[5-(3S-n-Butoxy-2S,4-dihydroxy-butyl)-pyrazin-2-yl]-butane-1R,2S,3R,4-tétraol,
1-[5-(3R-(2-Hydroxyéthyl)oxy-2S,4-dihydroxy-butyl)-pyrazin-2-yl]-butane-1R,2S,3R,4-tétraol,
1-[5-(3S-(2-Hydroxyéthyl)oxy-2S,4-dihydroxy-butyl)-pyrazin-2-yl]-butane-1R,2S,3R,4-tétraol,
1-[5-(3R-(3-Hydroxy-n-propyl)oxy-2S,4-dihydroxy-butyl)-pyrazin-2-yl]-butane-1R,2S,3R,4-tétraol,
1-[5-(3S-(3-Hydroxy-n-propyl)oxy-2S,4-dihydroxy-butyl)-pyrazin-2-yl]-butane-1R,2S,3R,4-tétraol,
1-[5-(3R-(Carboxyméthyl)oxy-2S,4-dihydroxy-butyl)-pyrazin-2-yl]-butane-1R,2S,3R,4-tétraol,
1-[5-(3S-(Carboxyméthyl)oxy-2S,4-dihydroxy-butyl)-pyrazin-2-yl]-butane-1R,2S,3R,4-tétraol,
1-[5-(3R-(3-Carboxy-n-propyl)oxy-2S,4-dihydroxy-butyl)-pyrazin-2-yl]-butane-1R,2S,3R,4-tétraol,
1-[5-(3S-(3-Carboxy-n-propyl)oxy-2S,4-dihydroxy-butyl)-pyrazin-2-yl]-butane-1R,2S,3R,4-tétraol,
4-[5-(2S,3R,4-Trihydroxy-butyl)-pyrazin-2-yl]-1,3S,4R-trihydroxy-butane-2-one,
1-[5-(2S,3R,4-Trihydroxy-butyl)-pyrazin-2-yl]-butane-1R,2R,4-triol,
1-[5-(2S,3R,4-Trihydroxy-butyl)-pyrazin-2-yl]-3R-fluoro-butane-1R,2S,4-triol,
1-[5-(2S,3R,4-Trihydroxy-butyl)-pyrazin-2-yl]-3S-fluoro-butane-1R,2S,4-triol,
1-[5-(2S,3R,4-Trihydroxy-butyl)-pyrazin-2-yl]-3R-amine-butane-1R,2R,4-triol,
1-[5-(2S,3R,4-Trihydroxy-butyl)-pyrazin-2-yl]-3S-amino-butane-1R,2R,4-triol,
1-[5-(2S,3R,4-Trihydroxy-butyl)-pyrazin-2-yl]-3R-(N-méthyl)amino-butane-1R,2R,4-triol,
1-[5-(2S,3R,4-Trihydroxy-butyl)-pyrazin-2-yl]-3S-(N-méthyl)amino-butane-1R,2R,4-triol,
1-[5-(2S,3R,4-Trihydroxy-butyl)-pyrazin-2-yl]-3R-(N-éthyl)amino-butane-1R,2R,4-triol,
1-[5-(2S,3R,4-Trihydroxy-butyl)-pyrazin-2-yl]-3S-(N-éthyl)amino-butane-1R,2R,4-triol,
1-[5-(2S,3R,4-Trihydroxy-butyl)-pyrazin-2-yl]-3R-(N-n-butyl)amino-butane-1R,2R,4-triol,
1-[5-(2S,3R,4-Trihydroxy-butyl)-pyrazin-2-yl]-3S-(N-n-butyl)amino-butane-1R,2R,4-triol,
1-[5-(2S,3R,4-Trihydroxy-butyl)-pyrazin-2-yl]-3R-(N-benzyl)amino-butane-1R,2R,4-triol,
1-[5-(2S,3R,4-Trihydroxy-butyl)-pyrazin-2-yl]-3S-(N-benzyl)amino-butane-1R,2R,4-triol,
1-[5-(2S,3R,4-Trihydroxy-butyl)-pyrazin-2-yl]-3R-(N-acétyl)amino-butane-1R,2R,4-triol,
1-[5-(2S,3R,4-Trihydroxy-butyl)-pyrazin-2-yl]-3S-(N-acétyl)amino-butane-1R,2R,4-triol,
1-[5-(2S,3R,4-Trihydroxy-butyl)-pyrazin-2-yl]-3R-(N-Butanoyl)amino-butane-1R,2R,4-triol,
1-[5-(2S,3R,4-Trihydroxy-butyl)-pyrazin-2-yl]-3S-(N-Butanoyl)amino-butane-1R,2R,4-triol,
1-[5-(2S,3R,4-Trihydroxy-butyl)-pyrazin-2-yl]-3R-(N-Benzoyl)amino-butane-1R,2R,4-triol,
1-[5-(2S,3R,4-Trihydroxy-butyl)-pyrazin-2-yl]-3S-(N-Benzoyl)amino-butane-1R,2R,4-triol,
1-[5-(2S,3R,4-Trihydroxy-butyl)-pyrazin-2-yl]-3R-méthoxy-butane-1R,2S,4-triol,
1-[5-(2S,3R,4-Trihydroxy-butyl)-pyrazin-2-yl]-3S-méthoxy-butane-1R,2S,4-triol,
1-[5-(2S,3R,4-Trihydroxy-butyl)-pyrazin-2-yl]-3R-éthoxy-butane-1R,2S,4-triol,
1-[5-(2S,3R,4-Trihydroxy-butyl)-pyrazin-2-yl]-3S-éthoxy-butane-1R,2S,4-triol,
1-[5-(2S,3R,4-Trihydroxy-butyl)-pyrazin-2-yl]-3R-n-butoxy-butane-1R,2S,4-triol,
1-[5-(2S,3R,4-Trihydroxy-butyl)-pyrazin-2-yl]-3S-n-butoxy-butane-1R,2S,4-triol,
1-[5-(2S,3R,4-Trihydroxy-butyl)-pyrazin-2-yl]-3R-(2-hydroxyéthyl)oxy-butane-1R,2S,4-triol,
1-[5-(2S,3R,4-Trihydroxy-butyl)-pyrazin-2-yl]-3S-(2-hydroxyéthyl)oxy-butane-1R,2S,4-triol,
1-[5-(2S,3R,4-Trihydroxy-butyl)-pyrazin-2-yl]-3R-(3-hydroxy-n-propyl)oxy-butane-1R,2S,4-triol,
1-[5-(2S,3R,4-Trihydroxy-butyl)-pyrazin-2-yl]-3S-(3-hydroxy-n-propyl)oxy-butane-1R,2S,4-triol,
1-[5-(2S,3R,4-Trihydroxy-butyl)-pyrazin-2-yl]-3R-(carboxyméthyl)oxy-butane-1R,2S,4-triol,
1-[5-(2S,3R,4-Trihydroxy-butyl)-pyrazin-2-yl]-3S-(carboxyméthyl)oxy-butane-1R,2S,4-triol,
1-[5-(2S,3R,4-Trihydroxy-butyl)-pyrazin-2-yl]-3R-(3-carboxy-n-propyl)oxy-butane-1R,2S,4-triol,
1-[5-(2S,3R,4-Trihydroxy-butyl)-pyrazin-2-yl]-3S-(3-carboxy-n-propyl)oxy-butane-1R,2S,4-triol,
1-[5-(2S,3R,4-Trihydroxy-butyl)-pyrazin-2-yl]-1R,3R,4-trihydroxy-butane-2-one,
1-[5-(2S,3R,4-Trihydroxy-butyl)-pyrazin-2-yl]-butane-1R,3R,4-triol,
1-[5-(2S,3R,4-Trihydroxy-butyl)-pyrazin-2-yl]-2R-fluoro-butane-1R,3R,4-triol,
1-[5-(2S,3R,4-Trihydroxy-butyl)-pyrazin-2-yl]-2S-fluoro-butane-1R,3R,4-triol,
1-[5-(2S,3R,4-Trihydroxy-butyl)-pyrazin-2-yl]-2R-amino-butane-1S,3S,4-triol,
1-[5-(2S,3R,4-Trihydroxy-butyl)-pyrazin-2-yl]-2S-amino-butane-1S,3S,4-triol,
1-[5-(2S,3R,4-Trihydroxy-butyl)-pyrazin-2-yl]-2R-(N-méthyl)amino-butane-1S,3S,4-triol,
1-[5-(2S,3R,4-Trihydroxy-butyl)-pyrazin-2-yl)-2S-(N-méthyl)amino-butane-1S,3S,4-triol,
1-[5-(2S,3R,4-Trihydroxy-butyl)-pyrazin-2-yl]-2R-(N-éthyl)amino-butane-1S,3S,4-triol,
1-[5-(2S,3R,4-Trihydroxy-butyl)-pyrazin-2-yl]-2S-(N-éthyl)amino-butane-1S,3S,4-triol,
1-[5-(2S,3R,4-Trihydroxy-butyl)-pyrazin-2-yl]-2R-(N-n-butyl)amino-butane-1S,3S,4-triol,
1-[5-(2S,3R,4-Trihydroxy-butyl)-pyrazin-2-yl]-2S-(N-n-butyl)amino-butane-1S,3S,4-triol,
1-[5-(2S,3R,4-Trihydroxy-butyl)-pyrazin-2-yl]-2R-(N-benzyl)amino-butane-1S,3S,4-triol,
1-[5-(2S,3R,4-Trihydroxy-butyl)-pyrazin-2-yl]-2S-(N-benzyl)amine-butane-1S,3S,4-triol,
1-[5-(2S,3R,4-Trihydroxy-butyl)-pyrazin-2-yl]-2R-(N-acétyl)amino-butane-1S,3S,4-triol,
1-[5-(2S,3R,4-Trihydroxy-butyl)-pyrazin-2-yl]-2S-(N-acétyl)amino-butane-1S,3S,4-triol,
1-[5-(2S,3R,4-Trihydroxy-butyl)-pyrazin-2-yl]-2R-(N-Butanoyl)amino-butane-1S,3S,4-triol,
1-[5-(2S,3R,4-Trihydroxy-butyl)-pyrazin-2-yl]-2S-(N-Butanoyl)amino-butane-1S,3S,4-triol,
1-[5-(2S,3R,4-Trihydroxy-butyl)-pyrazin-2-yl]-2R-(N-Benzoyl)amino-butane-1S,3S,4-triol,
1-[5-(2S,3R,4-Trihydroxy-butyl)-pyrazin-2-yl]-2S-(N-Benzoyl)amino-butane-1S,3S,4-triol,
1-[5-(2S,3R,4-Trihydroxy-butyl)-pyrazin-2-yl]-2R-méthoxy-butane-1R,3R,4-triol,
1-[5-(2S,3R,4-Trihydroxy-butyl)-pyrazin-2-yl]-2S-méthoxy-butane-1R,3R,4-triol,
1-[5-(2S,3R,4-Trihydroxy-butyl)-pyrazin-2-yl]-2R-éthoxy-butane-1R,3R,4-triol,
1-[5-(2S,3R,4-Trihydroxy-butyl)-pyrazin-2-yl]-2S-éthoxy-butane-1R,3R,4-triol,
1-[5-(2S,3R,4-Trihydroxy-butyl)-pyrazin-2-yl]-2R-n-butoxy-butane-1R,3R,4-triol,
1-[5-(2S,3R,4-Trihydroxy-butyl)-pyrazin-2-yl]-2S-n-butoxy-butane-1R,3R,4-triol,
1-[5-(2S,3R,4-Trihydroxy-butyl)-pyrazin-2-yl]-2R-(2-hydroxyéthyl)oxy-butane-1R,3R,4-triol,
1-[5-(2S,3R,4-Trihydroxy-butyl)-pyrazin-2-yl]-2S-(2-hydroxyéthyl)oxy-butane-1R,3R,4-triol,
1-[5-(2S,3R,4-Trihydroxy-butyl)-pyrazin-2-yl]-2R-(3-hydroxy-n-propyl)oxy-butane-1R,3R,4-triol,
1-[5-(2S,3R,4-Trihydroxy-butyl)-pyrazin-2-yl]-2S-(3-hydroxy-n-propyl)oxy-butane-1R,3R,4-triol,
1-[5-(2S,3R,4-Trihydroxy-butyl)-pyrazin-2-yl]-2R-(carboxyméthyl)oxy-butane-1R,3R,4-triol,
1-[5-(2S,3R,4-Trihydroxy-butyl)-pyrazin-2-yl]-2S-(carboxyméthyl)oxy-butane-1R,3R,4-triol,
1-[5-(2S,3R,4-Trihydroxy-butyl)-pyrazin-2-yl]-2R-(3-carboxy-n-propyl)oxy-butane-1R,3R,4-triol,
1-[5-(2S,3R,4-Trihydroxy-butyl)-pyrazin-2-yl]-2S-(3-carboxy-n-propyl)oxy-butane-1R,3R,4-triol,
1-[5-(2S,4-Dihydroxy-3-oxo-butyl)-pyrazin-2-yl]-2S,4-dihydroxy-butane-1,3-dione,
4-[5-(2S,4-Dihydroxy-butyl)-pyrazin-2-yl]-butane-1,3S-diol,
4-[5-(3R-Fluoro-2S,4-dihydroxy-butyl)-pyrazin-2-yl]-2R,4R-difluoro-butane-1,3S-diol,
4-[5-(3S-Fluoro-2S,4-dihydroxy-butyl)-pyrazin-2-yl]-2S,4S-difluoro-butane-1,3S-diol,
1-[5-(3R-Amino-2S,4-dihydroxy-butyl)-pyrazin-2-yl]-2R,4R-diamino-butane-1,3S-diol,
1-[5-(3S-Amino-2S,4-dihydroxy-butyl)-pyrazin-2-yl]-2S,4S-diamino-butane-1,3S-diol,
1-[5-(3R-(N-Méthyl)amino-2S,4-dihydroxy-butyl)-pyrazin-2-yl]-2R,4R-di-(N-Méthyl)amino-butane-1,3S-diol,
1-[5-(3S-(N-Méthyl)amino-2S,4-dihydroxy-butyl)-pyrazin-2-yl]-2S,4S-di-(N-Méthyl)amino-butane-1,3S-diol,
1-[5-(3R-(N-Ethyl)amino-2S,4-dihydroxy-butyl)-pyrazin-2-yl]-2R,4R-di-(N-éthyl)amino-butane-1,3S-diol,
1-[5-(3S-(N-Ethyl)amino-2S,4-dihydroxy-butyl)-pyrazin-2-yl]-2S,4S-di-(N-éthyl)amino-butane-1,3S-diol,
1-[5-(3R-(N-n-Butyl)amino-2S,4-dihydroxy-butyl)-pyrazin-2-yl]-2R,4R-di-(N-n-Butyl)amino-butane-1,3S-diol,
1-[5-(3S-(N-n-Butyl)amino-2S,4-dihydroxy-butyl)-pyrazin-2-yl]-2S,4S-di-(N-n-Butyl)amino-butane-1,3S-diol,
1-[5-(3R-(N-Benzyl)amino-2S,4-dihydroxy-butyl)-pyrazin-2-yl]-2R,4R-di-(N-Benzyl)amino-butane-1,3S-diol,
1-[5-(3S-(N-Benzyl)amino-2S,4-dihydroxy-butyl)-pyrazin-2-yl]-2S,4S-di-(N-Benzyl)amino-butane-1,3S-diol,
1-[5-(3R-(N-Acétyl)amino-2S,4-dihydroxy-butyl)-pyrazin-2-yl]-2R,4R-di-(N-Acétyl)amino-butane-1,3S-diol,
1-[5-(3S-(N-Acétyl)amino-2S,4-dihydroxy-butyl)-pyrazin-2-yl]-2S,4S-di-(N-Acétyl)amino-butane-1,3S-diol,
1-[5-(3R-(N-Butanoyl)amino-2S,4-dihydroxy-butyl)-pyrazin-2-yl]-2R,4R-di-(N-Butanoyl)amino-butane-1,3S-diol,
1-[5-(3S-(N-Butanoyl)amino-2S,4-dihydroxy-butyl)-pyrazin-2-yl]-2S,4S-di-(N-Butanoyl)amino-butane-1,3S-diol,
1-[5-(3R-(N-Benzoyl)amino-2S,4-dihydroxy-butyl)-pyrazin-2-yl]-2R,4R-di-(N-Benzoyl)amino-butane-1,3S-diol,
1-[5-(3S-(N-Benzoyl)amino-2S,4-dihydroxy-butyl)-pyrazin-2-yl]-2S,4S-di-(N-Benzoyl)amino-butane-1,3S-diol,
1-[5-(3R-Méthoxy-2S,4-dihydroxy-butyl)-pyrazin-2-yl]-2R,4R-di-méthoxy-butane-1,3S-diol,
1-[5-(3S-Méthoxy-2S,4-dihydroxy-butyl)-pyrazin-2-yl]-2S,4S-di-méthoxy-butane-1,3S-diol,
1-[5-(3R-Ethoxy-2S,4-dihydroxy-butyl)-pyrazin-2-yl]-2R,4R-di-éthoxy-butane-1,3S-diol,
1-[5-(3S-Ethoxy-2S,4-dihydroxy-butyl)-pyrazin-2-yl]-2S,4S-di-éthoxy-butane-1,3S-diol,
1-[5-(3R-n-Butoxy-2S,4-dihydroxy-butyl)-pyrazin-2-yl]-2R,4R-di-n-butoxy-butane-1,3S-diol,
1-[5-(3S-n-Butoxy-2S,4-dihydroxy-butyl)-pyrazin-2-yl]-2S,4S-di-n-butoxy-butane-1,3S-diol,
1-[5-(3R-(2-Hydroxyéthyl)oxy-2S,4-dihydroxy-butyl)-pyrazin-2-yl]-2R,4R-di-(2-hydroxyéthyl)oxy-butane-1,3S-diol,
1-[5-(3S-(2-Hydroxyéthyl)oxy-2S,4-dihydroxy-butyl)-pyrazin-2-yl]-2S,4S-di-(2-hydroxyéthyl)oxy-butane-1,3S-diol,
1-[5-(3R-(3-Hydroxy-n-propyl)oxy-2S,4-dihydroxy-butyl)-pyrazin-2-yl]-2R,4R-di-(3-hydroxy-n-propyl)oxy-butane-1,3S-diol,
1-[5-(3S-(3-Hydroxy-n-propyl)oxy-2S,4-dihydroxy-butyl)-pyrazin-2-yl]-2S,4S-di-(3-hydroxy-n-propyl)oxy-butane-1,3S-diol,
1-[5-(3R-(Carboxyméthyl)oxy-2S,4-dihydroxy-butyl)-pyrazin-2-yl]-2R,4R-di-(carboxyméthyl)oxy-butane-1,3S-diol,
1-[5-(3S-(Carboxyméthyl)oxy-2S,4-dihydroxy-butyl)-pyrazin-2-yl]-2S,4S-di-(carboxyméthyl)oxy-butane-1,3S-diol,
1-[5-(3R-(3-Carboxy-n-propyl)oxy-2S,4-dihydroxy-butyl)-pyrazin-2-yl]-2R,4R-di-(3-carboxy-n-propyl)oxy-butane-1,3S-diol,
1-[5-(3S-(3-Carboxy-n-propyl)oxy-2S,4-dihydroxy-butyl)-pyrazin-2-yl]-2S,4S-di-(3-carboxy-n-propyl)oxy-butane-1,3S-diol,
1-[5-(3R,4-Dihydroxy-2-oxo-butyl)-pyrazin-2-yl]-3R,4-dihydroxy-butane-1,2-dione,
4-[5-(2S,4-Dihydroxy-butyl)-pyrazin-2-yl]-butane-1,3S-diol,
4-[5-(3R-Fluoro-2S,4-dihydroxy-butyl)-pyrazin-2-yl]-2R,4R-difluoro-butane-1,3S-diol,
4-[5-(3S-Fluoro-2S,4-dihydroxy-butyl)-pyrazin-2-yl]-2S,4S-difluoro-butane-1,3S-diol,
4-[5-(3R-Amino-2S,4-dihydroxy-butyl)-pyrazin-2-yl]-2R,4R-diamino-butane-1,3S-diol,
4-[5-(3S-Amino-2S,4-dihydroxy-butyl)-pyrazin-2-yl]-2S,4S-diamino-butane-1,3S-diol,
4-[5-(3R-(N-Méthyl)amino-2S,4-dihydroxy-butyl)-pyrazin-2-yl]-2R,4R-di-(N-méthyl)amino-butane-1,3S-diol,
4-[5-(3S-(N-Méthyl)amino-2S,4-dihydroxy-butyl)-pyrazin-2-yl]-2S,4S-di-(N-méthyl)amino-butane-1,3S-diol,
4-[5-(3R-(N-Ethyl)amino-2S,4-dihydroxy-butyl)-pyrazin-2-yl]-2R,4R-di-(N-éthyl)amino-butane-1,3S-diol,
4-[5-(3S-(N-Ethyl)amino-2S,4-dihydroxy-butyl)-pyrazin-2-yl]-2S,4S-di-(N-éthyl)amino-butane-1,3S-diol,
4-[5-(3R-(N-n-Butyl)amino-2S,4-dihydroxy-butyl)-pyrazin-2-yl]-2R,4R-di-(N-n-butyl)amino-butane-1,3S-diol,
4-[5-(3S-(N-n-Butyl)amino-2S,4-dihydroxy-butyl)-pyrazin-2-yl]-2S,4S-di-(N-n-butyl)amino-butane-1,3S-diol,
4-[5-(3R-(N-Benzyl)amino-2S,4-dihydroxy-butyl)-pyrazin-2-yl]-2R,4R-di-(N-benzyl)amino-butane-1,3S-diol,
4-[5-(3S-(N-Benzyl)amino-2S,4-dihydroxy-butyl)-pyrazin-2-yl]-2S,4S-di-(N-benzyl)amino-butane-1,3S-diol,
4-[5-(3R-(N-Acétyl)amino-2S,4-dihydroxy-butyl)-pyrazin-2-yl]-2R,4R-di-(N-acétyl)amino-butane-1,3S-diol,
4-[5-(3S-(N-Acétyl)amino-2S,4-dihydroxy-butyl)-pyrazin-2-yl]-2S,4S-di-(N-acétyl)amino-butane-1,3S-diol,
4-[5-(3R-(N-Butanoyl)amino-2S,4-dihydroxy-butyl)-pyrazin-2-yl]-2R,4R-di-(N-butanoyl)amino-butane-1,3S-diol,
4-[5-(3S-(N-Butanoyl)amino-2S,4-dihydroxy-butyl)-pyrazin-2-yl]-2S,4S-di-(N-butanoyl)amino-butane-1,3S-diol,
4-[5-(3R-(N-Benzoyl)amino-2S,4-dihydroxy-butyl)-pyrazin-2-yl]-2R,4R-di-(N-benzoyl)amino-butane-1,3S-diol,
4-[5-(3S-(N-Benzoyl)amino-2S,4-dihydroxy-butyl)-pyrazin-2-yl]-2S,4S-di-(N-benzoyl)amino-butane-1,3S-diol,
4-[5-(3R-Méthoxy-2S,4-dihydroxy-butyl)-pyrazin-2-yl]-2R,4R-diméthoxy-butane-1,3S-diol,
4-[5-(3S-Méthoxy-2S,4-dihydroxy-butyl)-pyrazin-2-yl]-2S,4S-diméthoxy-butane-1,3S-diol,
4-[5-(3R-Ethoxy-2S,4-dihydroxy-butyl)-pyrazin-2-yl]-2R,4R-diéthoxy-butane-1,3S-diol,
4-[5-(3S-Ethoxy-2S,4-dihydroxy-butyl)-pyrazin-2-yl]-2S,4S-diéthoxy-butane-1,3S-diol,
4-[5-(3R-n-Butoxy-2S,4-dihydroxy-butyl)-pyrazin-2-yl]-2R,4R-di-n-Butoxy-butane-1,3S-diol,
4-[5-(3S-n-Butoxy-2S,4-dihydroxy-butyl)-pyrazin-2-yl]-2S,4S-di-n-Butoxy-butane-1,3S-diol,
4-[5-(3R-(2-Hydroxyéthyl)oxy-2S,4-dihydroxy-butyl)-pyrazin-2-yl]-2R,4R-di-(2-hydroxyéthyl)oxy-butane-1,3S-diol,
4-[5-(3S-(2-Hydroxyéthyl)oxy-2S,4-dihydroxy-butyl)-pyrazin-2-yl]-2S,4S-di-(2-hydroxyéthyl)oxy-butane-1,3S-diol,
4-[5-(3R-(3-Hydroxy-n-propyl)oxy-2S,4-dihydroxy-butyl)-pyrazin-2-yl]-2R,4R-di-(3-hydroxy-n-propyl)oxy-butane-1,3S-diol,
4-[5-(3S-(3-Hydroxy-n-propyl)oxy-2S,4-dihydroxy-butyl)-pyrazin-2-yl]-2S,4S-di-(3-hydroxy-n-propyl)oxy-butane-1,3S-diol,
4-[5-(3R-(Carboxyméthyl)oxy-2S,4-dihydroxy-butyl)-pyrazin-2-yl]-2R,4R-di-(carboxyméthyl)oxy-butane-1,3S-diol,
4-[5-(3S-(Carboxyméthyl)oxy-2S,4-dihydroxy-butyl)-pyrazin-2-yl]-2S,4S-di-(carboxyméthyl)oxy-butane-1,3S-diol,
4-[5-(3R-(3-Carboxy-n-propyl)oxy-2S,4-dihydroxy-butyl)-pyrazin-2-yl]-2R,4R-di-(3-carboxy-n-propyl)oxy-butane-1,3S-diol,
4-[5-(3S-(3-Carboxy-n-propyl)oxy-2S,4-dihydroxy-butyl)-pyrazin-2-yl]-2S,4S-di-(3-carboxy-n-propyl)oxy-butane-1,3S-diol,
1-[5-(3S,4-Dihydroxy-1E-butényl)-pyrazin-2-yl]-butane-1R,2S,3R,4-tétraol,
et leurs sels avec un acide minéral ou organique pharmaceutiquement acceptable.

4. Médicaments contenant en tant que principe actif au moins un composé de formule (I) selon la revendication 1 pour lesquels
R₉ et R₁₀ représentent chacun un radical -CH₂OH, et soit
a) R₃ représente un radical méthylène, R₄ représente un radical -CHOH- et l'un des radicaux R₁, R₂, R₅ et R₆ représente un radical -CHFou -CH(OR₈) et les autres représentent chacun un radical -CHOH-,
b) R₂ et R₅ représentent chacun un radical -CHOH-, R₃ représente un radical méthylène et R₁, R₄ et R₆ sont identiques et représentent chacun un radical -CH(OR₈),
c) R₁ et R₆ représentent chacun un radical -CHOH-, R₃ représente un radical méthylène et R₂, R₄ et R₅ sont identiques et représentent chacun un radical -CH(OR₈),
d) R₁, R₄, R₅ et R₆ représentent chacun un radical -CHOH- et -R₂-R₃-représente un radical -CH=CH-,
R₈ représente un radical alkyle,
leurs stéréoisomères, les formes cis et trans du composé pour lequel -R₂-R₃-représente une chaîne -CH=CH- et leurs sels avec un acide minéral ou organique pharmaceutiquement acceptable.

5. Médicaments contenant en tant que principe actif au moins un composé de formule (I) selon la revendication 1 choisi parmi les composés suivants :
1-[5-(3S,4-Dihydroxy-butyl)-pyrazin-2-yl]-butane-1R,2S,3R,4-tétraol
1-[5-(3S,4-Dihydroxy-1E-butényl)-pyrazin-2-yl]-butane-1R,2S,3R,4-tétraol
1-[5-(2S-Méthoxy-3R,4-dihydroxy-butyl)-pyrazin-2-yl]-butane-1R,2S,3R,4-tétraol
1-[5-(2R-fluoro-3R,4-dihydroxy-butyl)-pyrazin-2-yl]-butane-1R,2S,3R,4-tétraol
1-[5-(2S,4-dihydroxy-3R-méthoxy-butyl)-pyrazin-2-yl]-butane-1R,2S,3R,4-tétraol
4-[5-(3R,4-dihydroxy-2S-méthoxy-butyl)]-pyrazin-2-yl]-3R,4R-diméthoxy-butane-1,2-diol
et leurs sels avec un acide minéral ou organique pharmaceutiquement acceptable.

6. Composés de formule : 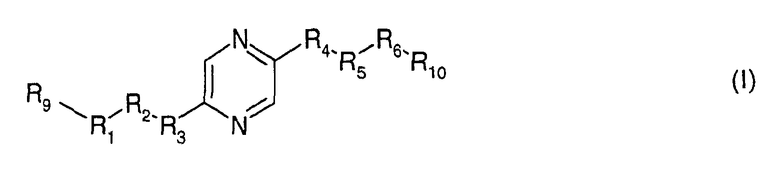 dans laquelle
R₉ et R₁₀ représentent chacun un radical -CH₂OH, et soit
a) R₃ représente un radical méthylène, R₄ représente un radical -CHOH- et l'un des radicaux R₁, R₂, R₅ et R₆ représente un radical carbonyle, méthylène, -CHF-, -CH(NHR₇)- ou -CH(OR₈) et les autres représentent chacun un radical -CHOH-,
b) R₂ et R₅ représentent chacun un radical -CHOH-, R₃ représente un radical méthylène et R₁, R₄ et R₆ sont identiques et représentent chacun un radical carbonyle, méthylène, -CHF-, -CH(NHR₇)- ou -CH(OR₈),
c) R₁ et R₆ représentent chacun un radical -CHOH-, R₃ représente un radical méthylène et R₂, R₄ et R₅ sont identiques et représentent chacun un radical carbonyle, méthylène, -CHF-, -CH(NHR₇)- ou -CH(OR₈),
d) R₁, R₄, R₅ et R₆ représentent chacun un radical -CHOH- et -R₂-R₃-représente un radical -CH=CH-,
R₇ représente un atome d'hydrogène ou un radical alkyle, -CO-alk, -CO-Ar ou -CO-Het,
R₈ représente un radical alkyle, -alk-COOH ou -alk-OH,
alk représente un radical alkyle,
Ar représente un radical phényle ou phényle substitué par un ou plusieurs substituants choisis parmi un atome d'halogène, un radical alkyle, alcoxy, alcoxycarbonyle, amino, monoalkylamino ou dialkylamino,
Het représente un hétérocycle mono, di ou tricyclique saturé ou insaturé contenant 1 à 9 atomes de carbone et un ou plusieurs hétéroatomes choisis parmi oxygène, soufre et azote,
les radicaux et portions alkyle et alcoxy contenant 1 à 6 atomes de carbone en chaîne droite ou ramifiée,
ou un de ses stéréoisomères ou pour les composés pour lesquels -R₂-R₃-représente un radical -CH=CH- leurs formes cis ou trans ou un de ses sels, à l'exception du composé de formule :

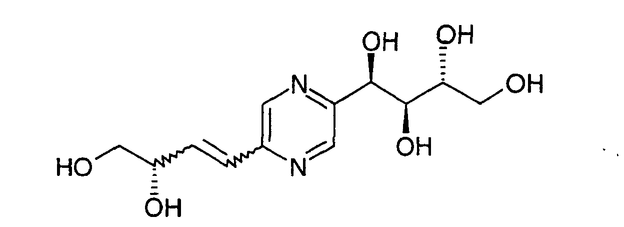

7. Procédé de préparation des composés de formule (I) selon la revendication 6 pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical carbonyle et les autres représentent chacun un radical -CHOH-, soit R₁ et R₆ représentent chacun un radical -CHOH-, R₃ représente un radical méthylène et R₂, R₄ et R₅ sont identiques et représentent chacun un radical carbonyle, soit R₂ et R₅ représentent chacun un radical -CHOH-, R₃ représente un radical méthylène et R₁, R₄ et R₆ sont identiques et représentent chacun un radical carbonyle **caractérisé en ce que** l'on oxyde un dérivé choisi parmi les formules : 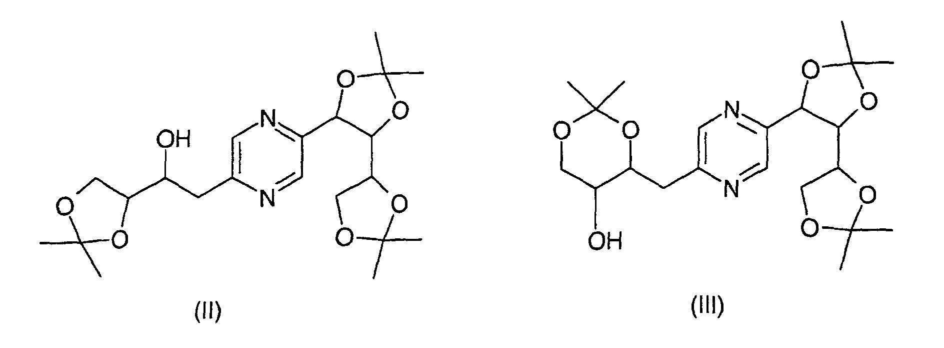 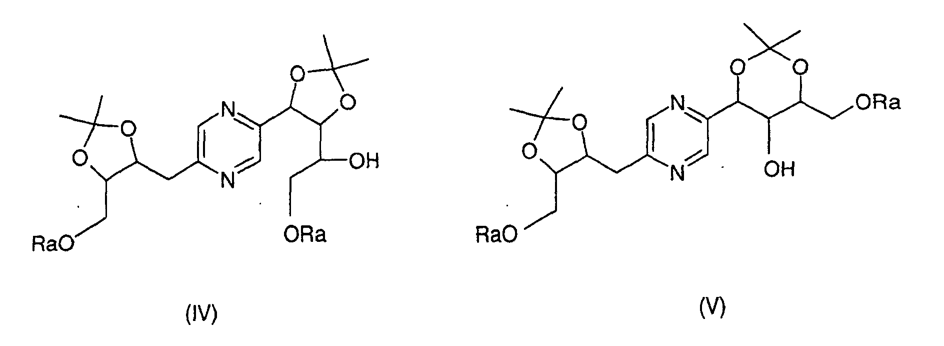 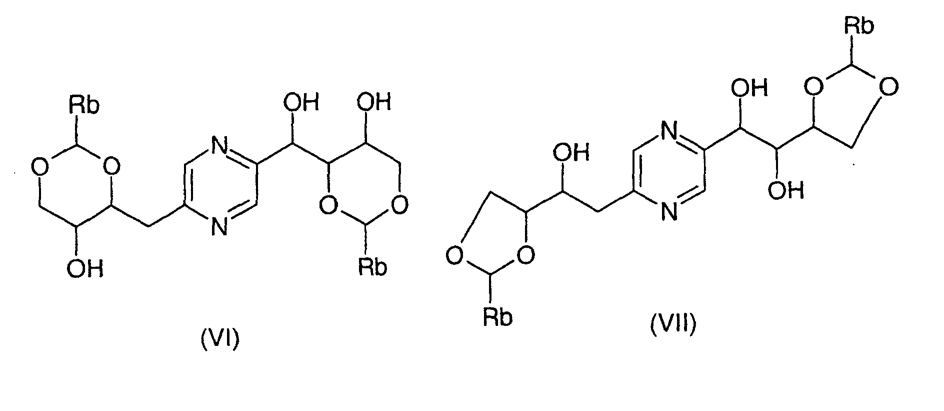 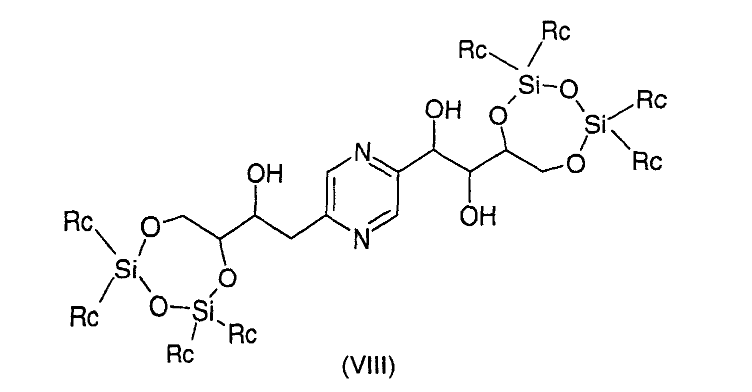 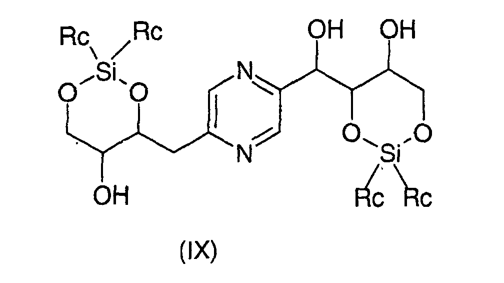 dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle, les radicaux et portions alkyle et alcoxy contenant 1 à 6 atomes de carbone en chaîne droite ou ramifiée, ou un stéréoisomère d'un tel dérivé, puis déprotège les hydroxyles, isole le produit et le transforme éventuellement en sel.

8. Procédé de préparation des composés de formule (I) selon la revendication 6 pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical méthylène et les autres représentent chacun un radical -CHOH-, soit R₁ et R₆ représentent chacun un radical -CHOH-, R₃ représente un radical méthylène et R₂, R₄ et R₅ sont identiques et représentent chacun un radical méthylène, soit R₂ et R₅ représentent chacun un radical -CHOH- et R₁, R₃, R₄ et R₆ représentent chacun un radical méthylène **caractérisé en ce que** l'on condense un chlorothionocarbonate d'alkyle ou de phényle sur un dérivé choisi parmi les formules suivantes : 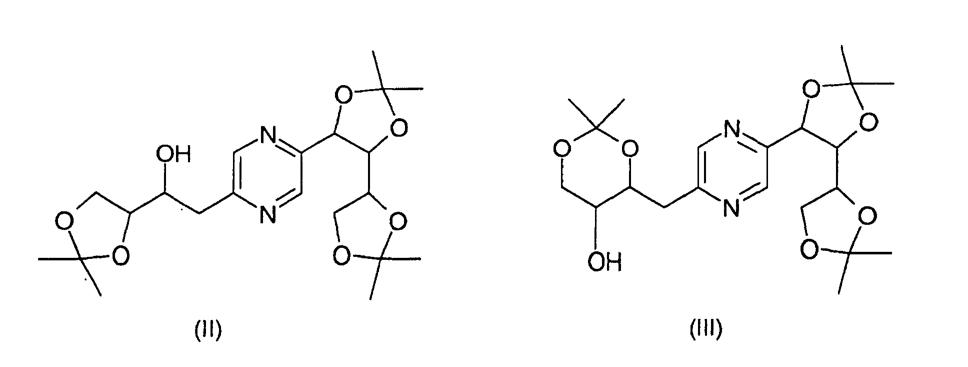 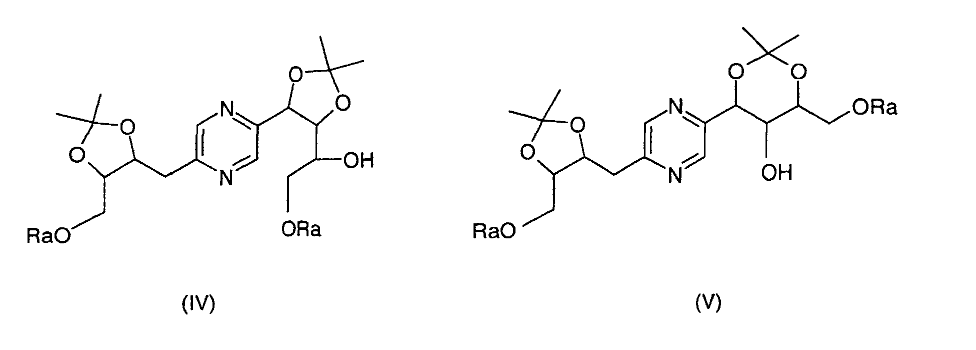 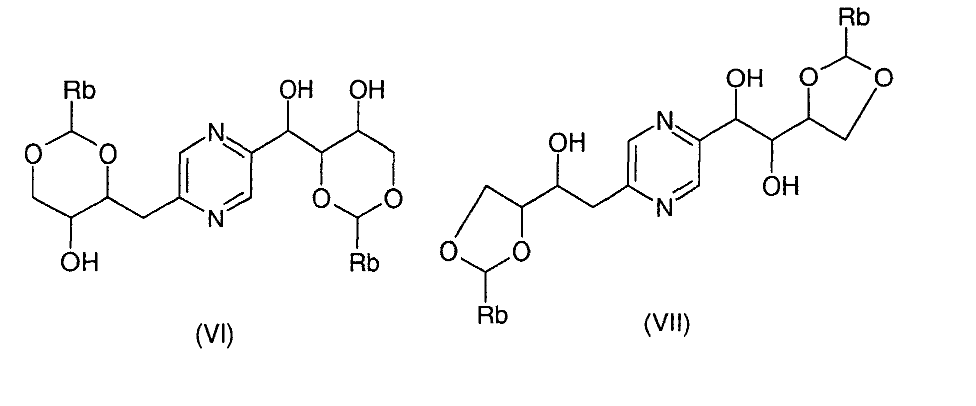 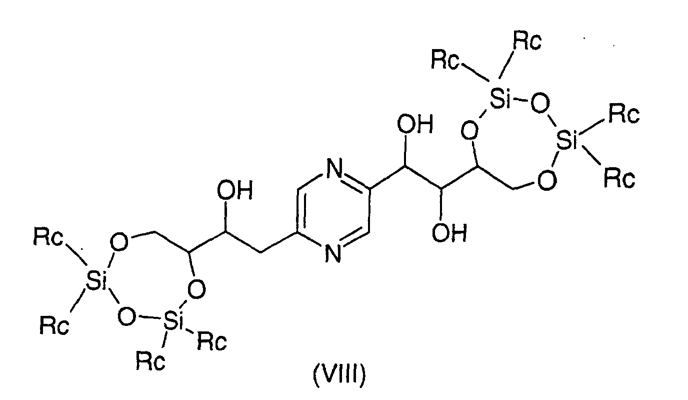 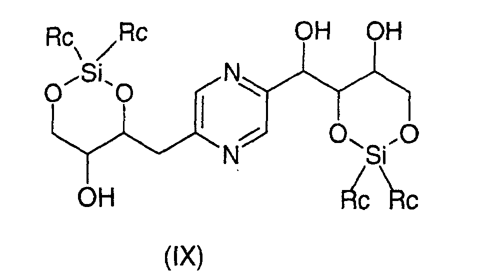 dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle, les radicaux et portions alkyle et alcoxy contenant 1 à 6 atomes de carbone en chaîne droite ou ramifiée, ou un stéréoisomère d'un tel dérivé, puis déprotège les hydroxyles, isole le produit et le transforme éventuellement en sel.

9. Procédé de préparation des composés de formule (I) selon la revendication 6 pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical -CHF- et les autres représentent chacun un radical -CHOH-, soit R₁ et R₆ représentent chacun un radical -CHOH-, R₃ représente un radical méthylène et R₂, R₄ et R₅ sont identiques et représentent chacun un radical -CHF-, soit R₂ et R₅ représentent chacun un radical -CHOH-, R₃ représente un radical méthylène et R₁, R₄ et R₆ sont identiques et représentent chacun un radical -CHF-**caractérisé en ce que** l'on fluorure un dérivé choisi parmi les formules: 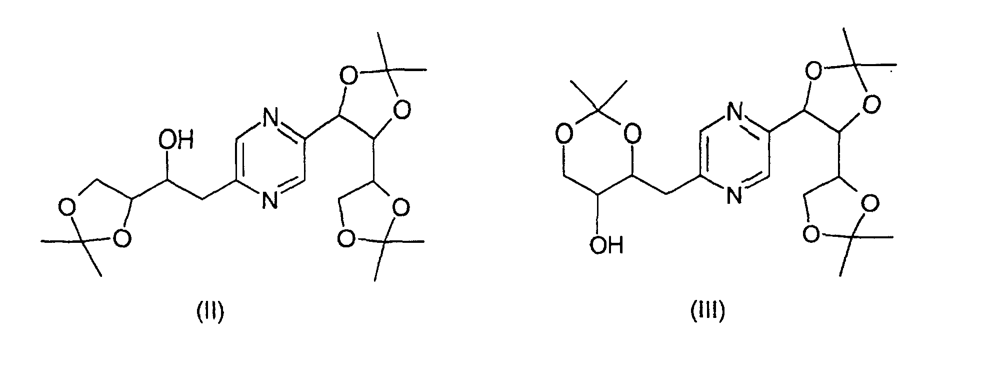 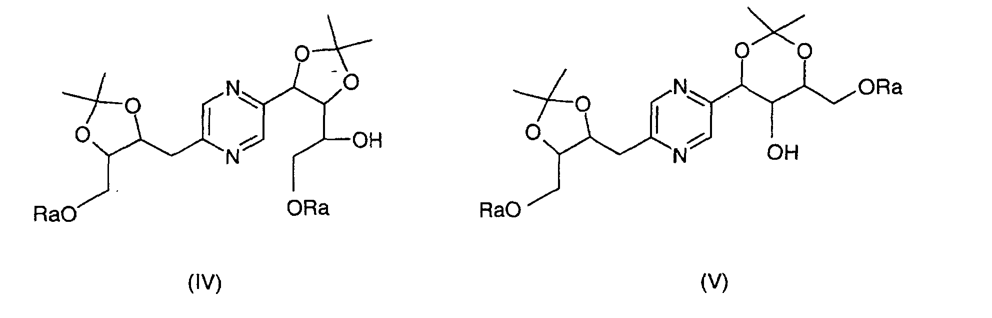 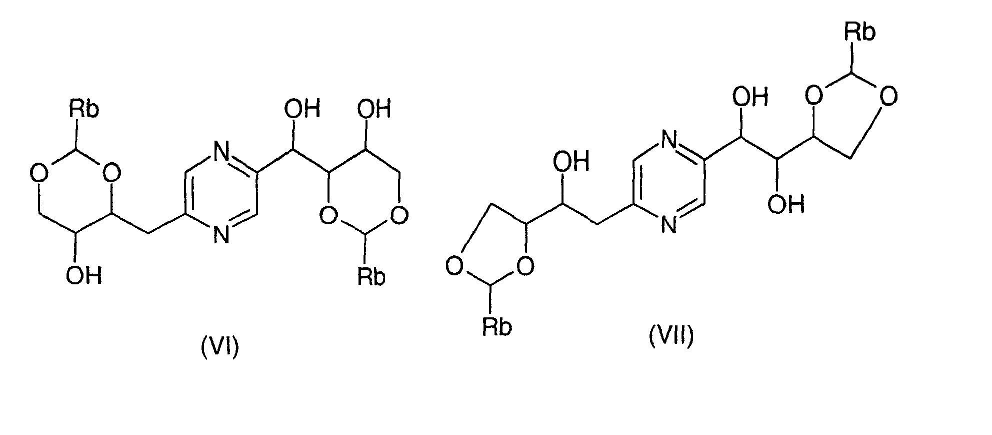 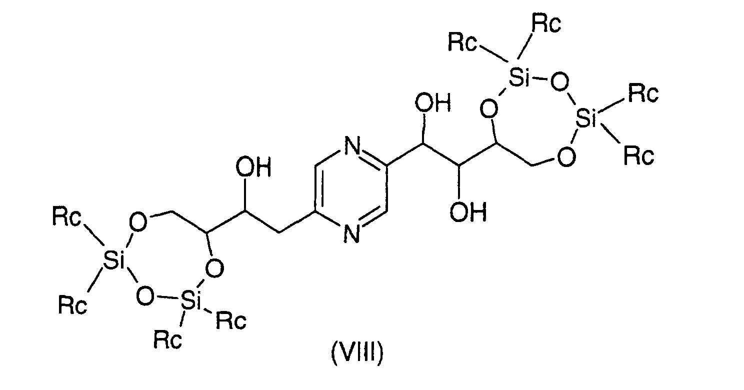 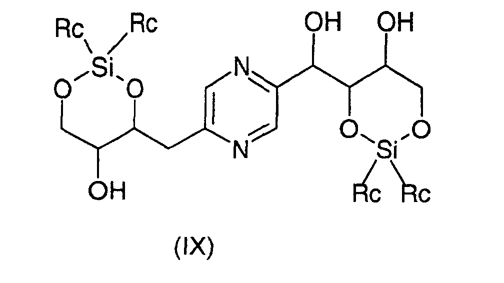 dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle, les radicaux et portions alkyle et alcoxy contenant 1 à 6 atomes de carbone en chaîne droite ou ramifiée, ou un stéréoisomère d'un tel dérivé, puis déprotège les hydroxyles, isole le produit et le transforme éventuellement en sel.

10. Procédé de préparation des composés de formule (I) selon la revendication 6 pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical -CH(NHR₇)- et les autres représentent chacun un radical -CHOH-, soit R₁ et R₆ représentent chacun un radical -CHOH-, R₃ représente un radical méthylène et R₂, R₄ et R₅ sont identiques et représentent chacun un radical -CH(NHR₇)-, soit R₂ et R₅ représentent chacun un radical -CHOH-, R₃ représente un radical méthylène et R₁, R₄ et R₆ sont identiques et représentent chacun un radical -CH(NHR₇)- **caractérisé en ce que** l'on réduit un dérivé choisi parmi les formules : 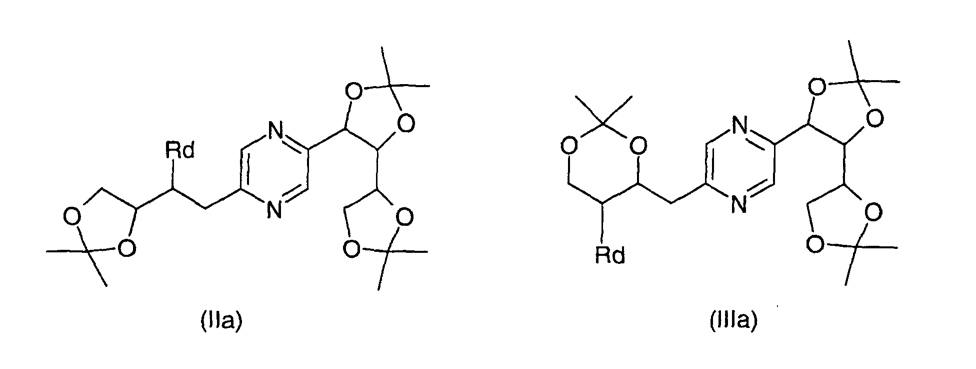 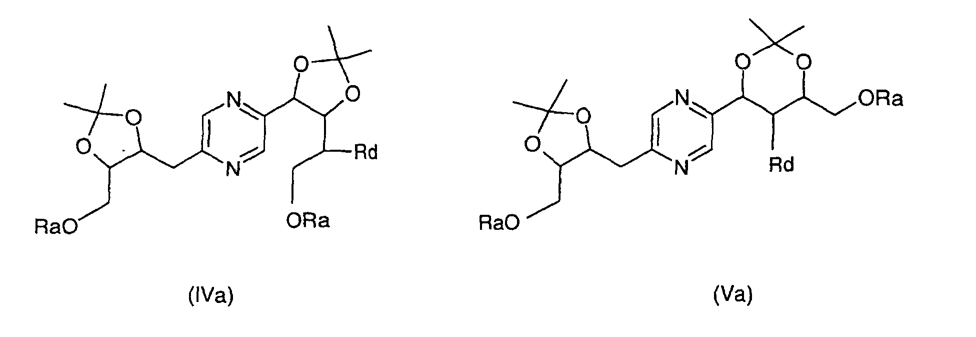 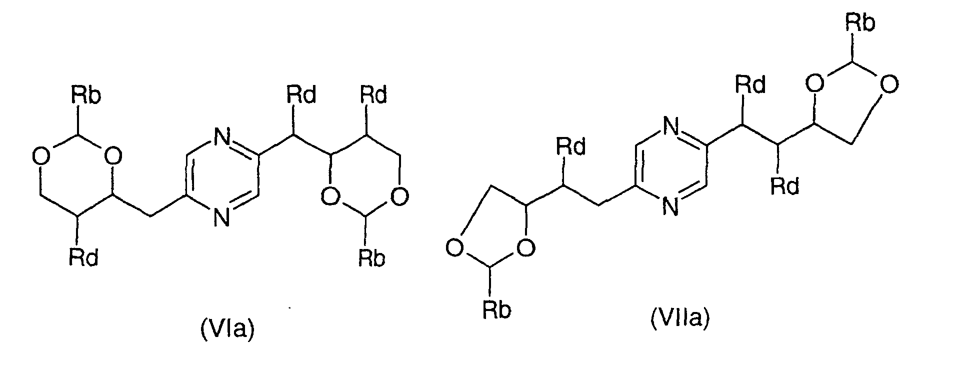 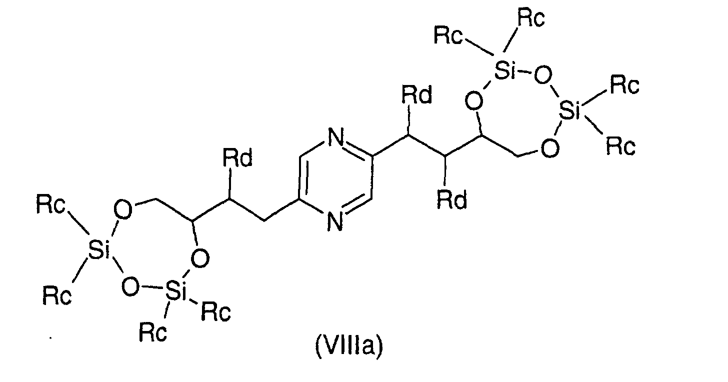 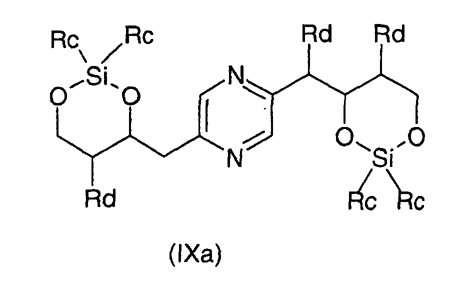 dans lesquelles Ra représente un radical trialkylsilyle, alkyldiphénylsilyle ou dialkylphénylsilyle, Rb représente un radical phényle éventuellement substitué par au moins un radical alcoxy, Rc représente un radical alkyle ou phényle et Rd représente un radical azido, les radicaux et portions alkyle et alcoxy contenant 1 à 6 atomes de carbone en chaîne droite ou ramifiée, ou un stéréoisomère d'un tel dérivé, suivie éventuellement de la réaction d'un dérivé de formule HalR₇ pour lequel R₇ a les mêmes significations que dans la revendication 4 sauf hydrogène, Hal représente un atome d'halogène, déprotège les hydroxyles, isole le produit et le transforme éventuellement en sel.

11. Procédé de préparation des composés de formule (I) selon la revendication 6 pour lesquels R₉ et R₁₀ représentent chacun un radical -CH₂OH et soit R₃ représente un radical méthylène, R₄ représente un radical -CHOH- et l'un des radicaux R₁, R₂, R₅ et R₆ représente un radical -CH(OR₈) et les autres représentent chacun un radical -CHOH-, soit R₁ et R₆ représentent chacun un radical -CHOH-, R₃ représente un radical méthylène et R₂, R₄ et R₅ sont identiques et représentent chacun un radical -CH(OR₈), soit R₂ et R₅ représentent chacun un radical -CHOH-, R₃ représente un radical méthylène et R₁, R₄ et R₆ sont identiques et représentent chacun un radical -CH(OR₈) **caractérisé en ce que** l'on fait réagir un dérivé de formule 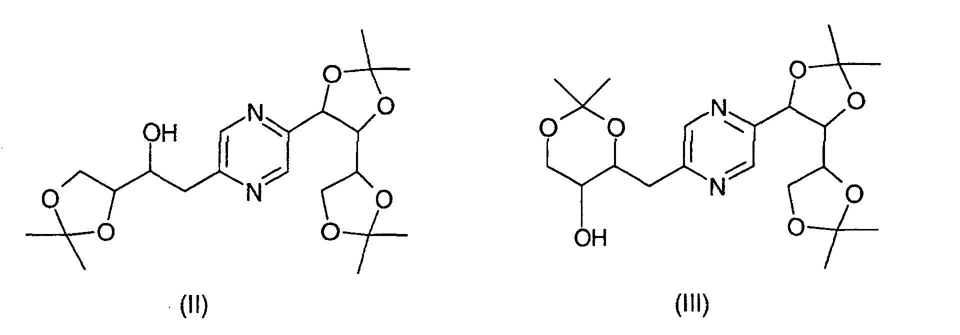 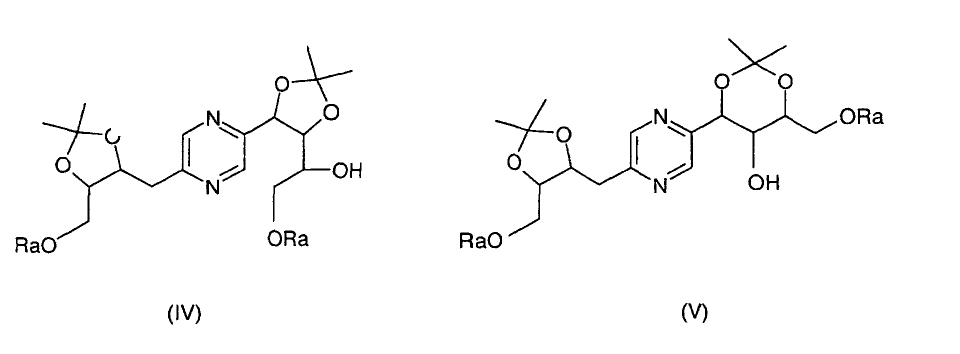 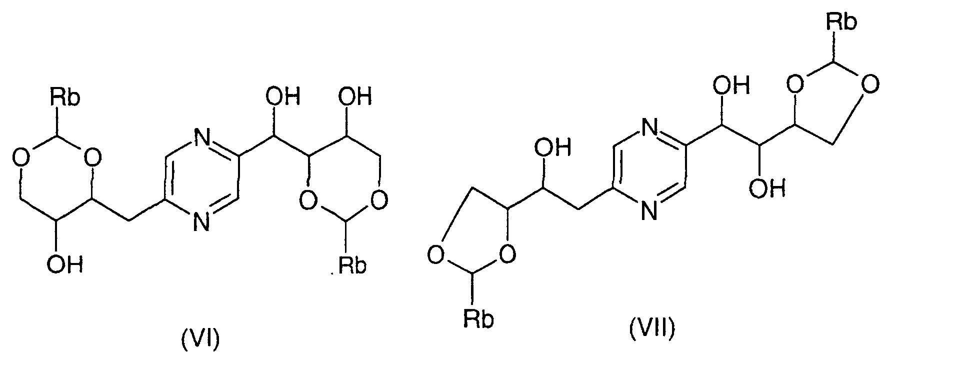 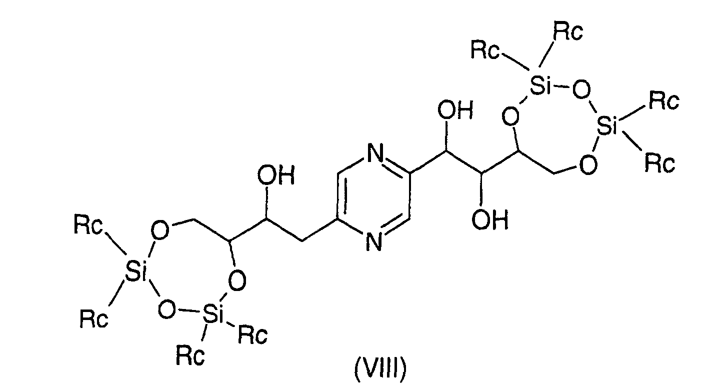 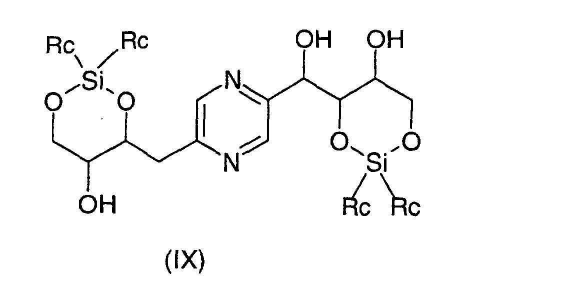 dans lesquelles Ra représente un radical trialkylsilyle, alkyldiphénylsilyle ou dialkylphénylsilyle, Rb représente un radical phényle éventuellement substitué par au moins un radical alcoxy et Rc représente un radical alkyle ou phényle, les radicaux et portions alkyle et alcoxy contenant 1 à 6 atomes de carbone en chaîne droite ou ramifiée, ou un stéréoisomère d'un tel dérivé, sur un dérivé HaIR₈ dans lequel R₈ a les mêmes significations que dans la revendication 4, puis déprotège les hydroxyles, isole le produit et le transforme éventuellement en sel.

12. Procédé de préparation des composés de formule (I) selon la revendication 6 pour lesquels R₉ et R₁₀ représentent chacun un radical -CH₂OH et R₁, R₄, R₅ et R₆ représentent chacun un radical -CHOH- et -R₂-R₃- représente un radical -CH=CH- **caractérisé en ce que** l'on déshydrate un dérivé de formule : 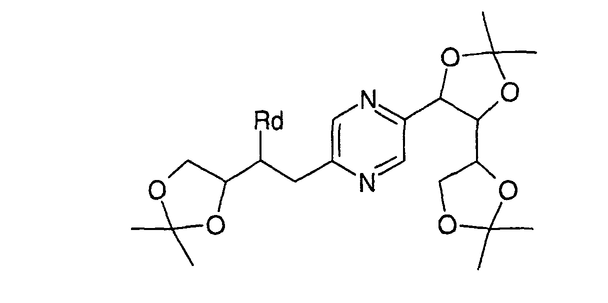 dans laquelle Rd représente un radical -OSO₂-Re et Re représente un radical méthyle, trifluorométhyle ou 4-méthylphényle ou un stéréoisomère d'un tel dérivé, puis déprotège les hydroxyles, isole le produit et le transforme éventuellement en sel.

13. Utilisation des composés de formule générale : 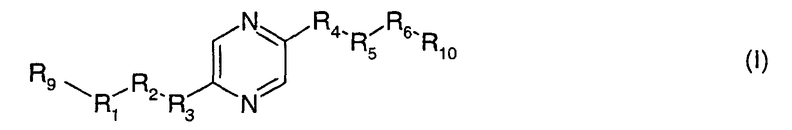 dans laquelle
R₉ et R₁₀ sont identiques et représentent un radical -CH₂OH, et soit
a) R₃ représente un radical méthylène, R₄ représente un radical -CHOH- et l'un des radicaux R₁, R₂, R₅ et R₆ représente un radical carbonyle, méthylène, -CHF-, -CH(NHR₇)- ou -CH(OR₈) et les autres représentent chacun un radical -CHOH-,
b) R₂ et R₅ représentent chacun un radical -CHOH-, R₃ représente un radical méthylène et R₁, R₄ et R₆ sont identiques et représentent chacun un radical carbonyle, méthylène, -CHF-, -CH(NHR₇)- ou -CH(OR₈),
c) R₁ et R₆ représentent chacun un radical -CHOH-, R₃ représente un radical méthylène et R₂, R₄ et R₅ sont identiques et représentent chacun un radical carbonyle, méthylène, -CHF-, -CH(NHR₇)- ou -CH(OR₈),
d) R₁, R₄, R₅ et R₆ représentent chacun un radical -CHOH- et -R₂-R₃-représente un radical -CH=CH-,
R₇ représente un atome d'hydrogène ou un radical alkyle, -CO-alk, -CO-Ar ou -CO-Het,
R₈ représente un radical alkyle, -alk-COOH ou -alk-OH,
alk représente un radical alkyle,
Ar représente un radical phényle ou phényle substitué par un ou plusieurs substituants choisis parmi un atome d'halogène, un radical alkyle, alcoxy, alcoxycarbonyle, amino, monoalkylamino ou dialkylamino,
Het représente un hétérocycle mono, di ou tricyclique saturé ou insaturé contenant 1 à 9 atomes de carbone et un ou plusieurs hétéroatomes choisis parmi oxygène, soufre et azote,
les radicaux et portions alkyle et alcoxy contenant 1 à 6 atomes de carbone en chaîne droite ou ramifiée,
ou un de ses stéréoisomères ou pour le composé pour lequel -R₂-R₃-représente un radical -CH=CH- ses formes cis ou trans ou un de ses sels avec un acide minéral ou organique pharmaceutiquement acceptable pour la préparation d'un médicament utile pour le traitement ou la prévention du diabète et des complications du diabète.

## Patentansprüche

1. Arzneimittel, enthaltend als Wirkstoff mindestens eine Verbindung der Formel (I) 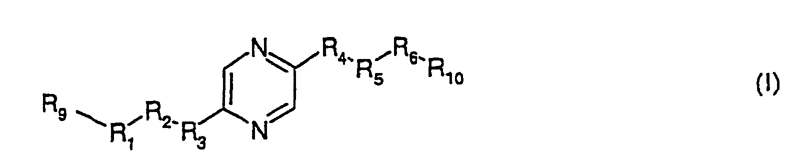 in der
R₉ und R₁₀ gleich sind und einen Rest -CH₂OH darstellen, und entweder
a) R₃ einen Rest Methylen bedeutet, R₄ ein Rest -CHOH- ist und einer der Reste R₁, R₂, R₅ und R₆ einen Rest Carbonyl, Methylen, -CHF-, -CH(NHR₇)- oder -CH(OR₈) darstellt und die anderen jeweils einen Rest -CHOH- bedeuten,
b) R₂ und R₅ jeweils einen Rest -CHOH- darstellen, R₃ ein Rest Methylen ist und R₁, R₄ und R₆ gleich sind und jeweils einen Rest Carbonyl, Methylen, -CHF-, -CH(NHR₇)- oder -CH(OR₈) bedeuten,
c) R₁ und R₆ jeweils einen Rest -CHOH- darstellen, R₃ ein Rest Methylen ist und R₂, R₄ und R₅ gleich sind und jeweils einen Rest Carbonyl, Methylen, -CHF-, -CH(NHR₇)- oder -CH(OR₈) bedeuten,
d) R₁, R₄, R₅ und R₆ jeweils einen Rest -CHOH- darstellen und -R₂-R₃- einen Rest -CH=CH- bedeutet,
R₇ ein Wasserstoffatom oder einen Rest Alkyl, -CO-alk, -CO-Ar oder -CO-Het darstellt,
R₈ einen Rest Alkyl, -alk-COOH oder -alk-OH darstellt, alk einen Rest Alkyl darstellt,
Ar einen Rest Phenyl oder Phenyl darstellt, substituiert durch einen oder mehrere Substituenten, ausgewählt unter einem Halogenatom, einem Rest Alkyl, Alkoxy, Alkoxycarbonyl, Amino, Monoalkylamino oder Dialkylamino,
Het einen mono-, di- oder tricyclischen, gesättigten oder ungesättigten Heterocyclus mit 1 bis 9 Kohlenstoffatomen und einem oder mehreren Heteroatomen, ausgewählt unter Sauerstoff, Schwefel und Stickstoff, darstellt,
die Reste und Teile Alkyl und Alkoxy 1 bis 6 Kohlenstoffatome in gerader oder verzweigter Kette enthalten,
oder eines ihrer Stereoisomeren und bei der Verbindung, in der -R₂-R₃- einen Rest -CH=CH- bedeutet, ihre Formen cis oder trans, sowie ihre Salze mit einer pharmazeutisch akzeptablen Mineralsäure oder organischen Säure.

2. Arzneimittel nach Anspruch 1, bei denen in den Verbindungen der Formel (I) Het einen Heterocyclus darstellt, ausgewählt unter den Ringen 2-, 3- oder 4-Pyridyl, Imidazolyl, Thiazolyl und Oxazolyl.

3. Arzneimittel nach Anspruch 1, bei denen die Verbindung der Formel (I) unter den folgenden Verbindungen ausgewählt wird:
1-[5-(3R,4-Dihydroxy-2-oxo-butyl)-pyrazin-2-yl]-butan-1R,2S,3R,4-tetraol,
1-[5-(3S,4-Dihydroxy-butyl)-pyrazin-2-yl]-butan-1R,2S,3R,4-tetraol,
1-[5-(2S-Fluor-3R,4-dihydroxy-butyl)-pyrazin-2-yl]-butan-1R,2S,3R,4-tetraol,
1-[5-(2R-Fluor-3R,4-dihydroxy-butyl)-pyrazin-2-yl]-butan-1R,2S,3R,4-tetraol,
1-[5-(2S-Amino-3S,4-dihydroxy-butyl)-pyrazin-2-yl]-butan-1R,2S,3R,4-tetraol,
1-[5-(2R-Amino-3S,4-dihydroxy-butyl)-pyrazin-2-yl]-butan-1R,2S,3R,4-tetraol,
1-{5-[2S-(N-Methyl)-amino-3S,4-dihydroxy-butyl]-pyrazin-2-yl}-butan-1R,2S,3R,4-tetraol,
1-{5-[2R-(N-Methyl)-amino-3S,4-dihydroxy-butyl]-pyrazin-2-yl}-butan-1R,2S,3R,4-tetraol,
1-{5-[2S-(N-Ethyl)-amino-3S,4-dihydroxy-butyl]-pyrazin-2-yl}-butan-1R,2S,3R,4-tetraol,
1-{5-[2R-(N-Ethyl)-amino-3S,4-dihydroxy-butyl]-pyrazin-2-yl}-butan-1R,2S,3R,4-tetraol,
1-{5-[2S-(N-n-Butyl)-amino-3S,4-dihydroxy-butyl]-pyrazin-2-yl}-butan-1R,2S,3R,4-tetraol,
1-{5-[2R-(N-n-Butyl)-amino-3S,4-dihydroxy-butyl]-pyrazin-2-yl}-butan-1R,2S,3R,4-tetraol,
1-{5-[2S-(N-Benzyl)-amino-3S,4-dihydroxy-butyl]-pyrazin-2-yl}-butan-1R,2S,3R,4-tetraol,
1-{5-[2R-(N-Benzyl)-amino-3S,4-dihydroxy-butyl]-pyrazin-2-yl}-butan-1R,2S,3R,4-tetraol,
1-{5-[2S-(N-Acetyl)-amino-3S,4-dihydroxy-butyl]-pyrazin-2-yl}-butan-1R,2S,3R,4-tetraol,
1-{5-[2R-(N-Acetyl)-amino-3S,4-dihydroxy-butyl]-pyrazin-2-yl}-butan-1R,2S,3R,4-tetraol,
1-{5-[2S-(N-Butanoyl)-amino-3S,4-dihydroxy-butyl]-pyrazin-2-yl}-butan-1R,2S,3R,4-tetraol,
1-{5-[2R-(N-Butanoyl)-amino-3S,4-dihydroxy-butyl]-pyrazin-2-yl}-butan-1R,2S,3R,4-tetraol,
1-{5-[2S-(N-Benzoyl)-amino-3S,4-dihydroxy-butyl]-pyrazin-2-yl}-butan-1R,2S,3R,4-tetraol,
1-{5-[2R-(N-Benzoyl)-amino-3S,4-dihydroxy-butyl]-pyrazin-2-yl}-butan-1R,2S,3R,4-tetraol,
1-[5-(2S-Methoxy-3R,4-dihydroxy-butyl)-pyrazin-2-yl]-butan-1R,2S,3R,4-tetraol,
1-[5-(2R-Methoxy-3R,4-dihydroxy-butyl)-pyrazin-2-yl]-butan-1R,2S,3R,4-tetraol,
1-[5-(2S-Ethoxy-3R,4-dihydroxy-butyl)-pyrazin-2-yl]-butan-1R,2S,3R,4-tetraol,
1-[5-(2R-Ethoxy-3R,4-dihydroxy-butyl)-pyrazin-2-yl]-butan-1R,2S,3R,4-tetraol,
1-[5-(2S-n-Butoxy-3S,4-dihydroxy-butyl)-pyrazin-2-yl]-butan-1R,2S,3R,4-tetraol,
1-[5-(2R-n-Butoxy-3R,4-dihydroxy-butyl)-pyrazin-2-yl]-butan-1R,2S,3R,4-tetraol,
1-{5-[2S-(2-Hydroxyethyl)-oxy-3R,4-dihydroxy-butyl]-pyrazin-2-yl}-butan-1R,2S,3R,4-tetraol,
1-{5-[2R-(2-Hydroxyethyl)-oxy-3R,4-dihydroxy-butyl]-pyrazin-2-yl}-butan-1R,2S,3R,4-tetraol,
1-{5-[2S-(3-Hydroxy-n-propyl)-oxy-3R,4-dihydroxy-butyl]-pyrazin-2-yl}-butan-1R,2S,3R,4-tetraol,
1-{5-[2R-(3-Hydroxy-n-propyl)-oxy-3R,4-dihydroxy-butyl]-pyrazin-2-yl}-butan-1R,2S,3R,4-tetraol,
1-{5-[2S-(Carboxymethyl)-oxy-3R,4-dihydroxy-butyl]-pyrazin-2-yl}-butan-1R,2S,3R,4-tetraol,
1-{5-[2R-(Carboxymethyl)-oxy-3R,4-dihydroxy-butyl]-pyrazin-2-yl}-butan-1R,2S,3R,4-tetraol,
1-{5-[2S-(3-Carboxy-n-propyl)-oxy-3R,4-dihydroxy-butyl]-pyrazin-2-yl}-butan-1R,2S,3R,4-tetraol,
1-{5-[2R-(3-Carboxy-n-propyl)-oxy-3R,4-dihydroxy-butyl]-pyrazin-2-yl}-butan-1R,2S,3R,4-tetraol,
1-[5-(2S,4-Dihydroxy-3-oxo-butyl)-pyrazin-2-yl]-butan-1R,2S,3R,4-tetraol,
1-[5-(2S,4-Dihydroxy-butyl)-pyrazin-2-yl]-butan-1R,2S,3R,4-tetraol,
1-[5-(3R-Fluor-2S,4-dihydroxy-butyl)-pyrazin-2-yl]-butan-1R,2S,3R,4-tetraol,
1-[5-(3S-Fluor-2S,4-dihydroxy-butyl)-pyrazin-2-yl]-butan-1R,2S,3R,4-tetraol,
1-[5-(3R-Amino-2R,4-dihydroxy-butyl)-pyrazin-2-yl]-butan-1R,2S,3R,4-tetraol,
1-[5-(3S-Amino-2R,4-dihydroxy-butyl)-pyrazin-2-yl]-butan-1R,2S,3R,4-tetraol,
1-{5-[3R-(N-Methyl) -amino-2R,4-dihydroxy-butyl]-pyrazin-2-yl}-butan-1R,2S,3R,4-tetraol,
1-{5-[3S-(N-Methyl)-amino-2R,4-dihydroxy-butyl]-pyrazin-2-yl}-butan-1R,2S,3R,4-tetraol,
1-{5-[3R-(N-Ethyl)-amino-2R,4-dihydroxy-butyl]-pyrazin-2-yl}-butan-2R,2S,3R,4-tetraol,
1-{5-[3S-(N-Ethyl)-amino-2R,4-dihydroxy-butyl]pyrazin-2-yl}-butan-1R,2S,3R,4-tetraol,
1-{5-[3R-(N-n-Butyl)-amino-2R,4-dihydroxy-butyl]-pyrazin-2-yl}-butan-1R,2S,3R,4-tetraol,
1-{5-[3S-(N-n-Butyl)-amino-2R,4-dihydroxy-butyl]-pyrazin-2-yl}-butan-1R,2S,3R,4-tetraol,
1-{5-[3R-(N-Benzyl)-amino-2R,4-dihydroxy-butyl]-pyrazin-2-yl}-butan-1R,2S,3R,4-tetraol,
1-{5-[3S-(N-Benzyl)-amino-2R,4-dihydroxy-butyl]-pyrazin-2-yl}-butan-1R,2S,3R,4-tetraol,
1-{5-[3R-(N-Acetyl)-amino-2R,4-dihydroxy-butyl]-pyrazin-2-yl}-butan-1R,2S,3R,4-tetraol,
1-{5-[3S-(N-Acetyl)-amino-2R,4-dihydroxy-butyl]-pyrazin-2-yl}-butan-1R,2S,3R,4-tetraol,
1-{5-[3R-(N-Butanoyl)-amino-2R,4-dihydroxy-butyl]-pyrazin-2-yl}-butan-1R,2S,3R,4-tetraol,
1-{5-[3S-(N-Butanoyl)-amino-2R,4-dihydroxy-butyl]-pyrazin-2-yl}-butan-1R,2S,3R,4-tetraol,
1-{5-[3R-(N-Benzoyl)-amino-2R,4-dihydroxy-butyl]-pyrazin-2-yl}-butan-1R,2S,3R,4-tetraol,
1-{5-[3S-(N-Benzoyl)-amino-2R,4-dihydroxy-butyl]-pyrazin-2-yl}-butan-1R,2S,3R,4-tetraol,
1-[5-(3R-Methoxy-2S,4-dihydroxy-butyl)-pyrazin-2-yl]-butan-1R,2S,3R,4-tetraol,
1-[5-(3S-Methoxy-2S,4-dihydroxy-butyl)-pyrazin-2-yl]-butan-1R,2S,3R,4-tetraol,
1-[5-(3R-Ethoxy-2S,4-dihydroxy-butyl)-pyrazin-2-yl]-butan-1R,2S,3R,4-tetraol,
1-[5-(3S-Ethoxy-2S,4-dihydroxy-butyl)-pyrazin-2-yl]-butan-1R,2S,3R,4-tetraol,
1-[5-(3R-n-Butoxy-2S,4-dihydroxy-butyl)-pyrazin-2-yl]-butan-1R,2S,3R,4-tetraol,
1-[5-(3S-n-Butoxy-2S,4-dihydroxy-butyl)-pyrazin-2-yl]-butan-1R,2S,3R,4-tetraol,
1-{5-[3R-(2-Hydroxyethyl)-oxy-2S,4-dihydroxy-butyl]-pyrazin-2-yl}-butan-1R,2S,3R,4-tetraol,
1-{5-[3S-(2-Hydroxyethyl)-oxy-2S,4-dihydroxy-butyl]-pyrazin-2-yl}-butan-1R,2S,3R,4-tetraol,
1-{5-[3R-(3-Hydroxy-n-propyl)-oxy-2S,4-dihydroxy-butyl]-pyrazin-2-yl}-butan-1R,2S,3R,4-tetraol,
1-{5-[3S-(3-Hydroxy-n-propyl)-oxy-2S,4-dihydroxy-butyl]-pyrazin-2-yl}-butan-1R,2S,3R,4-tetraol,
1-{5-[3R-(Carboxymethyl)-oxy-2S,4-dihydroxy-butyl]-pyrazin-2-yl}-butan-1R,2S,3R,4-tetraol,
1-{5-[3S-(Carboxymethyl)-oxy-2S,4-dihydroxy-butyl]-pyrazin-2-yl}-butan-1R,2S,3R,4-tetraol,
1-{5-[3R-(Carboxy-n-propyl)-oxy-2S,4-dihydroxy-butyl]-pyrazin-2-yl}-butan-1R,2S,3R,4-tetraol,
1-{5-[3S-(Carboxy-n-propyl)-oxy-2S,4-dihydroxy-butyl]-pyrazin-2-yl}-butan-1R,2S,3R,4-tetraol,
4-[5-(2S,3R,4-Trihydroxy-butyl)-pyrazin-2-yl]-1,3S,4R-trihydroxy-butan-2-on,
1-[5-(2S,3R,4-Trihydroxy-butyl)-pyrazin-2-yl]-butan-1R,2R,4-triol,
1-[5-(2S,3R,4-Trihydroxy-butyl)-pyrazin-2-yl]-3R-fluor-butan-1R,2S,4-triol,
1-[5-(2S,3R,4-Trihydroxy-butyl)-pyrazin-2-yl]-3S-fluor-butan-1R,2S,4-triol,
1-[5-(2S,3R,4-Trihydroxy-butyl)-pyrazin-2-yl]-3R-amino-butan-1R,2R,4-triol,
1-[5-(2S,3R,4-Trihydroxy-butyl)-pyrazin-2-yl]-3S-amino-butan-1R,2R,4-triol,
1-[5-(2S,3R,4-Trihydroxy-butyl)-pyrazin-2-yl]-3R-(N-methyl)-amino-butan-1R,2R,4-triol,
1-[5-(2S,3R,4-Trihydroxy-butyl)-pyrazin-2-yl]-3S-(N-methyl)-amino-butan-1R,2R,4-triol,
1-[5-(2S,3R,4-Trihydroxy-butyl)-pyrazin-2-yl]-3R-(N-ethyl)-amino-butan-1R,2R,4-triol,
1-[5-(2S,3R,4-Trihydroxy-butyl)-pyrazin-2-yl]-3S-(N-ethyl)-amino-butan-1R,2R,4-triol,
1-[5-(2S,3R,4-Trihydroxy-butyl)-pyrazin-2-yl]-3R-(N-n-butyl)-amino-butan-1R,2R,4-triol,
1-[5-(2S,3R,4-Trihydroxy-butyl)-pyrazin-2-yl]-3S-(N-n-butyl)-amino-butan-1R,2R,4-triol,
1-[5-(2S,3R,4-Trihydroxy-butyl)-pyrazin-2-yl]-3R-(N-benzyl)-amino-butan-1R,2R,4-triol,
1-[5-(2S,3R,4-Trihydroxy-butyl)-pyrazin-2-yl]-3S-(N-benzyl)-amino-butan-1R,2R,4-triol,
1-[5-(2S,3R,4-Trihydroxy-butyl)-pyrazin-2-yl]-3R-(N-acetyl)-amino-butan-1R,2R,4-triol,
1-[5-(2S,3R,4-Trihydroxy-butyl)-pyrazin-2-yl]-3S-(N-acetyl)-amino-butan-1R,2R,4-triol,
1-[5-(2S,3R,4-Trihydroxy-butyl)-pyrazin-2-yl]-3R-(N-butanoyl)-amino-butan-1R,2R,4-triol,
1-[5-(2S,3R,4-Trihydroxy-butyl)-pyrazin-2-yl]-3S-(N-butanoyl)-amino-butan-1R,2R,4-triol,
1-[5-(2S,3R,4-Trihydroxy-butyl)-pyrazin-2-yl)-3R-(N-benzoyl)-amino-butan-1R,2R,4-triol,
1-[5-(2S,3R,4-Trihydroxy-butyl)-pyrazin-2-yl]-3S-(N-benzoyl)-amino-butan-1R,2R,4-triol,
1-[5-(2S,3R,4-Trihydroxy-butyl)-pyrazin-2-yl]-3R-methoxy-butan-1R,2S,4-triol,
1-[5-(2S,3R,4-Trihydroxy-butyl)-pyrazin-2-yl]-3S-methoxy-butan-1R,2S,4-triol,
1-[5-(2S,3R,4-Trihydroxy-butyl)-pyrazin-2-yl]-3R-ethoxy-butan-1R,2S,4-triol,
1-[5-(2S,3R,4-Trihydroxy-butyl)-pyrazin-2-yl]-3S-ethoxy-butan-1R,2S,4-triol,
1-[5-(2S,3R,4-Trihydroxy-butyl)-pyrazin-2-yl]-3R-n-butoxy-butan-1R,2S,4-triol,
1-[5-(2S,3R,4-Trihydroxy-butyl)-pyrazin-2-yl]-3S-n-butoxy-butan-1R,2S,4-triol,
1-[5-(2S,3R,4-Trihydroxy-butyl)-pyrazin-2-yl]-3R-(2-hydroxy-ethyl)-oxy-butan-1R,2S,4-triol,
1-[5-(2S,3R,4-Trihydroxy-butyl)-pyrazin-2-yl]-3S-(2-hydroxy-ethyl)-oxy-butan-1R,2S,4-triol,
1-[5-(2S,3R,4-Trihydroxy-butyl)-pyrazin-2-yl]-3R-(3-hydroxy-n-propyl)-oxy-butan-1R,2S,4-triol,
1-[5-(2S,3R,4-Trihydroxy-butyl)-pyrazin-2-yl]-3S-(3-hydroxy-n-propyl)-oxy-butan-1R,2S,4-triol,
1-[5-(2S,3R,4-Trihydroxy-butyl)-pyrazin-2-yl]-3R-(carboxymethyl)-oxy-butan-1R,2S,4-triol,
1-[5-(2S,3R,4-Trihydroxy-butyl)-pyrazin-2-yl]-3S-(carboxymethyl)-oxy-butan-1R,2S,4-triol,
1-[5-(2S,3R,4-Trihydroxy-butyl)-pyrazin-2-yl]-3R-(3-carboxy-n-propyl)-oxy-butan-1R,2S,4-triol,
1-[5-(2S,3R,4-Trihydroxy-butyl)-pyrazin-2-yl]-3S-(3-carboxy-n-propyl)-oxy-butan-1R,2S,4-triol,
1-[5-(2S,3R,4-Trihydroxy-butyl)-pyrazin-2-yl]-1R,3R,4-trihydroxy-butan-2-on,
1-[5-(2S,3R,4-Trihydroxy-butyl)-pyrazin-2-yl]-butan-1R,3R,4-triol,
1-[5-(2S,3R,4-Trihydroxy-butyl)-pyrazin-2-yl]-2R-fluor-butan-1R,3R,4-triol,
1-[5-(2S,3R,4-Trihydroxy-butyl)-pyrazin-2-yl]-2S-fluor-butan-1R,3R,4-triol,
1-[5-(2S,3R,4-Trihydroxy-butyl)-pyrazin-2-yl]-2R-amino-butan-1S,3S,4-triol,
1-[5-(2S,3R,4-Trihydroxy-butyl)-pyrazin-2-yl]-2S-amino-butan-1S,3S,4-triol,
1-[5-(2S,3R,4-Trihydroxy-butyl)-pyrazin-2-yl]-2R-(N-methyl)-amino-butan-1S,3S,4-triol,
1-[5-(2S,3R,4-Trihydroxy-butyl)-pyrazin-2-yl]-2S-(N-methyl)-amino-butan-1S,3S,4-triol,
1-[5-(2S,3R,4-Trihydroxy-butyl)-pyrazin-2-yl]-2R-(N-ethyl)-amino-butan-1S,3S,4-triol,
1-[5-(2S,3R,4-Trihydroxy-butyl)-pyrazin-2-yl]-2S-(N-ethyl)-amino-butan-1S,3S,4-triol,
1-[5-(2S,3R,4-Trihydroxy-butyl)-pyrazin-2-yl]-2R-(N-n-butyl)-amino-butan-1S,3S,4-triol,
1-[5-(2S,3R,4-Trihydroxy-butyl)-pyrazin-2-yl]-2S-(N-n-butyl)-amino-butan-1S,3S,4-triol,
1-[5-(2S,3R,4-Trihydroxy-butyl)-pyrazin-2-yl]-2R-(N-benzyl)-amino-butan-1S,3S,4-triol,
1-[5-(2S,3R,4-Trihydroxy-butyl)-pyrazin-2-yl]-2S-(N-benzyl)-amino-butan-1S,3S,4-triol,
1-[5-(2S,3R,4-Trihydroxy-butyl)-pyrazin-2-yl]-2R-(N-acetyl)-amino-butan-1S,3S,4-triol,
1-[5-(2S,3R,4-Trihydroxy-butyl)-pyrazin-2-yl]-2S-(N-acetyl)-amino-butan-1S,3S,4-triol,
1-[5-(2S,3R,4-Trihydroxy-butyl)-pyrazin-2-yl]-2R-(N-butanoyl)-amino-butan-1S,3S,4-triol,
1-[5(2S,3R,4-Trihydroxy-butyl)-pyrazin-2-yl]-2S-(N-butanoyl)-amino-butan-1S,3S,4-triol,
1-[5-(2S,3R,4-Trihydroxy-butyl)-pyrazin-2-yl]-2R-(N-benzoyl)-amino-butan-1S,3S,4-triol,
1-[5-(2S,3R,4-Trihydroxy-butyl)-pyrazin-2-yl]-2S-(N-benzoyl)-amino-butan-1S,3S,4-triol,
1-[5-(2S,3R,4-Trihydroxy-butyl)-pyrazin-2-yl]-2R-methoxy-butan-1R,3R,4-triol,
1-[5-(2S,3R,4-Trihydroxy-butyl)-pyrazin-2-yl)-2S-methoxy-butan-1R,3R,4-triol,
1-[5-(2S,3R,4-Trihydroxy-butyl)-pyrazin-2-yl]-2R-ethoxy-butan-1R,3R,4-triol,
1-[5-(2S,3R,4-Trihydroxy-butyl)-pyrazin-2-yl]-2S-ethoxy-butan-1R,3R,4-triol,
1-[5-(2S,3R,4-Trihydroxy-butyl)-pyrazin-2-yl]-2R-n-butoxy-butan-1R,3R,4-triol,
1-[5-(2S,3R,4-Trihydroxy-butyl)-pyrazin-2-yl]-2S-n-butoxy-butan-1R,3R,4-triol,
1-[5-(2S,3R,4-Trihydroxy-butyl)-pyrazin-2-yl]-2R-(2-hydroxy-ethyl)-oxy-butan-1R,3R,4-triol,
1-[5-(2S,3R,4-Trihydroxy-butyl)-pyrazin-2-yl]-2S-(2-hydroxy-ethyl)-oxy-butan-1R,3R,4-triol,
1-[5-(2S,3R,4-Trihydroxy-butyl)-pyrazin-2-yl]-2R-(3-hydroxy-n-propyl)-oxy-butan-1R,3R,4-triol,
1-[5-(2S,3R,4-Trihydroxy-butyl)-pyrazin-2-yl]-2S-(3-hydroxy-n-propyl)-oxy-butan-1R,3R,4-triol,
1-[5-(2S,3R,4-Trihydroxy-butyl)-pyrazin-2-yl]-2R-(carboxymethyl)-oxy-butan-1R,3R,4-triol,
1-[5-(2S,3R,4-Trihydroxy-butyl)-pyrazin-2-yl]-2S-(carboxymethyl)-oxy-butan-1R,3R,4-triol,
1-[5-(2S,3R,4-Trihydroxy-butyl)-pyrazin-2-yl]-2R-(3-carboxy-n-propyl)-oxy-butan-1R,3R,4-triol,
1-[5-(2S,3R,4-Trihydroxy-butyl)-pyrazin-2-yl]-2S-(3-carboxy-n-propyl)-oxy-butan-1R,3R,4-triol,
1-[5-(2S,4-Dihydroxy-3-oxo-butyl)-pyrazin-2-yl]-2S,4-dihydroxy-butan-1,3-dion,
4-[5-(2S,4-Dihydroxy-butyl)-pyrazin-2-yl]-butan-1,3S-diol,
4-[5-(3R-Fluor-2S,4-dihydroxy-butyl)-pyrazin-2-yl]-2R,4R-difluor-butan-1,3S-diol,
4-[5-(3S-Fluor-2S,4-dihydroxy-butyl)-pyrazin-2-yl]-2S,4S-difluor-butan-1,3S-diol,
1-[5-(3R-Amino-2S,4-dihydroxy-butyl)-pyrazin-2-yl]-2R,4R-diamino-butan-1,3S-diol,
1-[5-(3S-Amino-2S,4-dihydroxy-butyl)-pyrazin-2-yl]-2S,4S-diamino-butan-1,3S-diol,
1-{5-[3R-(N-Methyl)-amino-2S,4-dihydroxy-butyl]-pyrazin-2-yl}-2R,4R-di-(N-methyl)-amino-butan-1,3S-diol,
1-{5-[3S-(N-Methyl)-amino-2S,4-dihydroxy-butyl]-pyrazin-2-yl}-2S,4S-di-(N-methyl)-amino-butan-1,3S-diol,
1-{5-[3R-(N-Ethyl)-amino-2S,4-dihydroxy-butyl]-pyrazin-2-yl}-2R,4R-di-(N-ethyl)-amino-butan-1,3S-diol,
1-{5-[3S-(N-Ethyl)-amino-2S,4-dihydroxy-butyl]-pyrazin-2-yl}-2S,4S-di-(N-ethyl)-amino-butan-1,3S-diol,
1-{5-[3R-(N-n-Butyl)-amino-2S,4-dihydroxy-butyl]-pyrazin-2-yl}-2R,4R-di-(N-n-butyl)-amino-butan-1,3S-diol,
1-{5-[3S-(N-n-Butyl)-amino-2S,4-dihydroxy-butyl]-pyrazin-2-yl}-2S,4S-di-(N-n-butyl)-amino-butan-1,3S-diol,
1-{5-[3R-(N-Benzyl)-amino-2S,4-dihydroxy-butyl]-pyrazin-2-yl}-2R,4R-di-(N-benzyl)-amino-butan-1,3S-diol,
1-{5-[3S-(N-Benzyl)-amino-2S,4-dihydroxy-butyl]-pyrazin-2-yl}-2S,4S-di-(N-benzyl)-amino-butan-1,3S-diol,
1-{5-[3R-(N-Acetyl)-amino-2S,4-dihydroxy-butyl]-pyrazin-2-yl}-2R,4R-di-(N-acetyl)-amino-butan-1,3S-diol,
1-{5-[3S-(N-Acetyl)-amino-2S,4-dihydroxy-butyl]-pyrazin-2-yl}-2S,4S-di-(N-acetyl)-amino-butan-1,3S-diol,
1-{5-[3R-(N-Butanoyl)-amino-2S,4-dihydroxy-butyl]-pyrazin-2-yl}-2R,4R-di-(N-butanoyl)-amino-butan-1,3S-diol,
1-{5-[3S-(N-Butanoyl)-amino-2S,4-dihydroxy-butyl]-pyrazin-2-yl}-2S,4S-di-(N-butanoyl)-amino-butan-1,3S-diol,
1-{5-[3R-(N-Benzoyl)-amino-2S,4-dihydroxy-butyl]-pyrazin-2-yl}-2R,4R-di-(N-benzoyl)-amino-butan-1,3S-diol,
1-{5-[3S-(N-Benzoyl)-amino-2S,4-dihydroxy-butyl]-pyrazin-2-yl}-2S,4S-di-(N-benzoyl)-amino-butan-1,3S-diol,
1-[5-(3R-Methoxy-2S,4-dihydroxy-butyl)-pyrazin-2-yl]-2R,4R-dimethoxy-butan-1,3S-diol,
1-[5-(3S-Methoxy-2S,4-dihydroxy-butyl)-pyrazin-2-yl]-2S,4S-dimethoxy-butan-1,3S-diol,
1-[5-(3R-Ethoxy-2S,4-dihydroxy-butyl)-pyrazin-2-yl]-2R,4R-di-ethoxy-butan-1,3S-diol,
1-[5-(3S-Ethoxy-2S,4-dihydroxy-butyl)-pyrazin-2-yl]-2S,4S-di-ethoxy-butan-1,3S-diol,
1-[5-(3R-n-Butoxy-2S,4-dihydroxy-butyl)-pyrazin-2-yl]-2R,4R-di-n-butoxy-butan-1,3S-diol,
1-[5-(3S-n-Butoxy-2S,4-dihydroxy-butyl)-pyrazin-2-yl]-2S,4S-di-n-butoxy-butan-1,3S-diol,
1-{5-[3R-(2-Hydroxyethyl)-oxy-2S,4-dihydroxy-butyl]-pyrazin-2-yl}-2R,4R-di-(2-hydroxyethyl)-oxy-butan-1,3S-diol,
1-{5-[3S-(2-Hydroxyethyl)-oxy-2S,4-dihydroxy-butyl]-pyrazin-2-yl}-2S,4S-di-(2-hydroxyethyl)-oxy-butan-1,3S-diol,
1-{5-[3R-(3-Hydroxy-n-propyl)-oxy-2S,4-dihydroxy-butyl]-pyrazin-2-yl}-2R,4R-di-(3-hydroxy-n-propyl)-oxy-butan-1,3S-diol,
1-{5-[3S-(3-Hydroxy-n-propyl)-oxy-2S,4-dihydroxy-butyl]-pyrazin-2-yl}-2S,4S-di-(3-hydroxy-n-propyl)-oxy-butan-1,3S-diol,
1-{5-[3R-(Carboxymethyl)-oxy-2S,4-dihydroxy-butyl]-pyrazin-2-yl}-2R,4R-di-(carboxymethyl)-oxy-butan-1,3S-diol,
1-{5-[3S-(Carboxymethyl)-oxy-2S,4-dihydroxy-butyl]-pyrazin-2-yl}-2S,4S-di-(carboxymethyl)-oxy-butan-1,3S-diol,
1-{5-[3R-(3-Carboxy-n-propyl)-oxy-2S,4-dihydroxy-butyl]-pyrazin-2-yl}-2R,4R-di-(3-carboxy-n-propyl)-oxy-butan-1,3S-diol,
1-{5-[3S-(3-Carboxy-n-propyl)-oxy-2S,4-dihydroxy-butyl]-pyrazin-2-yl}-2S,4S-di-(3-carboxy-n-propyl)-oxy-butan-1,3S-diol,
1-[5-(3R,4-Dihydroxy-2-oxo-butyl)-pyrazin-2-yl]-3R,4-dihydroxy-butan-1,2-dion,
4-[5-(2S,4-Dihydroxy-butyl)-pyrazin-2-yl]-butan-1,3S-diol,
4-[5-(3R-Fluor-2S,4-dihydroxy-butyl)-pyrazin-2-yl]-2R,4R-difluor-butan-1,3S-diol,
4-[5-(3S-Fluor-2S,4-dihydroxy-butyl)-pyrazin-2-yl]-2S,4S-difluor-butan-1,3S-diol,
4-[5-(3R-Amino-2S,4-dihydroxy-butyl)-pyrazin-2-yl]-2R,4R-diamino-butan-1,3S-diol,
4-[5-(3S-Amino-2S,4-dihydroxy-butyl)-pyrazin-2-yl]-2S,4S-diamino-butan-1,3S-diol,
4-{5-[3R-(N-Methyl)-amino-2S,4-dihydroxy-butyl]-pyrazin-2-yl}-2R,4R-di-(N-methyl)-amino-butan-1,3S-diol,
4-{5-[3S-(N-Methyl)-amino-2S,4-dihydroxy-butyl]-pyrazin-2-yl}-2S,4S-di-(N-methyl)-amino-butan-1,3S-diol,
4-{5-[3R-(N-Ethyl)-amino-2S,4-dihydroxy-butyl]-pyrazin-2-yl}-2R,4R-di-(N-ethyl)-amino-butan-1,3S-diol,
4-{5-[3S-(N-Ethyl)-amino-2S,4-dihydroxy-butyl]-pyrazin-2-yl}-2S,4S-di-(N-ethyl)-amino-butan-1,3S-diol,
4-{5-[3R-(N-n-Butyl)-amino-2S,4-dihydroxy-butyl]-pyrazin-2-yl}-2R,4R-di-(N-n-butyl)-amino-butan-1,3S-diol,
4-{5-[3S-(N-n-Butyl)-amino-2S,4-dihydroxy-butyl]-pyrazin-2-yl}-2S,4S-di-(N-n-butyl)-amino-butan-1,3S-diol,
4-{5-[3R-(N-Benzyl)-amino-2S,4-dihydroxy-butyl]-pyrazin-2-yl}-2R,4R-di-(N-benzyl)-amino-butan-1,3S-diol,
4-{5-[3S-(N-Benzyl)-amino-2S,4-dihydroxy-butyl]-pyrazin-2-yl}-2S,4S-di-(N-benzyl)-amino-butan-1,3S-diol,
4-{5-[3R-(N-Acetyl)-amino-2S,4-dihydroxy-butyl]-pyrazin-2-yl}-2R,4R-di-(N-acetyl)-amino-butan-1,3S-diol,
4-{5-[3S-(N-Acetyl)-amino-2S,4-dihydroxy-butyl]-pyrazin-2-yl}-2S,4S-di-(N-acetyl)-amino-butan-1,3S-diol,
4-{5-[3R-(N-Butanoyl)-amino-2S,4-dihydroxy-butyl]-pyrazin-2-yl}-2R,4R-di-(N-butanoyl)-amino-butan-1,3S-diol,
4-{5-[3S-(N-Butanoyl)-amino-2S,4-dihydroxy-butyl]-pyrazin-2-yl}-2S,4S-di-(N-butanoyl)-amino-butan-1,3S-diol,
4-{5-[3R-(N-Benzoyl)-amino-2S,4-dihydroxy-butyl]-pyrazin-2-yl}-2R,4R-di-(N-benzoyl)-amino-butan-1,3S-diol,
4-{5-[3S-(N-Benzoyl)-amino-2S,4-dihydroxy-butyl]-pyrazin-2-yl}-2S,4S-di-(N-benzoyl)-amino-butan-1,3S-diol,
4-[5-(3R-Methoxy-2S,4-dihydroxy-butyl)-pyrazin-2-yl]-2R,4R-dimethoxy-butan-1,3S-diol,
4-[5-(3S-Methoxy-2S,4-dihydroxy-butyl)-pyrazin-2-yl]-2S,4S-dimethoxy-butan-1,3S-diol,
4-[5-(3R-Ethoxy-2S,4-dihydroxy-butyl)-pyrazin-2-yl]-2R,4R-diethoxy-butan-1,3S-diol,
4-[5-(3S-Methoxy-2S,4-dihydroxy-butyl)-pyrazin-2-yl]-2S,4S-diethoxy-butan-1,3S-diol,
4-[5-(3R-n-Butoxy-2S,4-dihydroxy-butyl)-pyrazin-2-yl]-2R,4R-di-n-butoxy-butan-1,3S-diol,
4-[5-(3S-n-Butoxy-2S,4-dihydroxy-butyl)-pyrazin-2-yl]-2S,4S-di-n-butoxy-butan-1,3S-diol,
4-{5-[3R-(2-Hydroxyethyl)-oxy-2S,4-dihydroxy-butyl)-pyrazin-2-yl]-2R,4R-di-(2-hydroxyethyl)-oxy-butan-1,3S-diol,
4-{5-[3S-(2-Hydroxyethyl)-oxy-2S,4-dihydroxy-butyl)-pyrazin-2-yl]-2S,4S-di-(2-hydroxyethyl)-oxy-butan-1,3S-diol,
4-{5-[3R-(3-Hydroxy-n-propyl)-oxy-2S,4-dihydroxy-butyl)-pyrazin-2-yl]-2R,4R-di-(3-hydroxy-n-propyl)-oxy-butan-1,3S-diol,
4-{5-[3S-(3-Hydroxy-n-propyl)-oxy-2S,4-dihydroxy-butyl)-pyrazin-2-yl]-2S,4S-di-(3-hydroxy-n-propyl)-oxy-butan-1,3S-diol,
4-{5-[3R-(Carboxymethyl)-oxy-2S,4-dihydroxy-butyl)-pyrazin-2-yl]-2R,4R-di-(carboxymethyl)-oxy-butan-1,3S-diol,
4-{5-[3S-(Carboxymethyl)-oxy-2S,4-dihydroxy-butyl)-pyrazin-2-yl]-2S,4S-di-(carboxymethyl)-oxy-butan-1,3S-diol,
4-{5-[3R-(3-Carboxy-n-propyl)-oxy-2S,4-dihydroxy-butyl)-pyrazin-2-yl]-2R,4R-di-(3-Carboxy-n-propyl)-oxy-butan-1,3S-diol,
4-{5-[3S-(3-Carboxy-n-propyl)-oxy-2S,4-dihydroxy-butyl)-pyrazin-2-yl]-2S,4S-di-(3-Carboxy-n-propyl)-oxy-butan-1,3S-diol,
1-[5-(3S,4-Dihydroxy-1E-butenyl)-pyrazin-2-yl]-butan-1R,2S,3R,4-tetraol,
und ihre Salze mit einer pharmazeutisch akzeptablen Mineralsäure oder organischen Säure.

4. Arzneimittel, enthaltend als Wirkstoff mindestens eine Verbindung der Formel (I) nach Anspruch 1, in der
R₉ und R₁₀ jeweils einen Rest -CH₂OH darstellen, und entweder
a) R₃ einen Rest Methylen bedeutet, R₄ ein Rest -CHOH- ist und einer der Reste R₁, R₂, R₅ und R₆ einen Rest -CHF- oder -CH(OR₈) darstellt und die anderen jeweils einen Rest -CHOH- bedeuten;
b) R₂ und R₅ jeweils einen Rest -CHOH- darstellen, R₃ ein Rest Methylen ist und R₁, R₄ und R₆ gleich sind und jeweils einen Rest -CH(OR₈) bedeuten,
c) R₁ und R₆ jeweils einen Rest -CHOH- darstellen, R₃ ein Rest Methylen ist und R₂, R₄ und R₅ gleich sind und jeweils einen Rest -CH(OR₈) bedeuten,
d) R₁, R₄, R₅ und R₆ jeweils einen Rest -CHOH- darstellen und -R₂-R₃- einen Rest -CH=CH- bedeutet,
R₈ einen Rest Alkyl darstellt,
ihre Stereoisomeren, die Formen cis und trans bei der Verbindung, in der -R₂-R₃- eine Kette -CH=CH- bedeutet, sowie ihre Salze mit einer pharmazeutisch akzeptablen Mineralsäure oder organischen Säure.

5. Arzneimittel, enthaltend als Wirkstoff mindestens eine Verbindung der Formel (I) nach Anspruch 1, ausgewählt unter den folgenden Verbindungen:
1-[5-(3S,4-Dihydroxy-butyl)-pyrazin-2-yl]-butan-1R,2S,3R,4-tetraol,
1-[5-(3S,4-Dihydroxy-1E-butenyl)-pyrazin-2-yl]-butan-1R,2S,3R,4-tetraol,
1-[5-(2S-Methoxy-3R,4-dihydroxy-butyl)-pyrazin-2-yl]-butan-1R,2S,3R,4-tetraol,
1-[5-(2R-Fluor-3R,4-dihydroxy-butyl)-pyrazin-2-yl]-butan-1R,2S,3R,4-tetraol,
1-[5-(2S,4-Dihydroxy-3R-methoxy-butyl)-pyrazin-2-yl]-butan-1R,2S,3R,4-tetraol,
1-[5-(3R,4-Dihydroxy-2S-methoxy-butyl)-pyrazin-2-yl]-3R,4Rdimethoxy-butan-1,2-diol,
und ihre Salze mit einer pharmazeutisch akzeptablen Mineralsäure oder organischen Säure.

6. Verbindungen der Formel (I) 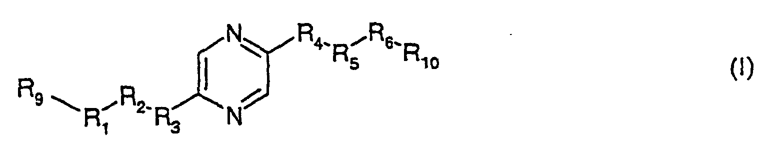 in der
R₉ und R₁₀ jeweils einen Rest -CH₂OH darstellen, und entweder
a) R₃ einen Rest Methylen bedeutet, R₄ ein Rest -CHOH- ist und einer der Reste R₁, R₂, R₅ und R₆ einen Rest Carbonyl, Methylen, -CHF-, -CH(NHR₇)- oder -CH(OR₈) darstellt und die anderen jeweils einen Rest -CHOH- bedeuten,
b) R₂ und R₅ jeweils einen Rest -CHOH- darstellen, R₃ ein Rest Methylen ist und R₁, R₄ und R₆ gleich sind und jeweils einen Rest Carbonyl, Methylen, -CHF-, -CH(NHR₇)- oder -CH(OR₈) bedeuten,
c) R₁ und R₆ jeweils einen Rest -CHOH- darstellen, R₃ ein Rest Methylen ist und R₂, R₄ und R₅ gleich sind und jeweils einen Rest Carbonyl, Methylen, -CHF-, -CH(NHR₇)- oder -CH(OR₈) bedeuten,
d) R₁, R₄, R₅ und R₆ jeweils einen Rest -CHOH- darstellen und -R₂-R₃- einen Rest -CH=CH- bedeutet,
R₇ ein Wasserstoffatom oder einen Rest Alkyl, -CO-alk, -CO-Ar oder -CO-Het darstellt,
R₈ einen Rest Alkyl, -alk-COOH oder -alk-OH darstellt, alk einen Rest Alkyl darstellt,
Ar einen Rest Phenyl oder Phenyl darstellt, substituiert durch einen oder mehrere Substituenten, ausgewählt unter einem Halogenatom, einem Rest Alkyl, Alkoxy, Alkoxycarbonyl, Amino, Monoalkylamino oder Dialkylamino,
Het einen mono-, di- oder tricyclischen, gesättigten oder ungesättigten Heterocyclus mit 1 bis 9 Kohlenstoffatomen und einem oder mehreren Heteroatomen, ausgewählt unter Sauerstoff, Schwefel und Stickstoff, darstellt,
die Reste und Teile Alkyl und Alkoxy 1 bis 6 Kohlenstoffatome in gerader oder verzweigter Kette enthalten,
oder eines ihrer Stereoisomeren oder bei den Verbindungen, in denen -R₂-R₃- einen Rest -CH=CH- bedeutet, ihre Formen cis oder trans, sowie eines ihrer Salze, mit Ausnahme der Verbindung der Formel:

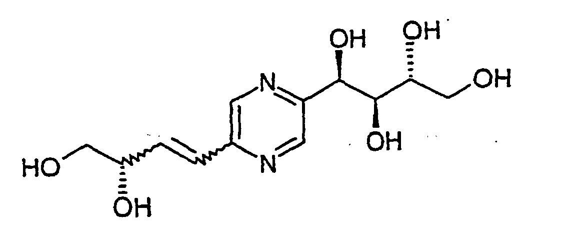

7. Verfahren zur Herstellung der Verbindungen der Formel (I) nach Anspruch 6, worin R₉ und R₁₀ jeweils einen Rest -CH₂OH darstellen, und entweder R₃ einen Rest Methylen bedeutet, R₄ ein Rest -CHOHist und einer der Reste R₁, R₂, R₅ und R₆ einen Rest Carbonyl darstellt und die anderen jeweils einen Rest -CHOH- bedeuten, oder R₁ und R₆ jeweils einen Rest -CHOH- darstellen, R₃ ein Rest Methylen ist und R₂, R₄ und R₅ gleich sind und jeweils einen Rest Carbonyl bedeuten, oder R₂ und R₅ jeweils einen Rest -CHOH- darstellen, R₃ ein Rest Methylen ist und R₁, R₄ und R₆ gleich sind und jeweils einen Rest Carbonyl bedeuten, **dadurch gekennzeichnet, daß** man ein Derivat oder ein Stereoisomer eines solchen Derivates oxidiert, ausgewählt unter den Formeln: 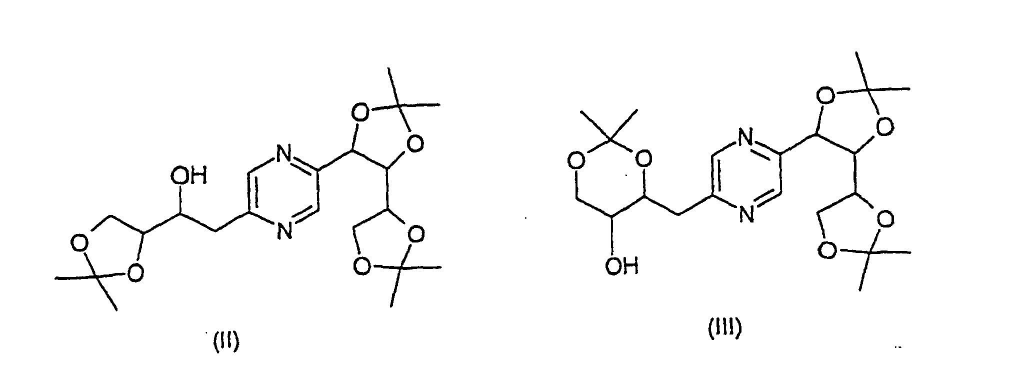 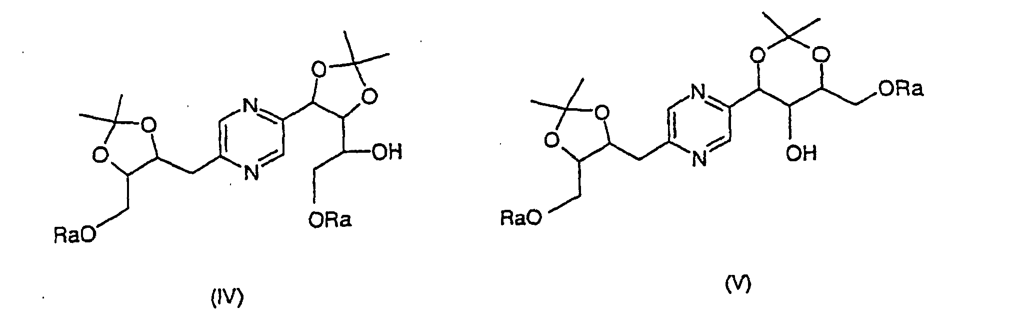 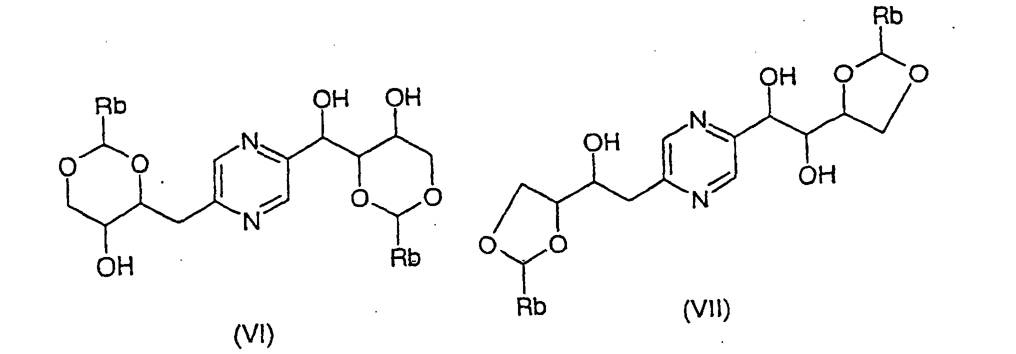 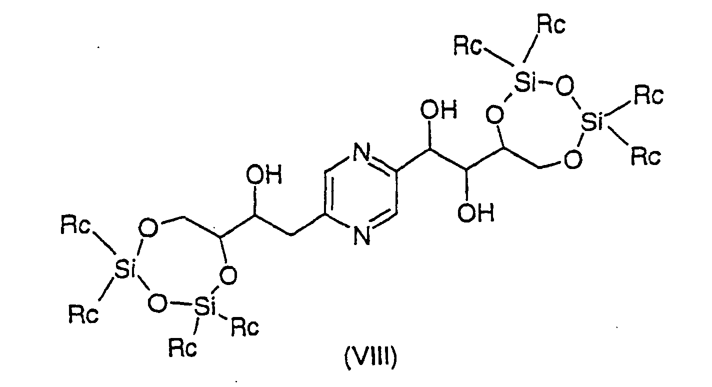 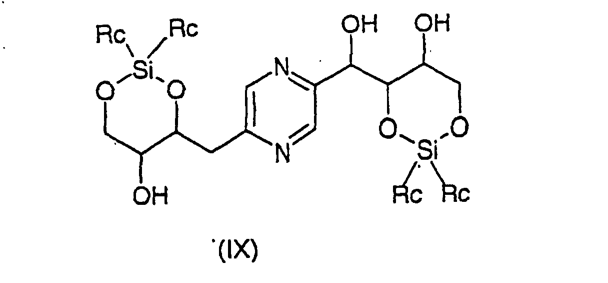 in denen Ra einen Rest Trialkylsilyl, Alkyldiphenylsilyl oder Dialkylphenylsilyl darstellt, Rb einen Rest Phenyl bedeutet, gegebenenfalls substituiert durch mindestens einen Rest Alkoxy, und Rc einen Rest Alkyl oder Phenyl darstellt, wobei die Reste und Teile Alkyl und Alkoxy 1 bis 6 Kohlenstoffatome in gerader oder verzweigter Kette enthalten, und man anschließend die Schutzgruppen von den Hydroxylen entfernt, das Produkt isoliert und gegebenenfalls in Salz überführt.

8. Verfahren zur Herstellung der Verbindungen der Formel (I) nach Anspruch 6, worin R₉ und R₁₀ jeweils einen Rest -CH₂OH darstellen, und entweder R₃ einen Rest Methylen bedeutet, R₄ ein Rest -CHOHist und einer der Reste R₁, R₂, R₅ und R₆ einen Rest Methylen darstellt und die anderen jeweils einen Rest -CHOH- bedeuten, oder R₁ und R₆ jeweils einen Rest -CHOH- darstellen, R₃ ein Rest Methylen ist und R₂, R₄ und R₅ gleich sind und jeweils einen Rest Methylen bedeuten, oder R₂ und R₅ jeweils einen Rest -CHOH- darstellen und R₁, R₃, R₄ und R₆ jeweils einen Rest Methylen bedeuten, **dadurch gekennzeichnet, daß** man ein Chlorthionocarbonat von Alkyl oder Phenyl mit einem Derivat oder einem Stereoisomer eines solchen Derivates kondensiert, ausgewählt unter den folgenden Formeln: 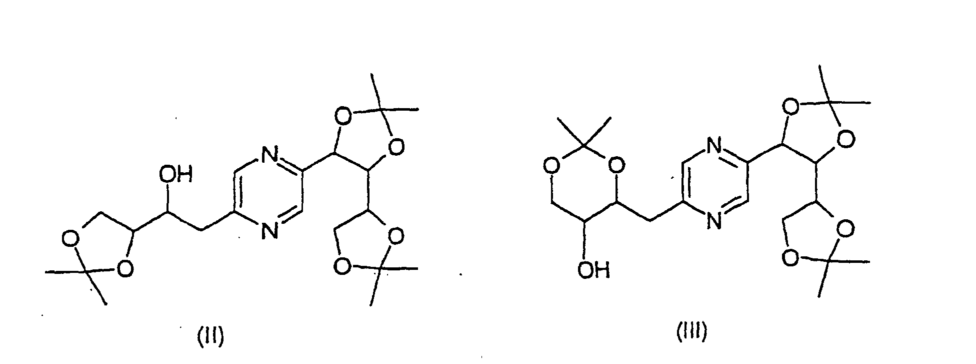 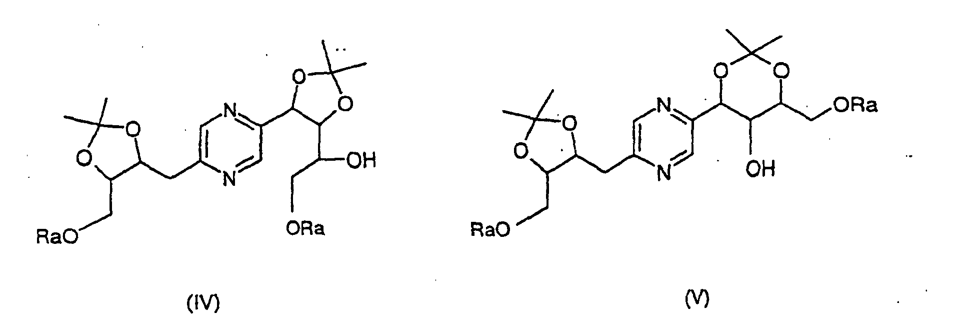 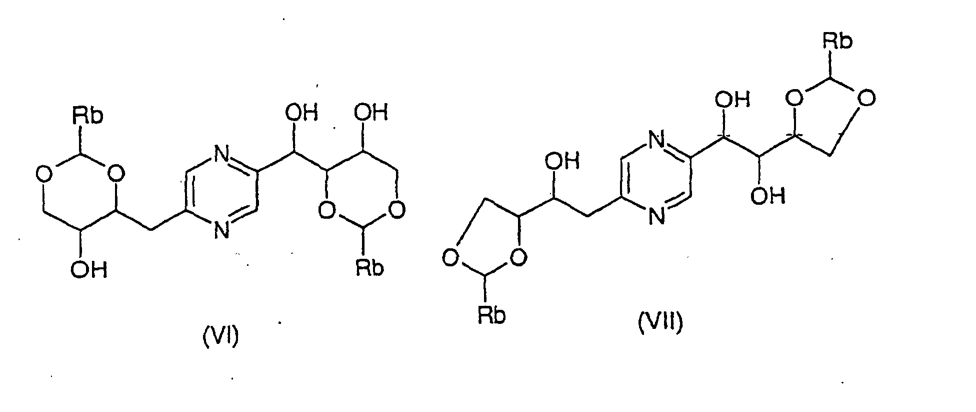 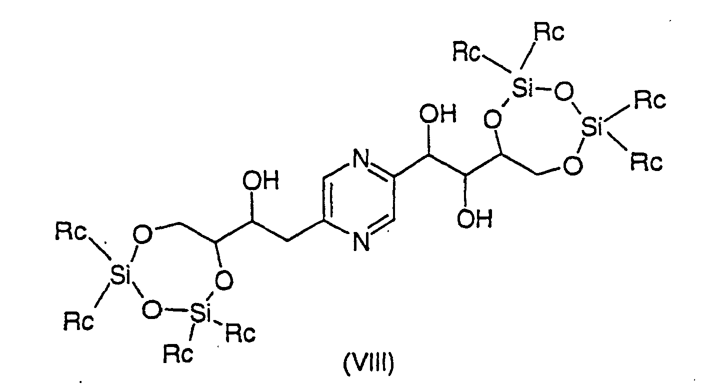 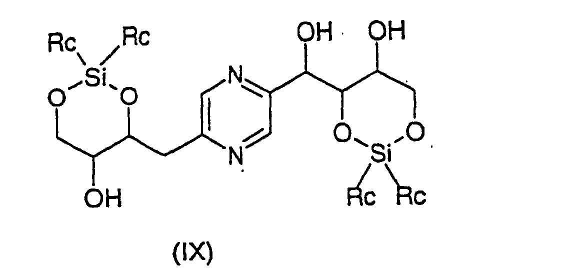 in denen Ra einen Rest Trialkylsilyl, Alkyldiphenylsilyl oder Dialkylphenylsilyl darstellt, Rb einen Rest Phenyl bedeutet, gegebenenfalls substituiert durch mindestens einen Rest Alkoxy, und Rc einen Rest Alkyl oder Phenyl darstellt, wobei die Reste und Teile Alkyl und Alkoxy 1 bis 6 Kohlenstoffatome in gerader oder verzweigter Kette enthalten, und man anschließend die Schutzgruppen von den Hydroxylen entfernt, das Produkt isoliert und gegebenenfalls in Salz überführt.

9. Verfahren zur Herstellung der Verbindungen der Formel (I) nach Anspruch 6, worin R₉ und R₁₀ jeweils einen Rest -CH₂OH darstellen, und entweder R₃ einen Rest Methylen bedeutet, R₄ ein Rest -CHOHist und einer der Reste R₁, R₂, R₅ und R₆ einen Rest -CHF- darstellt und die anderen jeweils einen Rest -CHOH- bedeuten, oder R₁ und R₆ jeweils einen Rest -CHOH- darstellen, R₃ ein Rest Methylen ist und R₂, R₄ und R₅ gleich sind und jeweils einen Rest -CHF- bedeuten, oder R₂ und R₅ jeweils einen Rest -CHOH- darstellen, R₃ ein Rest Methylen ist und R₁, R₄ und R₆ gleich sind und jeweils einen Rest -CHF- bedeuten, **dadurch gekennzeichnet, daß** man ein Derivat oder ein Stereoisomer eines solchen Derivates fluoriert, ausgewählt unter den Formeln: 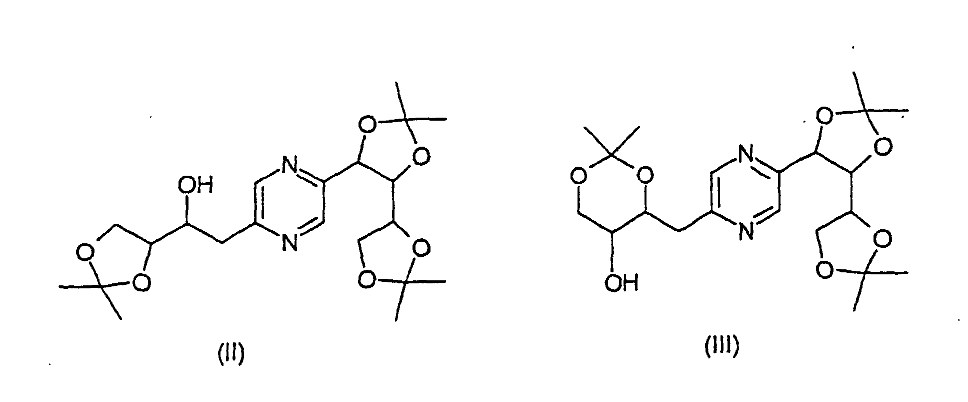 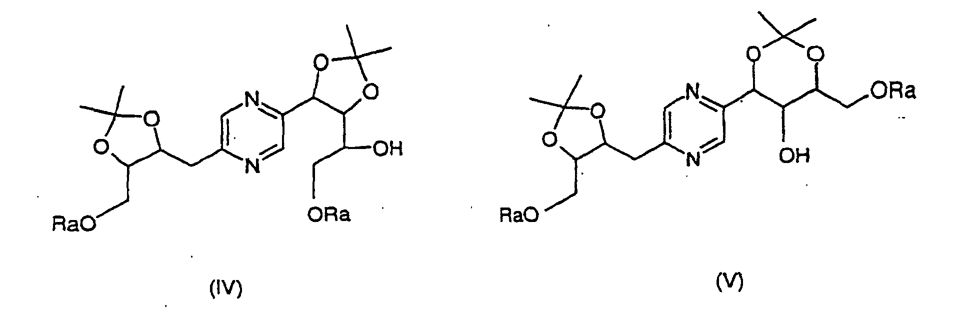 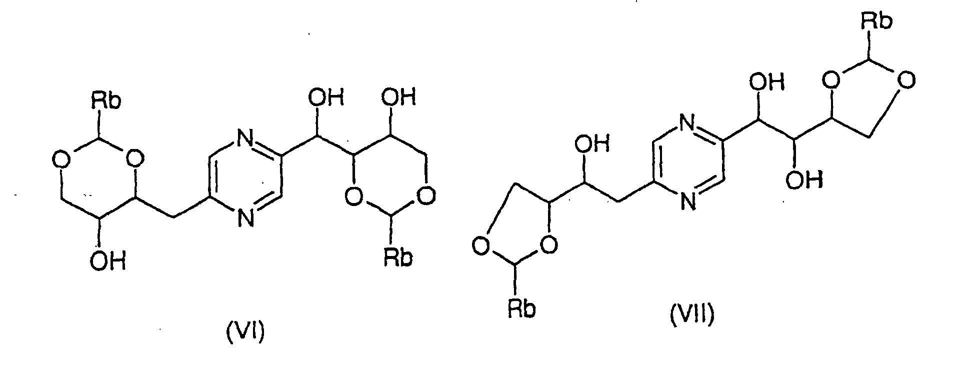 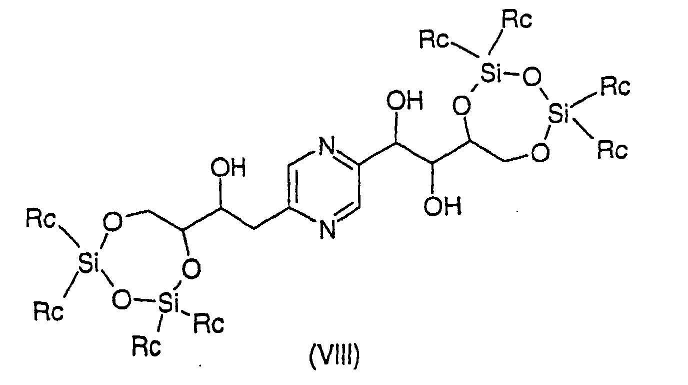 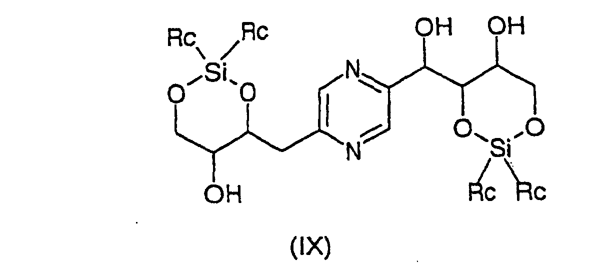 in denen Ra einen Rest Trialkylsilyl, Alkyldiphenylsilyl oder Dialkylphenylsilyl darstellt, Rb einen Rest Phenyl bedeutet, gegebenenfalls substituiert durch mindestens einen Rest Alkoxy, und Rc einen Rest Alkyl oder Phenyl darstellt, wobei die Reste und Teile Alkyl und Alkoxy 1 bis 6 Kohlenstoffatome in gerader oder verzweigter Kette enthalten, und man anschließend die Schutzgruppen von den Hydroxylen entfernt, das Produkt isoliert und gegebenenfalls in Salz überführt.

10. Verfahren zur Herstellung der Verbindungen der Formel (I) nach Anspruch 6, worin R₉ und R₁₀ jeweils einen Rest -CH₂OH darstellen, und entweder R₃ einen Rest Methylen bedeutet, R₄ ein Rest -CHOH- ist und einer der Reste R₁, R₂, R₅ und R₆ einen Rest -CH(NHR₇)- darstellt und die anderen jeweils einen Rest -CHOH- bedeuten, oder R₁ und R₆ jeweils einen Rest -CHOH- darstellen, R₃ ein Rest Methylen ist und R₂, R₄ und R₅ gleich sind und jeweils einen Rest -CH(NHR₇)- bedeuten, oder R₂ und R₅ jeweils einen Rest -CHOHdarstellen, R₃ ein Rest Methylen ist und R₁, R₄ und R₆ gleich sind und jeweils einen Rest -CH(NHR₇)- bedeuten, **dadurch gekennzeichnet, daß** man ein Derivat oder ein Stereoisomer eines solchen Derivates reduziert, ausgewählt unter den Formeln: 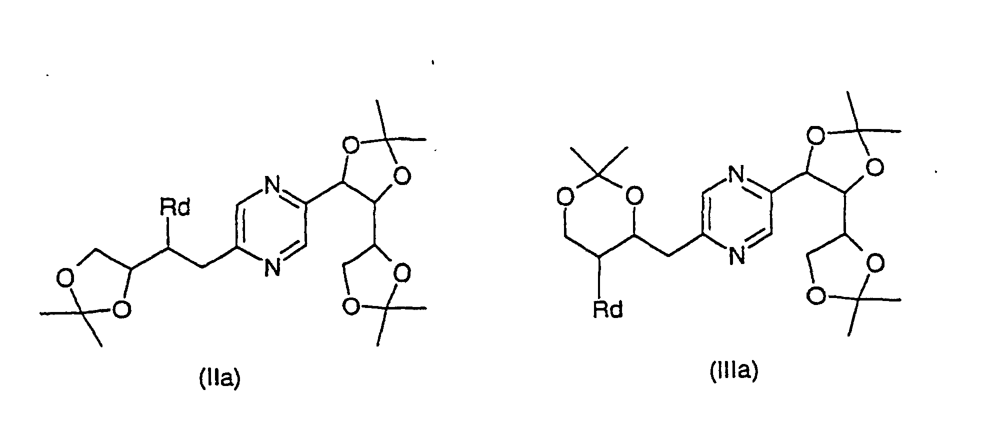 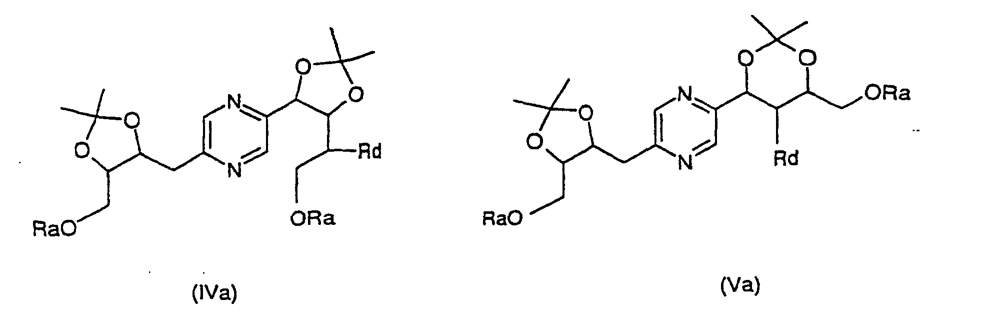 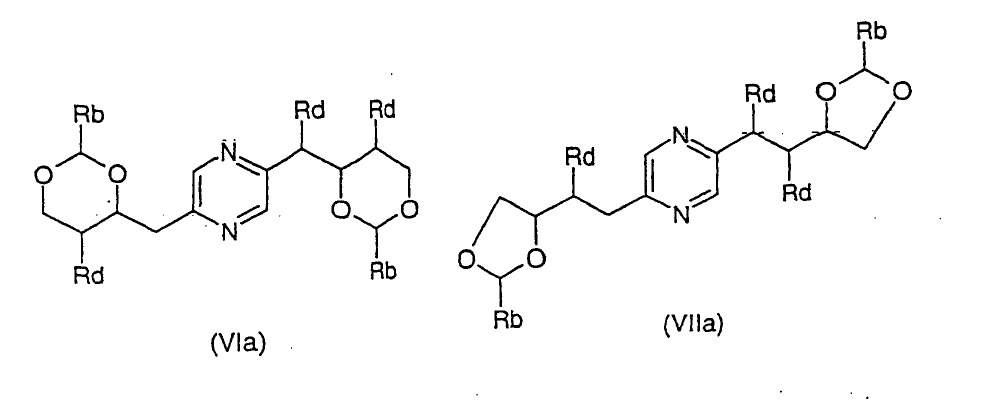 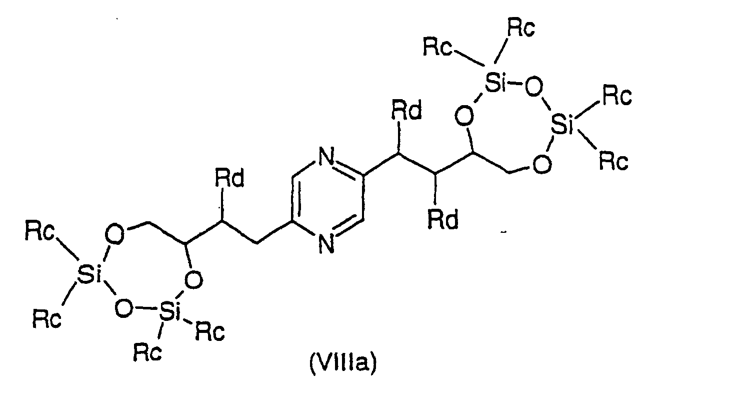 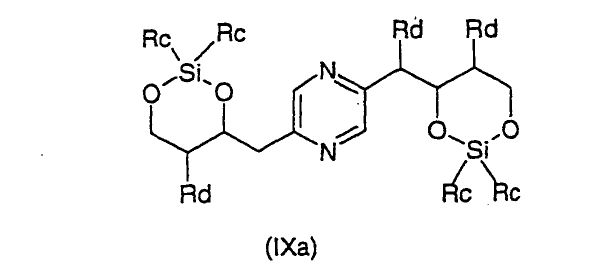 in denen Ra einen Rest Trialkylsilyl, Alkyldiphenylsilyl oder Dialkylphenylsilyl darstellt, Rb einen Rest Phenyl bedeutet, gegebenenfalls substituiert durch mindestens einen Rest Alkoxy, Rc einen Rest Alkyl oder Phenyl darstellt und Rd ein Rest Azido ist, wobei die Reste und Teile Alkyl und Alkoxy 1 bis 6 Kohlenstoffatome in gerader oder verzweigter Kette enthalten, gegebenenfalls gefolgt von der Reaktion eines Derivates der Formel HalR₇, worin R₇ die gleichen Bedeutungen wie in Anspruch 4 besitzt, mit Ausnahme von Wasserstoff, und Hal ein Halogenatom bedeutet, und man anschließend die Schutzgruppen von den Hydroxylen entfernt, das Produkt isoliert und gegebenenfalls in Salz überführt.

11. Verfahren zur Herstellung der Verbindungen der Formel (I) nach Anspruch 6, worin R₉ und R₁₀ jeweils einen Rest -CH₂OH darstellen, und entweder R₃ einen Rest Methylen bedeutet, R₄ ein Rest -CHOH- ist und einer der Reste R₁, R₂, R₅ und R₆ einen Rest -CH(OR₈) darstellt und die anderen jeweils einen Rest -CHOHbedeuten, oder R₁ und R₆ jeweils einen Rest -CHOH- darstellen, R₃ ein Rest Methylen ist und R₂, R₄ und R₅ gleich sind und jeweils einen Rest -CH(OR₈) bedeuten, oder R₂ und R₅ jeweils einen Rest -CHOH- darstellen, R₃ ein Rest Methylen ist und R₁, R₄ und R₆ gleich sind und jeweils einen Rest -CH(OR₈) bedeuten, **dadurch gekennzeichnet, daß** man ein Derivat der Formel: 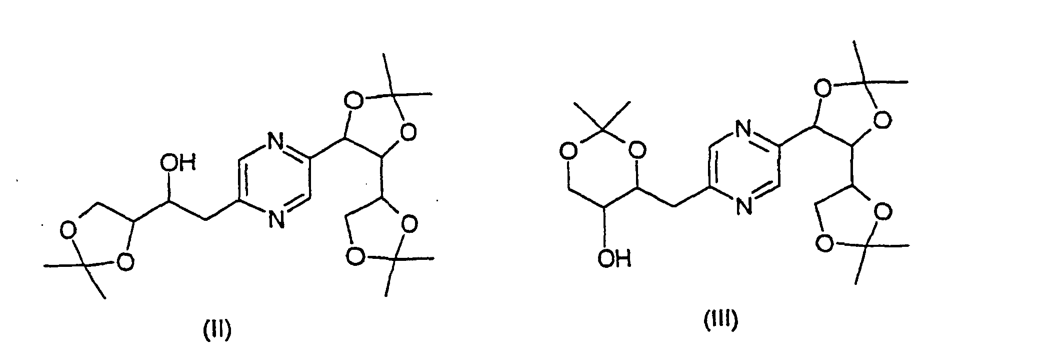 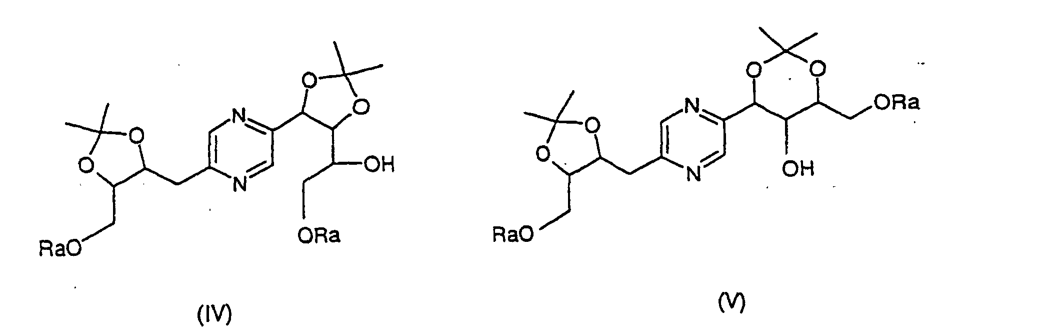 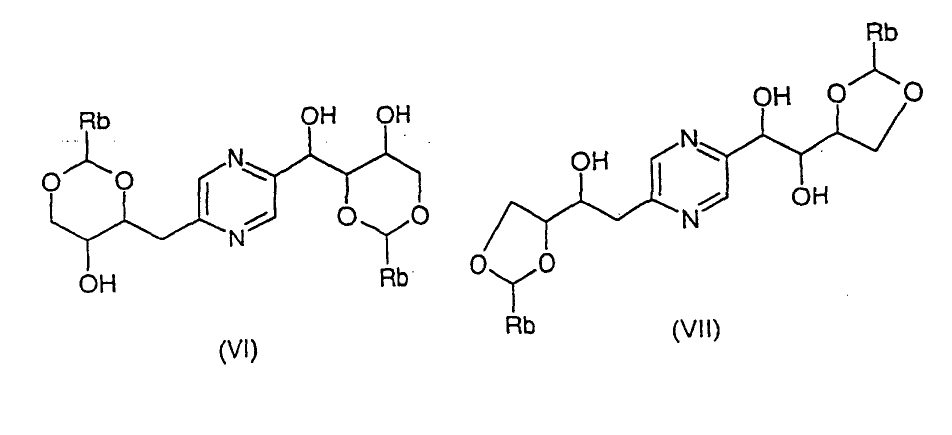 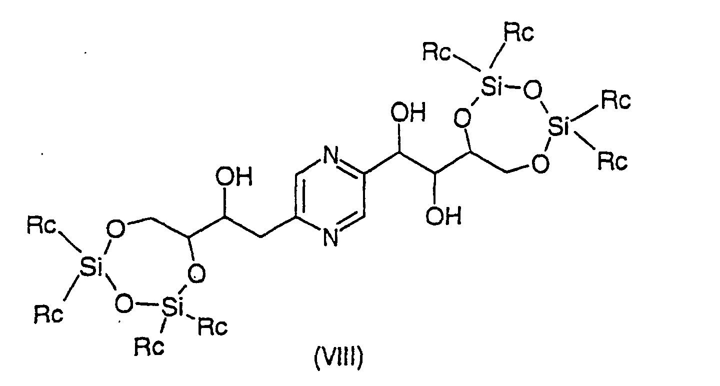 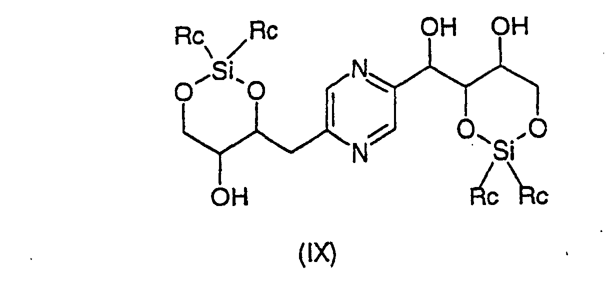 oder ein Stereoisomer eines solchen Derivates,
in denen Ra einen Rest Trialkylsilyl, Alkyldiphenylsilyl oder Dialkylphenylsilyl darstellt, Rb einen Rest Phenyl bedeutet, gegebenenfalls substituiert durch mindestens einen Rest Alkoxy, und Rc einen Rest Alkyl oder Phenyl darstellt, wobei die Reste und Teile Alkyl und Alkoxy 1 bis 6 Kohlenstoffatome in gerader oder verzweigter Kette enthalten, mit einem Derivat HalR₈, worin R₈ die gleichen Bedeutungen wie in Anspruch 4 besitzt, zur Reaktion bringt, und man anschließend die Schutzgruppen von den Hydroxylen entfernt, das Produkt isoliert und gegebenenfalls in Salz überführt.

12. Verfahren zur Herstellung der Verbindungen der Formel (I) nach Anspruch 6, worin R₉ und R₁₀ jeweils einen Rest -CH₂OH darstellen und R₁, R₄, R₅ und R₆ jeweils einen Rest -CHOH- bedeuten, und -R₂-R₃- einen Rest -CH=CH- darstellen, **dadurch gekennzeichnet, daß** man ein Derivat der Formel: 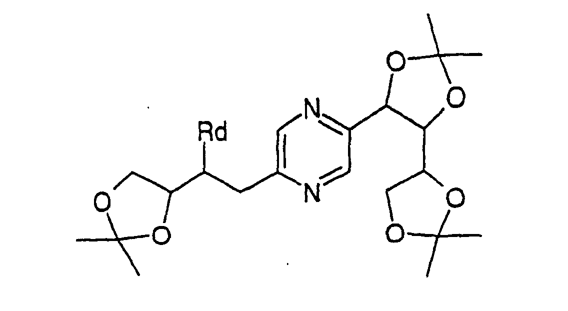 oder ein Stereoisomer eines solchen Derivates, worin Rd einen Rest -OSO₂-Re darstellt und Re einen Rest Methyl, Trifluormethyl oder 4-Methylphenyl bedeutet, dehydratisiert, und man anschließend die Schutzgruppen von den Hydroxylen entfernt, das Produkt isoliert und gegebenenfalls in Salz überführt.

13. Verwendung der Verbindungen der Formel (I) 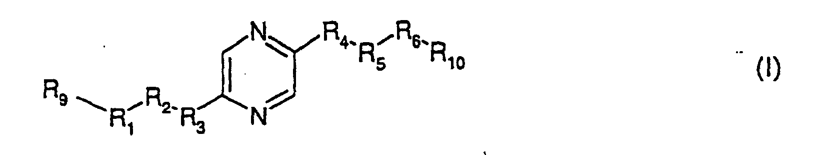 in der
R₉ und R₁₀ gleich sind und einen Rest -CH₂OH darstellen, und entweder
a) R₃ einen Rest Methylen bedeutet, R₄ ein Rest -CHOH- ist und einer der Reste R₁, R₂, R₅ und R₆ einen Rest Carbonyl, Methylen, -CHF-, -CH(NHR₇)- oder -CH(OR₈) darstellt und die anderen jeweils einen Rest -CHOH- bedeuten,
b) R₂ und R₅ jeweils einen Rest -CHOH- darstellen, R₃ ein Rest Methylen ist und R₁, R₄ und R₆ gleich sind und jeweils einen Rest Carbonyl, Methylen, -CHF-, -CH(NHR₇)- oder -CH(OR₈) bedeuten,
c) R₁ und R₆ jeweils einen Rest -CHOH- darstellen, R₃ ein Rest Methylen ist und R₂, R₄ und R₅ gleich sind und jeweils einen Rest Carbonyl, Methylen, -CHF-, -CH(NHR₇)- oder -CH(OR₈) bedeuten,
d) R₁, R₄, R₅ und R₆ jeweils einen Rest -CHOH- darstellen und -R₂-R₃- einen Rest -CH=CH- bedeutet,
R₇ ein Wasserstoffatom oder einen Rest Alkyl, -CO-alk, -CO-Ar oder -CO-Het darstellt,
R₈ einen Rest Alkyl, -alk-COOH oder -alk-OH darstellt,
alk einen Rest Alkyl darstellt,
Ar einen Rest Phenyl oder Phenyl darstellt, substituiert durch einen oder mehrere Substituenten, ausgewählt unter einem Halogenatom, einem Rest Alkyl, Alkoxy, Alkoxycarbonyl, Amino, Monoalkylamino oder Dialkylamino,
Het einen mono-, di- oder tricyclischen, gesättigten oder ungesättigten Heterocyclus mit 1 bis 9 Kohlenstoffatomen und einem oder mehreren Heteroatomen, ausgewählt unter Sauerstoff, Schwefel und Stickstoff, darstellt,
die Reste und Teile Alkyl und Alkoxy 1 bis 6 Kohlenstoffatome in gerader oder verzweigter Kette enthalten,
oder eines ihrer Stereoisomeren und bei der Verbindung, in der -R₂-R₃- einen Rest -CH=CH- bedeutet, ihre Formen cis oder trans, sowie ihre Salze mit einer pharmazeutisch akzeptablen Mineralsäure oder organische Säure zur Herstellung eines Arzneimittels für die Behandlung oder Vorbeugung von Diabetes und Komplikationen von Diabetes.

## Claims

1. Medicaments comprising, as active principle, at least one compound of formula: 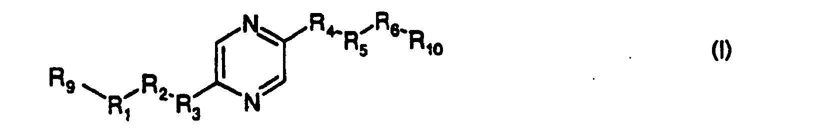 in which
R₉ and R₁₀ are identical and represent a -CH₂OH radical and either
a) R₃ represents a methylene radical, R₄ represents a -CHOH- radical and one of the R₁, R₂, R₅ and R₆ radicals represents a carbonyl, methylene, -CHF-, -CH(NHR₇)- or -CH(OR₈) radical and the others each represent a -CHOH-radical,
b) R₂ and R₅ each represent a -CHOH- radical, R₃ represents a methylene radical and R₁, R₄ and R₆ are identical and each represent a carbonyl, methylene, -CHF-, -CH(NHR₇)- or -CH(OR₈) radical,
c) R₁ and R₆ each represent a -CHOH- radical, R₃ represents a methylene radical and R₂, R₄ and R₅ are identical and each represent a carbonyl, methylene, -CHF-, -CH(NHR₇)- or -CH(OR₈) radical,
d) R₁, R₄, R₅ and R₆ each represent a -CHOH- radical and -R₂-R₃- represents a -CH=CH- radical,
R₇ represents a hydrogen atom or an alkyl, -CO-alk, -CO-Ar or -CO-Het radical,
R₈ represents an alkyl, -alk-COOH or -alk-OH radical, alk represents an alkyl radical,
Ar represents a phenyl radical or a phenyl radical substituted by one or more substituents chosen from a halogen atom or an alkyl, alkoxy, alkoxycarbonyl, amino, monoalkylamino or dialkylamino radical,
Het represents a saturated or unsaturated, mono-, di- or tricyclic heterocycle comprising 1 to 9 carbon atoms and one or more heteroatoms chosen from oxygen, sulphur and nitrogen,
the alkyl and alkoxy radicals and portions comprising 1 to 6 carbon atoms in a straight or branched chain,
or one of its stereoisomers or, for the compound in which -R₂-R₃- represents a -CH=CH- radical, its cis or trans forms, or one of its salts with a pharmaceutically acceptable inorganic or organic acid.

2. Medicaments according to Claim 1, in which, in the compounds of formula (I), Het represents a heterocycle chosen from the 2-, 3- or 4-pyridyl, imidazolyl, thiazolyl and oxazolyl rings.

3. Medicaments according to Claim 1, in which the compound of formula (I) is chosen from the. following compounds:
1-[5-(3R,4-Dihydroxy-2-oxobutyl)pyrazin-2-yl]butane-1R,2S,3R,4-tetraol,
1-[5-(3S,4-Dihydroxybutyl)pyrazin-2-yl]butane-1R,2S,3R,4-tetraol,
1-[5-(2S-Fluoro-3R,4-dihydroxybutyl)pyrazin-2-yl]butane-1R,2S,3R,4-tetraol,
1-[5-(2R-Fluoro-3R,4-dihydroxybutyl)pyrazin-2-yl]butane-1R,2S,3R,4-tetraol,
1-[5-(2S-Amino-3S,4-dihydroxybutyl)pyrazin-2-yl]butane-1R,2S,3R,4-tetraol,
1-[5-(2R-Amino-3S,4-dihydroxybutyl)pyrazin-2-yl]butane-1R,2S,3R,4-tetraol,
1-[5-(2S-(N-Methyl)amino-3S,4-dihydroxybutyl)pyrazin-2-yl]butane-1R,2S,3R,4-tetraol,
1-[5-(2R-(N-Methyl)amino-3S,4-dihydroxybutyl)pyrazin-2-yl]butane-1R,2S,3R,4-tetraol,
1-[5-(2S-(N-Ethyl)amino-3S,4-dihydroxybutyl)pyrazin-2-yl]butane-1R,2S,3R,4-tetraol,
1-[5-(2R-(N-Ethyl)amino-3S,4-dihydroxybutyl)pyrazin-2-yl]butane-1R,2S,3R,4-tetraol,
1-[5-(2S-(N-n-Butyl)amino-3S,4-dihydroxybutyl)pyrazin-2-yl]butane-1R,2S,3R,4-tetraol,
1-[5-(2R-(N-n-Butyl)amino-3S,4-dihydroxybutyl)pyrazin-2-yl]butane-1R,2S,3R,4-tetraol,
1-[5-(2S-(N-Benzyl)amino-3S,4-dihydroxybutyl)pyrazin-2-yl]butane-1R,2S,3R,4-tetraol,
1-[5-(2R-(N-Benzyl)amino-3S,4-dihydroxybutyl)pyrazin-2-yl]butane-1R,2S,3R,4-tetraol,
1-[5-(2S-(N-Acetyl)amino-3S,4-dihydroxybutyl)pyrazin-2-yl]butane-1R,2S,3R,4-tetraol,
1-[5-(2R-(N-Acetyl)amino-3S,4-dihydroxybutyl)pyrazin-2-yl]butane-1R,2S,3R,4-tetraol,
1-[5-(2S-(N-Butanoyl)amino-3S,4-dihydroxybutyl)pyrazin-2-yl]butane-1R,2S,3R,4-tetraol,
1-[5-(2R-(N-Butanoyl)amino-3S,4-dihydroxybutyl)pyrazin-2-yl]butane-1R,2S,3R,4-tetraol,
1-[5-(2S-(N-Benzoyl)amino-3S,4-dihydroxybutyl)pyrazin-2-yl]butane-1R,2S,3R,4-tetraol,
1-[5-(2R-(N-Benzoyl)amino-3S,4-dihydroxybutyl)pyrazin-2-yl]butane-1R,2S,3R,4-tetraol,
1-[5-(2S-Methoxy-3R,4-dihydroxybutyl)pyrazin-2-yl]butane-1R,2S,3R,4-tetraol,
1-[5-(2R-Methoxy-3R,4-dihydroxybutyl)pyrazin-2-yl]butane-1R,2S,3R,4-tetraol,
1-[5-(2S-Ethoxy-3R,4-dihydroxybutyl)pyrazin-2-yl]butane-1R,2S,3R,4-tetraol,
1-[5-(2R-Ethoxy-3R,4-dihydroxybutyl)pyrazin-2-yl]butane-1R,2S,3R,4-tetraol,
1-[5-(2S-n-Butoxy-3S,4-dihydroxybutyl)pyrazin-2-yl]butane-1R,2S,3R,4-tetraol,
1-[5-(2R-n-Butoxy-3R,4-dihydroxybutyl)pyrazin-2-yl]butane-1R,2S,3R,4-tetraol,
1-[5-(2S-(2-Hydroxyethyl)oxy-3R,4-dihydroxybutyl)-pyrazin-2-yl]butane-1R,2S,3R,4-tetraol,
1-[5-(2R-(2-Hydroxyethyl)oxy-3R,4-dihydroxybutyl)-pyrazin-2-yl]butane-1R,2S,3R,4-tetraol,
1-[5-(2S-(3-Hydroxy-n-propyl)oxy-3R,4-dihydroxybutyl)-pyrazin-2-yl]butane-1R,2S,3R,4-tetraol,
1-[5-(2R-(3-Hydroxy-n-propyl)oxy-3R,4-dihydroxybutyl)-pyrazin-2-yl]butane-1R,2S,3R,4-tetraol,
1-[5-(2S-(Carboxymethyl)oxy-3R,4-dihydroxybutyl)-pyrazin-2-yl]butane-1R,2S,3R,4-tetraol,
1-[5-(2R-(Carboxymethyl)oxy-3R,4-dihydroxybutyl)-pyrazin-2-yl]butane-1R,2S,3R,4-tetraol,
1-[5-(2S-(3-Carboxy-n-propyl)oxy-3R,4-dihydroxybutyl)-pyrazin-2-yl]butane-1R,2S,3R,4-tetraol,
1-[5-(2R-(3-Carboxy-n-propyl)oxy-3R,4-dihydroxybutyl)-pyrazin-2-yl]butane-1R,2S,3R,4-tetraol,
1-[5-(2S,4-Dihydroxy-3-oxobutyl)pyrazin-2-yl]butane-1R,2S,3R,4-tetraol,
1-[5-(2S,4-Dihydroxybutyl)pyrazin-2-yl]butane-1R,2S,3R,4-tetraol,
1-[5-(3R-Fluoro-2S,4-dihydroxybutyl)pyrazin-2-yl]-butane-1R,2S,3R,4-tetraol,
1-[5-(3S-Fluoro-2S,4-dihydroxybutyl)pyrazin-2-yl]-butane-1R,2S,3R,4-tetraol,
1-[5-(3R-Amino-2R,4-dihydroxybutyl)pyrazin-2-yl]butane-1R,2S,3R,4-tetraol,
1-[5-(3S-Amino-2R,4-dihydroxybutyl)pyrazin-2-yl]butane-1R,2S,3R,4-tetraol,
1-[5-(3R-(N-Methyl)amino-2R,4-dihydroxybutyl)pyrazin-2-yl]butane-1R,2S,3R,4-tetraol,
1-[5-(3S-(N-Methyl)amino-2R,4-dihydroxybutyl)pyrazin-2-yl]butane-1R,2S,3R,4-tetraol,
1-[5-(3R-(N-Ethyl)amino-2R,4-dihydroxybutyl)pyrazin-2-yl]butane-1R,2S,3R,4-tetraol,
1-[5-(3S-(N-Ethyl)amino-2R,4-dihydroxybutyl)pyrazin-2-yl]butane-1R,2S,3R,4-tetraol,
1-[5-(3R-(N-n-Butyl)amino-2R,4-dihydroxybutyl)pyrazin-2-yl]butane-1R,2S,3R,4-tetraol,
1-[5-(3S-(N-n-Butyl)amino-2R,4-dihydroxybutyl)pyrazin-2-yl]butane-1R,2S,3R,4-tetraol,
1-[5-(3R-(N-Benzyl)amino-2R,4-dihydroxybutyl)pyrazin-2-yl]butane-1R,2S,3R,4-tetraol,
1-[5-(3S-(N-Benzyl)amino-2R,4-dihydroxybutyl)pyrazin-2-yl]butane-1R,2S,3R,4-tetraol,
1-[5-(3R-(N-Acetyl)amino-2R,4-dihydroxybutyl)pyrazin-2-yl]butane-1R,2S,3R,4-tetraol,
1-[5-(3S-(N-Acetyl)amino-2R,4-dihydroxybutyl)pyrazin-2-yl]butane-1R,2S,3R,4-tetraol,
1-[5-(3R-(N-Butanoyl)amino-2R,4-dihydroxybutyl)pyrazin-2-yl]butane-1R,2S,3R,4-tetraol,
1-[5-(3S-(N-Butanoyl)amino-2R,4-dihydroxybutyl)pyrazin-2-yl]butane-1R,2S,3R,4-tetraol,
1-(5-(3R-(N-Benzoyl)amino-2R,4-dihydroxybutyl)pyrazin-2-yl]butane-1R,2S,3R,4-tetraol,
1-[5-(3S-(N-Benzoyl)amino-2R,4-dihydroxybutyl)pyrazin-2-yl]butane-1R,2S,3R,4-tetraol,
1-[5-(3R-Methoxy-2S,4-dihydroxybutyl)pyrazin-2-yl]-butane-1R,2S,3R,4-tetraol,
1-[5-(3S-Methoxy-2S,4-dihydroxybutyl)pyrazin-2-yl]-butane-1R,2S,3R,4-tetraol,
1-[5-(3R-Ethoxy-2S,4-dihydroxybutyl)pyrazin-2-yl]-butane-1R,2S,3R,4-tetraol,
1-[5-(3S-Ethoxy-2S,4-dihydroxybutyl)pyrazin-2-yl]-butane-1R,2S,3R,4-tetraol,
1-[5-(3R-n-Butoxy-2S,4-dihydroxybutyl)pyrazin-2-yl]-butane-1R,2S,3R,4-tetraol,
1-[5-(3S-n-Butoxy-2S,4-dihydroxybutyl)pyrazin-2-yl]-butane-1R,2S,3R,4-tetraol,
1-[5-(3R-(2-Hydroxyethyl)oxy-2S,4-dihydroxybutyl)-pyrazin-2-yl]butane-1R,2S,3R,4-tetraol,
1-[5-(3S-(2-Hydroxyethyl)oxy-2S,4-dihydroxybutyl)-pyrazin-2-yl]butane-1R,2S,3R,4-tetraol,
1-[5-(3R-(3-Hydroxy-n-propyl)oxy-2S,4-dihydroxybutyl)-pyrazin-2-yl]butane-1R,2S,3R,4-tetraol,
1-[5-(3S-(3-Hydroxy-n-propyl)oxy-2S,4-dihydroxybutyl)-pyrazin-2-yl]butane-1R,2S,3R,4-tetraol,
1-[5-(3R-(Carboxymethyl)oxy-2S,4-dihydroxybutyl)-pyrazin-2-yl]butane-1R,2S,3R,4-tetraol,
1-[5-(3S-(Carboxymethyl)oxy-2S,4-dihydroxybutyl)-pyrazin-2-yl]butane-1R,2S,3R,4-tetraol,
1-[5-(3R-(3-Carboxy-n-propyl)oxy-2S,4-dihydroxybutyl)-pyrazin-2-yl]butane-1R,2S,3R,4-tetraol,
1-[5-(3S-(3-Carboxy-n-propyl)oxy-2S,4-dihydroxybutyl)-pyrazin-2-yl]butane-1R,2S,3R,4-tetraol,
4-[5-(2S,3R,4-Trihydroxybutyl)pyrazin-2-yl]-1,3S,4R-trihydroxybutane-2-one,
1-[5-(2S,3R,4-Trihydroxybutyl)pyrazin-2-yl]butane-1R,2R,4-triol,
1-[5-(2S,3R,4-Trihydroxybutyl)pyrazin-2-yl]-3R-fluorobutane-1R,2S,4-triol,
1-[5-(2S,3R,4-Trihydroxybutyl)pyrazin-2-yl]-3S-fluorobutane-1R,2S,4-triol,
1-[5-(2S,3R,4-Trihydroxybutyl)pyrazin-2-yl]-3R-aminobutane-1R,2R,4-triol,
1-[5-(2S,3R,4-Trihydroxybutyl)pyrazin-2-yl]-3S-aminobutane-1R,2R,4-triol,
1-[5-(2S,3R,4-Trihydroxybutyl)pyrazin-2-yl]-3R-(N-methyl)aminobutane-1R,2R,4-triol,
1-[5-(2S,3R,4-Trihydroxybutyl)pyrazin-2-yl]-3S-(N-methyl)aminobutane-1R,2R,4-triol,
1-[5-(2S,3R,4-Trihydroxybutyl)pyrazin-2-yl]-3R-(N-ethyl)aminobutane-1R,2R,4-triol,
1-[5-(2S,3R,4-Trihydroxybutyl)pyrazin-2-yl]-3S-(N-ethyl)aminobutane-1R,2R,4-triol,
1-[5-(2S,3R,4-Trihydroxybutyl)pyrazin-2-yl]-3R-(N-n-butyl)aminobutane-1R,2R,4-triol,
1-[5-(2S,3R,4-Trihydroxybutyl)pyrazin-2-yl]-3S-(N-n-butyl)aminobutane-1R,2R,4-triol,
1-[5-(2S,3R,4-Trihydroxybutyl)pyrazin-2-yl]-3R-(N-benzyl)aminobutane-1R,2R,4-triol,
1-[5-(2S,3R,4-Trihydroxybutyl)pyrazin-2-yl]-3S-(N-benzyl)aminobutane-1R,2R,4-triol,
1-[5-(2S,3R,4-Trihydroxybutyl)pyrazin-2-yl]-3R-(N-acetyl)aminobutane-1R,2R,4-triol,
1-[5-(2S,3R,4-Trihydroxybutyl)pyrazin-2-yl]-3S-(N-acetyl)aminobutane-1R,2R,4-triol,
1-[5-(2S,3R,4-Trihydroxybutyl)pyrazin-2-yl]-3R-(N-butanoyl)aminobutane-1R,2R,4-triol,
1-[5-(2S,3R,4-Trihydroxybutyl)pyrazin-2-yl]-3S-(N-butanoyl)aminobutane-1R,2R,4-triol,
1-[5-(2S,3R,4-Trihydroxybutyl)pyrazin-2-yl]-3R-(N-benzoyl)aminobutane-1R,2R,4-triol,
1-[5-(2S,3R,4-Trihydroxybutyl)pyrazin-2-yl]-3S-(N-benzoyl)aminobutane-1R,2R,4-triol,
1-[5-(2S,3R,4-Trihydroxybutyl)pyrazin-2-yl]-3R-methoxybutane-1R,2S,4-triol,
1-[5-(2S,3R,4-Trihydroxybutyl)pyrazin-2-yl]-3S-methoxybutane-1R,2S,4-triol,
1-[5-(2S,3R,4-Trihydroxybutyl)pyrazin-2-yl]-3R-ethoxybutane-1R,2S,4-triol,
1-[5-(2S,3R,4-Trihydroxybutyl)pyrazin-2-yl]-3S-ethoxybutane-1R,2S,4-triol,
1-[5-(2S,3R,4-Trihydroxybutyl)pyrazin-2-yl]-3R-n-butoxybutane-1R,2S,4-triol,
1-[5-(2S,3R,4-Trihydroxybutyl)pyrazin-2-yl]-3S-n-butoxybutane-1R,2S,4-triol,
1-[5-(2S,3R,4-Trihydroxybutyl)pyrazin-2-yl]-3R-[(2-hydroxyethyl)oxy]butane-1R,2S,4-triol,
1-[5-(2S,3R,4-Trihydroxybutyl)pyrazin-2-yl]-3S-[(2-hydroxyethyl)oxy]butane-1R,2S,4-triol,
1-[5-(2S,3R,4-Trihydroxybutyl)pyrazin-2-yl]-3R-[(3-hydroxy-n-propyl)oxy]butane-1R,2S,4-triol,
1-[5-(2S,3R,4-Trihydroxybutyl)pyrazin-2-yl]-3S-[(3-hydroxy-n-propyl)oxy]butane-1R,2S,4-triol,
1-[5-(2S,3R,4-Trihydroxybutyl)pyrazin-2-yl]-3R-[(carboxymethyl)oxy]butane-1R,2S,4-triol,
1-[5-(2S,3R,4-Trihydroxybutyl)pyrazin-2-yl]-3S-[(carboxymethyl)oxy]butane-1R,2S,4-triol,
1-[5-(2S,3R,4-Trihydroxybutyl)pyrazin-2-yl]-3R-[(3-carboxy-n-propyl)oxy]butane-1R,2S,4-triol,
1-[5-(2S,3R,4-Trihydroxybutyl)pyrazin-2-yl]-3S-[(3-carboxy-n-propyl)oxy]butane-1R,2S,4-triol,
1-[5-(2S,3R,4-Trihydroxybutyl)pyrazin-2-yl]-1R,3R,4-trihydroxybutane-2-one,
1-[5-(2S,3R,4-Trihydroxybutyl)pyrazin-2-yl]butane-1R,3R,4-triol,
1-[5-(2S,3R,4-Trihydroxybutyl)pyrazin-2-yl]-2R-fluorobutane-1R,3R,4-triol,
1-[5-(2S,3R,4-Trihydroxybutyl)pyrazin-2-yl]-2S-fluorobutane-1R,3R,4-triol,
1-[5-(2S,3R,4-Trihydroxybutyl)pyrazin-2-yl]-2R-aminobutane-1S,3S,4-triol,
1-[5-(2S,3R,4-Trihydroxybutyl)pyrazin-2-yl]-2S-aminobutane-1S,3S,4-triol,
1-[5-(2S,3R,4-Trihydroxybutyl)pyrazin-2-yl]-2R-(N-methyl)aminobutane-1S,3S,4-triol,
1-[5-(2S,3R,4-Trihydroxybutyl)pyrazin-2-yl]-2S-(N-methyl)aminobutane-1S,3S,4-triol,
1-[5-(2S,3R,4-Trihydroxybutyl)pyrazin-2-yl]-2R-(N-ethyl)aminobutane-1S,3S,4-triol,
1-[5-(2S,3R,4-Trihydroxybutyl)pyrazin-2-yl]-2S-(N-ethyl)aminobutane-1S,3S,4-triol,
1-[5-(2S,3R,4-Trihydroxybutyl)pyrazin-2-yl]-2R-(N-n-butyl)aminobutane-1S,3S,4-triol,
1-[5-(2S,3R,4-Trihydroxybutyl)pyrazin-2-yl]-2S-(N-n-butyl)aminobutane-1S,3S,4-triol,
1-[5-(2S,3R,4-Trihydroxybutyl)pyrazin-2-yl]-2R-(N-benzyl)aminobutane-1S,3S,4-triol,
1-[5-(2S,3R,4-Trihydroxybutyl)pyrazin-2-yl]-2S-(N-benzyl)aminobutane-1S,3S,4-triol,
1-[5-(2S,3R,4-Trihydroxybutyl)pyrazin-2-yl]-2R-(N-acetyl)aminobutane-1S,3S,4-triol,
1-[5-(2S,3R,4-Trihydroxybutyl)pyrazin-2-yl]-2S-(N-acetyl)aminobutane-1S,3S,4-triol,
1-[5-(2S,3R,4-Trihydroxybutyl)pyrazin-2-yl]-2R-(N-butanoyl)aminobutane-1S,3S,4-triol,
1-[5-(2S,3R,4-Trihydroxybutyl)pyrazin-2-yl]-2S-(N-butanoyl)aminobutane-1S,3S,4-triol,
1-[5-(2S,3R,4-Trihydroxybutyl)pyrazin-2-yl]-2R-(N-benzoyl)aminobutane-1S,3S,4-triol,
1-[5-(2S,3R,4-Trihydroxybutyl)pyrazin-2-yl]-2S-(N-benzoyl)aminobutane-1S,3S,4-triol,
1-[5-(2S,3R,4-Trihydroxybutyl)pyrazin-2-yl]-2R-methoxy-butane-1R,3R,4-triol,
1-[5-(2S,3R,4-Trihydroxybutyl)pyrazin-2-yl]-2S-methoxy-butane-1R,3R,4-triol,
1-[5-(2S,3R,4-Trihydroxybutyl)pyrazin-2-yl]-2R-ethoxy-butane-1R,3R,4-triol,
1-[5-(2S,3R,4-Trihydroxybutyl)pyrazin-2-yl]-2S-ethoxy-butane-1R,3R,4-triol,
1-[5-(2S,3R,4-Trihydroxybutyl)pyrazin-2-yl]-2R-n-butoxybutane-1R,3R,4-triol,
1-[5-(2S,3R,4-Trihydroxybutyl)pyrazin-2-yl]-2S-n-butoxybutane-1R,3R,4-triol,
1-[5-(2S,3R,4-Trihydroxybutyl)pyrazin-2-yl]-2R-[(2-hydroxyethyl)oxy]butane-1R,3R,4-triol,
1-[5-(2S,3R,4-Trihydroxybutyl)pyrazin-2-yl]-2S-[(2-hydroxyethyl)oxy]butane-1R,3R,4-triol,
1-[5-(2S,3R,4-Trihydroxybutyl)pyrazin-2-yl]-2R-[(3-hydroxy-n-propyl)oxy]butane-1R,3R,4-triol,
1-[5-(2S,3R,4-Trihydroxybutyl)pyrazin-2-yl]-2S-[(3-hydroxy-n-propyl)oxy]butane-1R,3R,4-triol,
1-[5-(2S,3R,4-Trihydroxybutyl)pyrazin-2-yl]-2R-[(carboxymethyl)oxy]butane-1R,3R,4-triol,
1-[5-(2S,3R,4-Trihydroxybutyl)pyrazin-2-yl]-2S-[(carboxymethyl)oxy]butane-1R,3R,4-triol,
1-[5-(2S,3R,4-Trihydroxybutyl)pyrazin-2-yl]-2R-[(3-carboxy-n-propyl)oxy]butane-1R,3R,4-triol,
1-[5-(2S,3R,4-Trihydroxybutyl)pyrazin-2-yl]-2S-[(3-carboxy-n-propyl)oxy]butane-1R,3R,4-triol,
1-[5-(2S,4-Dihydroxy-3-oxobutyl)pyrazin-2-yl]-2S,4-dihydroxybutane-1,3-dione,
4-[5-(2S,4-Dihydroxybutyl)pyrazin-2-yl]butane-1,3S-diol,
4-[5-(3R-Fluoro-2S,4-dihydroxybutyl)pyrazin-2-yl]-2R,4R-difluorobutane-1,3S-diol,
4-[5-(3S-Fluoro-2S,4-dihydroxybutyl)pyrazin-2-yl]-2S,4S-difluorobutane-1,3S-diol,
1-[5-(3R-Amino-2S,4-dihydroxybutyl)pyrazin-2-yl]-2R,4R-diaminobutane-1,3S-diol,
1-[5-(3S-Amino-2S,4-dihydroxybutyl)pyrazin-2-yl]-2S,4S-diaminobutane-1,3S-diol,
1-[5-(3R-(N-Methyl)amino-2S,4-dihydroxybutyl)pyrazin-2-yl]-2R,4R-di[(N-methyl)amino]butane-1,3S-diol,
1-[5-(3S-(N-Methyl)amino-2S,4-dihydroxybutyl)pyrazin-2-yl]-2S,4S-di[(N-methyl)amino]butane-1,3S-diol,
1-[5-(3R-(N-Ethyl)amino-2S,4-dihydroxybutyl)pyrazin-2-yl]-2R,4R-di[(N-ethyl)amino]butane-1,3S-diol,
1-[5-(3S-(N-Ethyl)amino-2S,4-dihydroxybutyl)pyrazin-2-yl]-2S,4S-di[(N-ethyl)amino]butane-1,3S-diol,
1-[5-(3R-(N-n-Butyl)amino-2S,4-dihydroxybutyl)pyrazin-2-yl]-2R,4R-di[(N-n-butyl)amino]butane-1,3S-diol,
1-[5-(3S-(N-n-Butyl)amino-2S,4-dihydroxybutyl)pyrazin-2-yl]-2S,4S-di[(N-n-butyl)amino]butane-1,3S-diol,
1-[5-(3R-(N-Benzyl)amino-2S,4-dihydroxybutyl)pyrazin-2-yl]-2R,4R-di[(N-benzyl)amino]butane-1,3S-diol,
1-[5-(3S-(N-Benzyl)amino-2S,4-dihydroxybutyl)pyrazin-2-yl]-2S,4S-di[(N-benzyl)amino]butane-1,3S-diol,
1-[5-(3R-(N-Acetyl)amino-2S,4-dihydroxybutyl)pyrazin-2-yl]-2R,4R-di[(N-acetyl)amino]butane-1,3S-diol,
1-[5-(3S-(N-Acetyl)amino-2S,4-dihydroxybutyl)pyrazin-2-yl]-2S,4S-di[(N-acetyl)amino]butane-1,3S-diol,
1-[5-(3R-(N-Butanoyl)amino-2S,4-dihydroxybutyl)pyrazin-2-yl]-2R,4R-di[(N-butanoyl)amino]butane-1,3S-diol,
1-[5-(3S-(N-Butanoyl)amino-2S,4-dihydroxybutyl)pyrazin-2-yl]-2S,4S-di[(N-butanoyl)amino]butane-1,3S-diol,
1-[5-(3R-(N-Benzoyl)amino-2S,4-dihydroxybutyl)pyrazin-2-yl]-2R,4R-di[(N-benzoyl]amino]butane-1,3S-diol,
1-[5-(3S-(N-Benzoyl)amino-2S,4-dihydroxybutyl)pyrazin-2-yl]-2S,4S-di[(N-benzoyl)amino]butane-1,3S-diol,
1-[5-(3R-Methoxy-2S,4-dihydroxybutyl)pyrazin-2-yl]-2R,4R-dimethoxybutane-1,3S-diol,
1-[5-(3S-Methoxy-2S,4-dihydroxybutyl)pyrazin-2-yl]-2S,4S-dimethoxybutane-1,3S-diol,
1-[5-(3R-Ethoxy-2S,4-dihydroxybutyl)pyrazin-2-yl]-2R,4R-diethoxybutane-1,3S-diol,
1-[5-(3S-Ethoxy-2S,4-dihydroxybutyl)pyrazin-2-yl]-2S,4S-diethoxybutane-1,3S-diol,
1-[5-(3R-n-Butoxy-2S,4-dihydroxybutyl)pyrazin-2-yl]-2R,4R-di-n-butoxybutane-1,3S-diol,
1-[5-(3S-n-Butoxy-2S,4-dihydroxybutyl)pyrazin-2-yl]-2S,4S-di-n-butoxybutane-1,3S-diol,
1-[5-(3R-(2-Hydroxyethyl)oxy-2S,4-dihydroxybutyl)-pyrazin-2-yl]-2R,4R-di[(2-hydroxyethyl)oxy]butane-1,3S-diol,
1-[5-(3S-(2-Hydroxyethyl)oxy-2S,4-dihydroxybutyl)-pyrazin-2-yl]-2S,4S-di[(2-hydroxyethyl)oxy]butane-1,3S-diol,
1-[5-(3R-(3-Hydroxy-n-propyl)oxy-2S,4-dihydroxybutyl)-pyrazin-2-yl]-2R,4R-di[(3-hydroxy-n-propyl)oxy]butane-1,3S-diol,
1-[5-(3S-(3-Hydroxy-n-propyl)oxy-2S,4-dihydroxybutyl)-pyrazin-2-yl]-2S,4S-di[(3-hydroxy-n-propyl)oxy]butane-1,3S-diol,
1-[5-(3R-(Carboxymethyl)oxy-2S,4-dihydroxybutyl)-pyrazin-2-yl]-2R,4R-di[(carboxymethyl)oxy]butane-1,3S-diol,
1-[5-(3S-(Carboxymethyl)oxy-2S,4-dihydroxybutyl)-pyrazin-2-yl]-2S,4S-di[(carboxymethyl)oxy]butane-1,3S-diol,
1-[5-(3R-(3-Carboxy-n-propyl)oxy-2S,4-dihydroxybutyl)-pyrazin-2-yl]-2R,4R-di[(3-carboxy-n-propyl)oxy]butane-1,35-diol,
1-[5-(3S-(3-Carboxy-n-propyl)oxy-2S,4-dihydroxybutyl)-pyrazin-2-yl]-2S,4S-di[(3-carboxy-n-propyl)oxy]butane-1,3S-diol,
1-[5-(3R,4-Dihydroxy-2-oxobutyl)pyrazin-2-yl]-3R,4-dihydroxybutane-1,2-dione,
4-[5-(2S,4-Dihydroxybutyl)pyrazin-2-yl]butane-1,3S-diol,
4-[5-(3R-Fluoro-2S,4-dihydroxybutyl)pyrazin-2-yl]-2R,4R-difluorobutane-1,3S-diol,
4-[5-(3S-Fluoro-2S,4-dihydroxybutyl)pyrazin-2-yl]-2S,4S-difluorobutane-1,3S-diol,
4-[5-(3R-Amino-2S,4-dihydroxybutyl)pyrazin-2-yl]-2R,4R-diaminobutane-1,3S-diol,
4-[5- (3S-Amino-2S,4-dihydroxybutyl)pyrazin-2-yl]-2S,4S-diaminobutane-1,3S-diol,
4-[5-(3R-(N-Methyl)amino-2S,4-dihydroxybutyl)pyrazin-2-yl]-2R,4R-di[(N-methyl)amino]butane-1,3S-diol,
4-[5-(3S-(N-Methyl)amino-2S,4-dihydroxybutyl)pyrazin-2-yl]-2S,4S-di[(N-methyl)amino]butane-1,3S-diol,
4-[5-(3R-(N-Ethyl)amino-2S,4-dihydroxybutyl)pyrazin-2-yl]-2R,4R-di[(N-ethyl)amino]butane-1,3S-diol,
4-[5-(3S-(N-Ethyl)amino-2S,4-dihydroxybutyl)pyrazin-2-yl]-2S,4S-di[(N-ethyl)amino]butane-1,3S-diol,
4-[5-(3R-(N-n-Butyl)amino-2S,4-dihydroxybutyl)pyrazin-2-yl]-2R,4R-di[(N-n-butyl)amino]butane-1,3S-diol,
4-[5-(3S-(N-n-Butyl)amino-2S,4-dihydroxybutyl)pyrazin-2-yl]-2S,4S-di[(N-n-butyl)amino]butane-1,3S-diol,
4-[5-(3R-(N-Benzyl)amino-2S,4-dihydroxybutyl)pyrazin-2-yl]-2R,4R-di[(N-benzyl)amino]butane-1,3S-diol,
4-[5-(3S-(N-Benzyl)amino-2S,4-dihydroxybutyl)pyrazin-2-yl]-2S,4S-di[(N-benzyl)amino]butane-1,3S-diol,
4-[5-(3R-(N-Acetyl)amino-2S,4-dihydroxybutyl)pyrazin-2-yl]-2R,4R-di[(N-acetyl)amino]butane-1,3S-diol,
4-[5-(3S-(N-Acetyl)amino-2S,4-dihydroxybutyl)pyrazin-2-yl]-2S,4S-di[(N-acetyl)amino]butane-1,3S-diol,
4-[5-(3R-(N-Butanoyl)amino-2S,4-dihydroxybutyl)pyrazin-2-yl]-2R,4R-di[(N-butanoyl)amino]butane-1,3S-diol,
4-[5-(3S-(N-Butanoyl)amino-2S,4-dihydroxybutyl)pyrazin-2-yl]-2S,4S-di[(N-butanoyl)amino]butane-1,3S-diol,
4-[5-(3R-(N-Benzoyl)amino-2S,4-dihydroxybutyl)pyrazin-2-yl]-2R,4R-di[(N-benzoyl)amino]butane-1,3S-diol,
4-[5-(3S-(N-Benzoyl)amino-2S,4-dihydroxybutyl)pyrazin-2-yl]-2S,4S-di[(N-benzoyl)amino]butane-1,3S-diol,
4-[5-(3R-Methoxy-2S,4-dihydroxybutyl)pyrazin-2-yl]-2R,4R-dimethoxybutane-1,3S-diol,
4-[5-(3S-Methoxy-2S,4-dihydroxybutyl)pyrazin-2-yl]-2S,4S-dimethoxybutane-1,3S-diol,
4-[5-(3R-Ethoxy-2S,4-dihydroxybutyl)pyrazin-2-yl]-2R,4R-diethoxybutane-1,3S-diol,
4-[5-(3S-Ethoxy-2S,4-dihydroxybutyl)pyrazin-2-yl]-2S,4S-diethoxybutane-1,3S-diol,
4-[5-(3R-n-Butoxy-2S,4-dihydroxybutyl)pyrazin-2-yl]-2R,4R-di-n-butoxybutane-1,3S-diol,
4-[5-(3S-n-Butoxy-2S,4-dihydroxybutyl)pyrazin-2-yl]-2S,4S-di-n-butoxybutane-1,3S-diol,
4-[5-(3R-(2-Hydroxyethyl)oxy-2S,4-dihydroxybutyl)-pyrazin-2-yl]-2R,4R-di[(2-hydroxyethyl)oxy]butane-1,3S-diol,
4-[5-(3S-(2-Hydroxyethyl)oxy-2S,4-dihydroxybutyl)-pyrazin-2-yl]-2S,4S-di[(2-hydroxyethyl)oxy]butane-1,3S-diol,
4-[5-(3R-(3-Hydroxy-n-propyl)oxy-2S,4-dihydroxybutyl)-pyrazin-2-yl]-2R,4R-di[(3-hydroxy-n-propyl)oxy]butane-1,3S-diol,
4-[5-(3S-(3-Hydroxy-n-propyl)oxy-2S,4-dihydroxybutyl)-pyrazin-2-yl]-2S,4S-di[(3-hydroxy-n-propyl)oxy]butane-1,3S-diol,
4-[5-(3R-(Carboxymethyl)oxy-2S,4-dihydroxybutyl)-pyrazin-2-yl]-2R,4R-di[(carboxymethyl)oxy]butane-1,3S-diol,
4-[5-(3S-(Carboxymethyl)oxy-2S,4-dihydroxybutyl)-pyrazin-2-yl]-2S,4S-di[(carboxymethyl)oxy]butane-1,3S-diol,
4-[5-(3R-(3-Carboxy-n-propyl)oxy-2S,4-dihydroxybutyl)-pyrazin-2-yl]-2R,4R-di[(3-carboxy-n-propyl)oxy]butane-1,33-diol,
4-[5-(3S-(3-Carboxy-n-propyl)oxy-2S,4-dihydroxybutyl)-pyrazin-2-yl]-2S,4S-di[(3-carboxy-n-propyl)oxy]butane-1,3S-diol,
1-[5-(3S,4-Dihydroxy-1E-butenyl)pyrazin-2-yl]butane-1R,2S,3R,4-tetraol
and their salts with a pharmaceutically acceptable inorganic or organic acid.

4. Medicaments comprising, as active principle, at least one compound of formula (I) according to Claim 1 in which medicaments
R₉ and R₁₀ each represent a -CH₂OH radical, and either
a) R₃ represents a methylene radical, R₄ represents a -CHOH- radical and one of the R₁, R₂, R₅ and R₆ radicals represents a -CHF or -CH(OR₈) radical and the others each represent a -CHOH- radical,
b) R₂ and R₅ each represent a -CHOH- radical, R₃ represents a methylene radical and R₁, R₄ and R₆ are identical and each represent a -CH(OR₈) radical,
c) R₁ and R₆ each represent a -CHOH- radical, R₃ represents a methylene radical and R₂, R₄ and R₅ are identical and each represent a -CH(OR₈) radical,
d) R₁, R₄, R₅ and R₆ each represent a -CHOH- radical and -R₂-R₃- represents a -CH=CH- radical,
R₈ represents an alkyl radical,
their stereoisomers, the cis and trans forms of the compound in which -R₂-R₃- represents a -CH=CH- chain and their salts with a pharmaceutically acceptable inorganic or organic acid.

5. Medicaments comprising, as active principle, at least one compound of formula (I) according to Claim 1 chosen from the following compounds:
1-[5-(3S,4-Dihydroxybutyl)pyrazin-2-yl]butane-1R,2S,3R,4-tetraol,
1-[5-(3S,4-Dihydroxy-1E-butenyl)pyrazin-2-yl]butane-1R,2S,3R,4-tetraol,
1-[5-(2S-Methoxy-3R,4-dihydroxybutyl)pyrazin-2-yl]-butane-1R,2S,3R,4-tetraol,
1-[5-(2R-Fluoro-3R,4-dihydroxybutyl)pyrazin-2-yl]-butane-1R,2S,3R,4-tetraol,
1-[5-(2S,4-Dihydroxy-3R-methoxybutyl)pyrazin-2-yl]-butane-1R,2S,3R,4-tetraol,
4-[5-(3R,4-Dihydroxy-2S-methoxybutyl]]pyrazin-2-yl]-3R,4R-dimethoxybutane-1,2-diol
and their salts with a pharmaceutically acceptable inorganic or organic acid.

6. Compounds of formula: 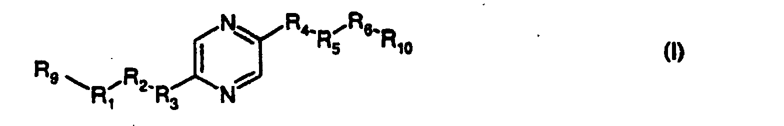 in which
R₉ and R₁₀ each represent a -CH₂OH radical, and either
a) R₃ represents a methylene radical, R₄ represents a -CHOH- radical and one of the R₁, R₂, R₅ and R₆ radicals represents a carbonyl, methylene, -CHF-, -CH(NHR₇)- or -CH(OR₈) radical and the others each represent a -CHOH-radical,
b) R₂ and R₅ each represent a -CHOH- radical, R₃ represents a methylene radical and R₁, R₄ and R₆ are identical and each represent a carbonyl, methylene, -CHF-, -CH(NHR₇)- or -CH(OR₈) radical,
c) R₁ and R₆ each represent a -CHOH- radical, R₃ represents a methylene radical and R₂, R₄ and R₅ are identical and each represent a carbonyl, methylene, -CHF-, -CH(NHR₇)- or -CH(OR₈) radical,
d) R₁, R₄, R₅ and R₆ each represent a -CHOH- radical and -R₂-R₃- represents a -CH=CH- radical,
R₇ represents a hydrogen atom or an alkyl, -CO-alk, -CO-Ar or -CO-Het radical,
R₈ represents an alkyl, -alk-COOH or -alk-OH radical, alk represents an alkyl radical,
Ar represents a phenyl radical or a phenyl radical substituted by one or more substituents chosen from a halogen atom or an alkyl, alkoxy, alkoxycarbonyl, amino, monoalkylamino or dialkylamino radical,
Het represents a saturated or unsaturated, mono-, di- or tricyclic heterocycle comprising 1 to 9 carbon atoms and one or more heteroatoms chosen from oxygen, sulphur and nitrogen,
the alkyl and alkoxy radicals and portions comprising 1 to 6 carbon atoms in a straight or branched chain,
or one of its stereoisomers or, for the compounds in which -R₂-R₃- represents a -CH=CH- radical, their cis or trans forms, or one of its salts, with the exception of the compound of formula:

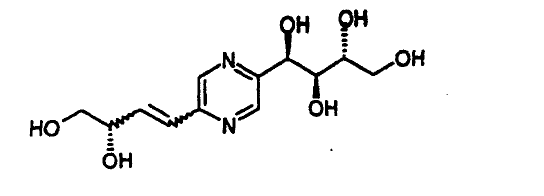

7. Process for the preparation of the compounds of formula (I) according to Claim 6 in which R₉ and R₁₀ each represent a -CH₂OH radical and either R₃ represents a methylene radical, R₄ represents a -CHOH-radical and one of the R₁, R₂, R₅ and R₆ radicals represents a carbonyl radical and the others each represent a -CHOH- radical or R₁ and R₆ each represent a -CHOH- radical, R₃ represents a methylene radical and R₂, R₄ and R₅ are identical and each represent a carbonyl radical or R₂ and R₅ each represent a -CHOH-radical, R₃ represents a methylene radical and R₁, R₄ and R₆ are identical and each represent a carbonyl radical, **characterized in that** a derivative chosen from the formulae: 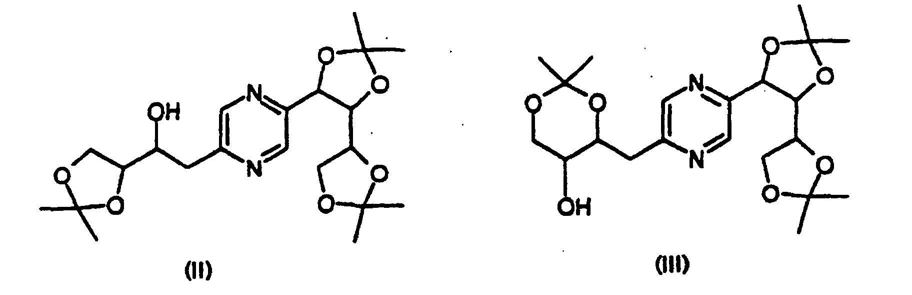 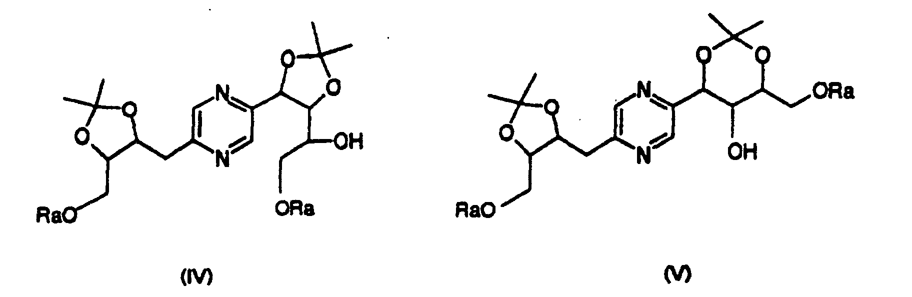 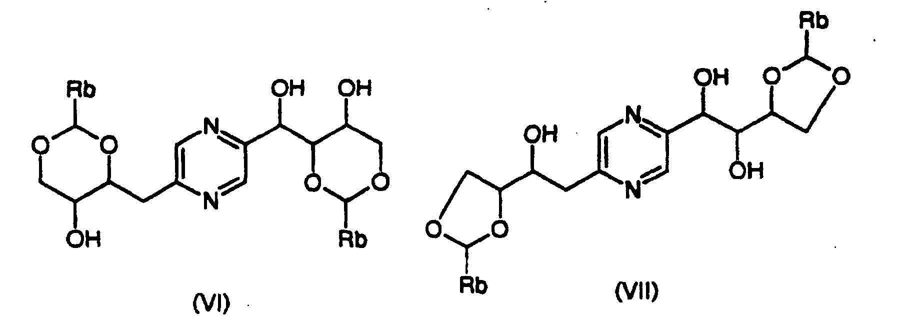 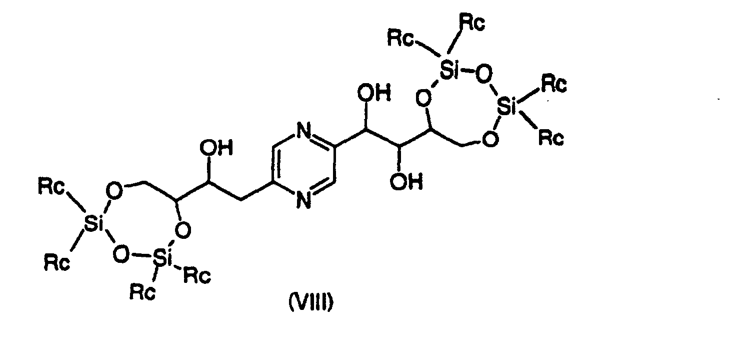 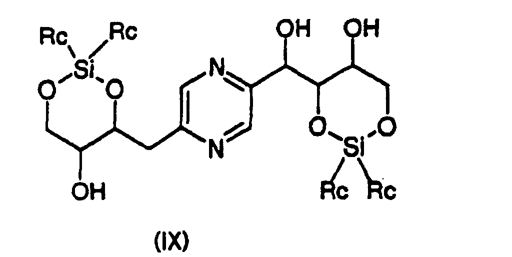 in which Ra represents a trialkylsilyl, alkyldiphenylsilyl or dialkylphenylsilyl radical, Rb represents a phenyl radical optionally substituted by at least one alkoxy radical and Rc represents an alkyl or phenyl radical, the alkyl and alkoxy radicals and portions comprising 1 to 6 carbon atoms in a straight or branched chain, or a stereoisomer of such a derivative is oxidized, the hydroxyls are then deprotected and the product is isolated and optionally converted to a salt.

8. Process for the preparation of the compounds of formula (I) according to Claim 6 in which R₉ and R₁₀ each represent a -CH₂OH radical and either R₃ represents a methylene radical, R₄ represents a -CHOH-radical and one of the R₁, R₂, R₅ and R₆ radicals represents a methylene radical and the others each represent a -CHOH- radical or R₁ and R₆ each represent a -CHOH- radical, R₃ represents a methylene radical and R₂, R₄ and R₅ are identical and each represent a methylene radical or R₂ and R₅ each represent a -CHOH-radical and R₁, R₃, R₄ and R₆ each represent a methylene radical, **characterized in that** an alkyl or phenyl chlorothionocarbonate is condensed with a derivative chosen from the following formulae: 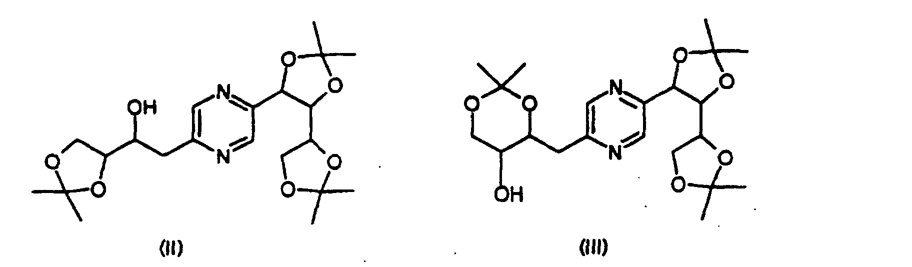 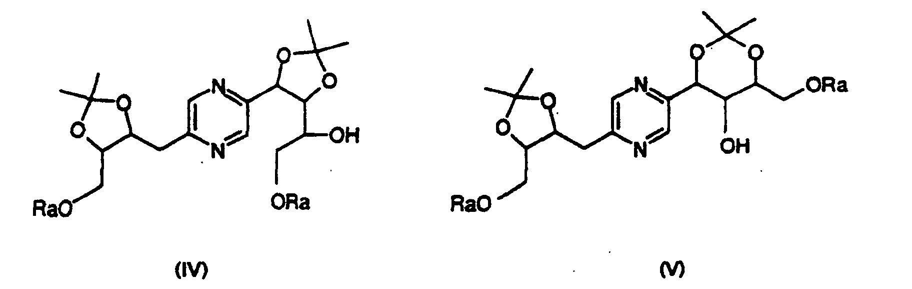 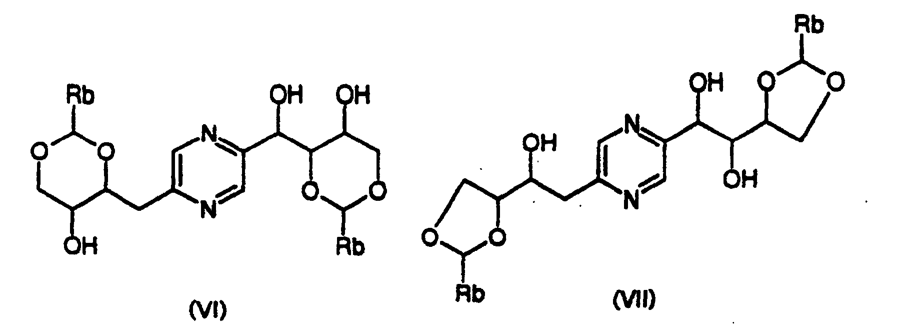 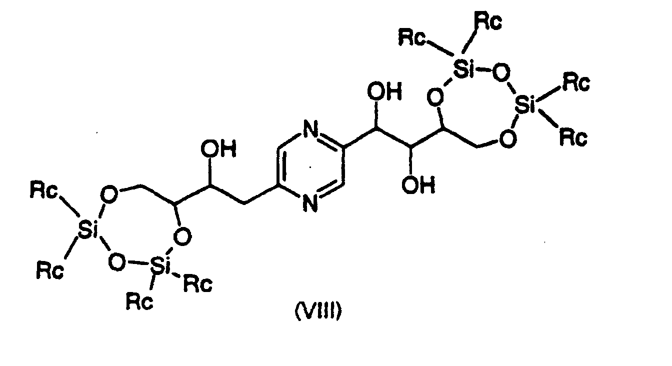 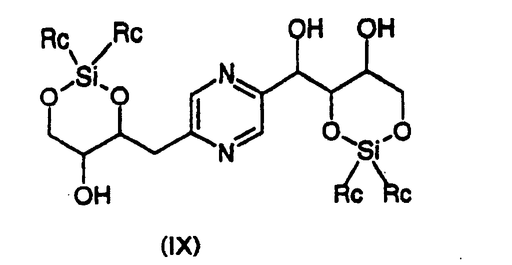 in which Ra represents a trialkylsilyl, alkyldiphenylsilyl or dialkylphenylsilyl radical, Rb represents a phenyl radical optionally substituted by at least one alkoxy radical and Rc represents an alkyl or phenyl radical, the alkyl and alkoxy radicals and portions comprising 1 to 6 carbon atoms in a straight or branched chain, or a stereoisomer of such a derivative, the hydroxyls are then deprotected and the product is isolated and optionally converted to a salt.

9. Process for the preparation of the compounds of formula (I) according to Claim 6 in which R₉ and R₁₀ each represent a -CH₂OH radical and either R₃ represents a methylene radical, R₄ represents a -CHOH-radical and one of the R₁, R₂, R₅ and R₆ radicals represents a -CHF- radical and the others each represent a -CHOH- radical or R₁ and R₆ each represent a -CHOH- radical, R₃ represents a methylene radical and R₂, R₄ and R₅ are identical and each represent a -CHF-radical or R₂ and R₅ each represent a -CHOH- radical, R₃ represents a methylene radical and R₁, R₄ and R₆ are identical and each represent a -CHF- radical, **characterized in that** a derivative chosen from the formulae: 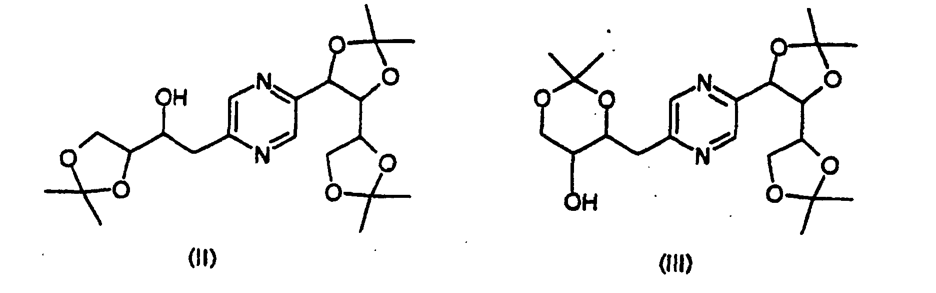 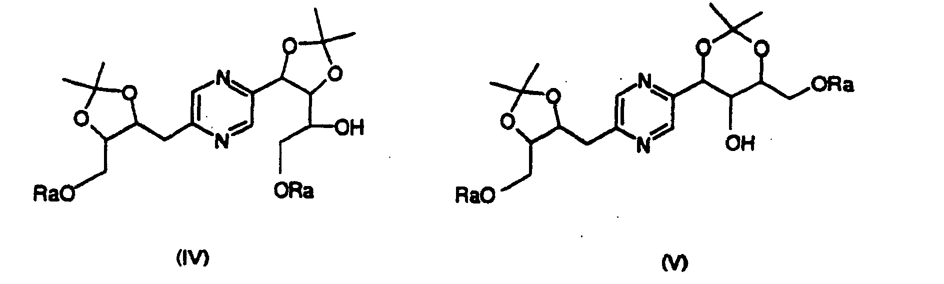 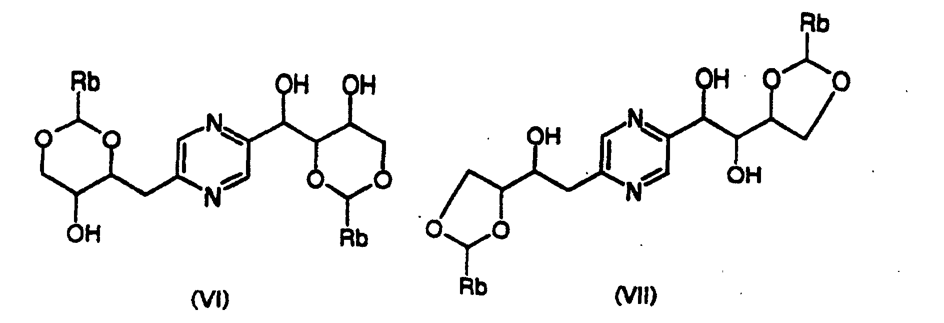 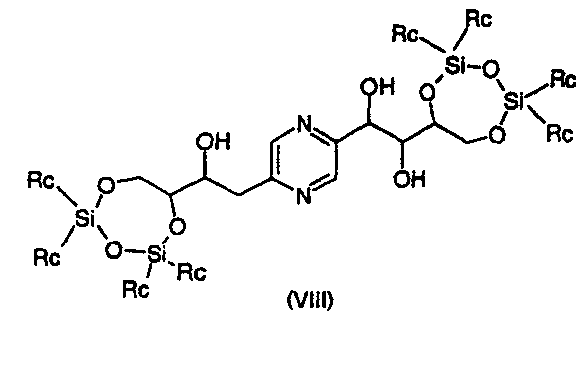 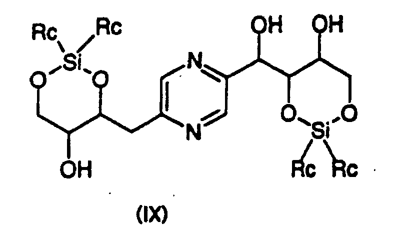 in which Ra represents a trialkylsilyl, alkyldiphenylsilyl or dialkylphenylsilyl radical, Rb represents a phenyl radical optionally substituted by at least one alkoxy radical and Rc represents an alkyl or phenyl radical, the alkyl and alkoxy radicals and portions comprising 1 to 6 carbon atoms in a straight or branched chain, or a stereoisomer of such a derivative is fluorinated, the hydroxyls are then deprotected and the product is isolated and optionally converted to a salt.

10. Process for the preparation of the compounds of formula (I) according to Claim 6 in which R₉ and R₁₀ each represent a -CH₂OH radical and either R₃ represents a methylene radical, R₄ represents a -CHOH-radical and one of the R₁, R₂, R₅ and R₆ radicals represents a -CH(NHR₇)- radical and the others each represent a -CHOH- radical or R₁ and R₆ each represent a -CHOH- radical, R₃ represents a methylene radical and R₂, R₄ and R₅ are identical and each represent a -CH(NHR₇)- radical or R₂ and R₅ each represent a -CHOH-radical, R₃ represents a methylene radical and R₁, R₄ and R₆ are identical and each represent a -CH(NHR₇)-radical, **characterized in that** a derivative chosen from the formulae: 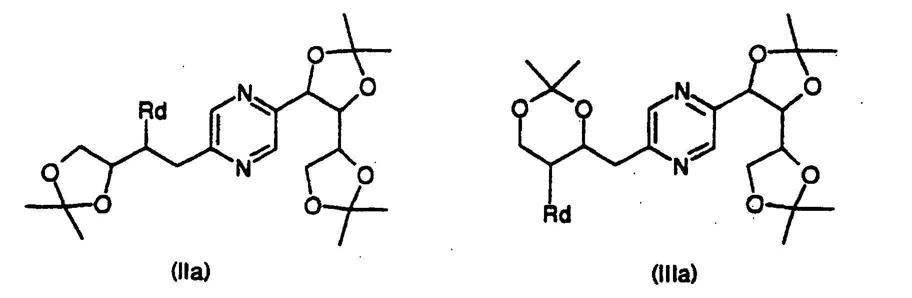 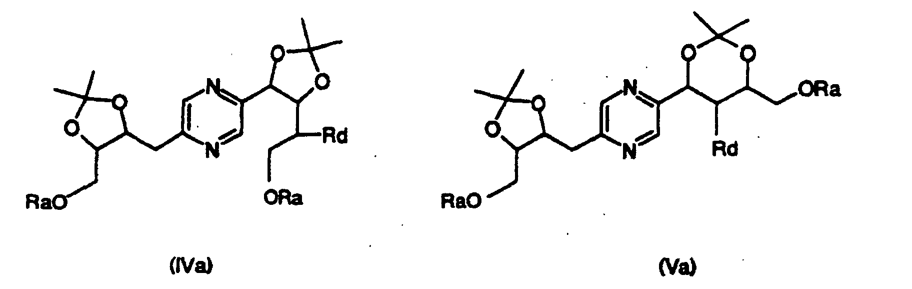 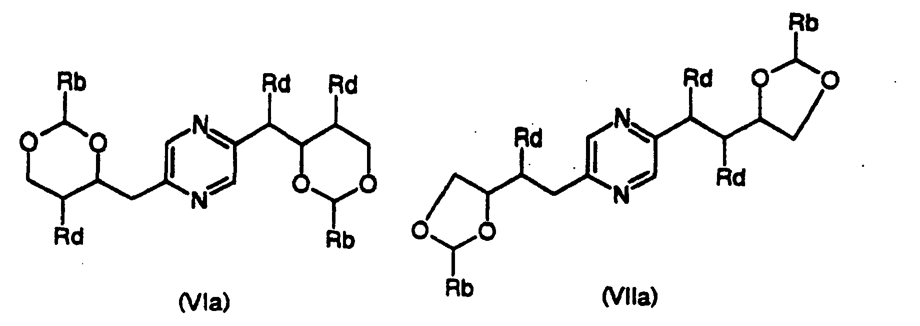 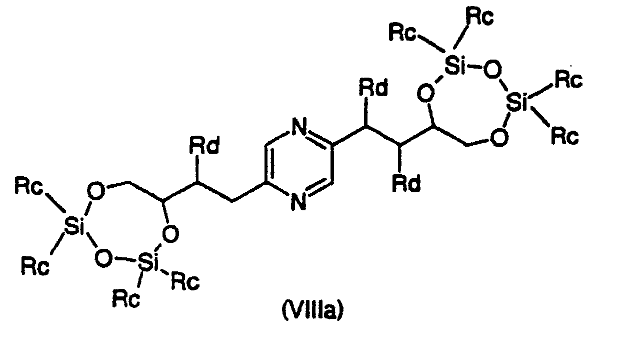 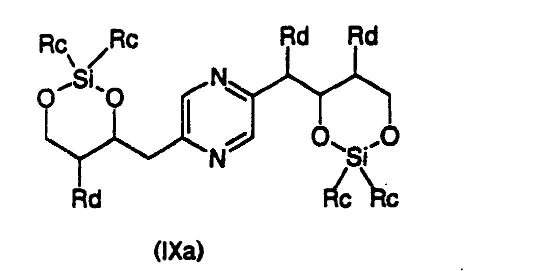 in which Ra represents a trialkylsilyl, alkyldiphenylsilyl or dialkylphenylsilyl radical, Rb represents a phenyl radical optionally substituted by at least one alkoxy radical, Rc represents an alkyl or phenyl radical and Rd represents an azido radical, the alkyl and alkoxy radicals and portions comprising 1 to 6 carbon atoms in a straight or branched chain, or a stereoisomer of such a derivative is reduced, optionally followed by reaction with a derivative of formula HalR₇, in which R₇ has the same meanings as in Claim 4, except hydrogen, Hal represents a halogen atom, the hydroxyls are deprotected and the product is isolated and optionally converted to a salt.

11. Process for the preparation of the compounds of formula (I) according to Claim 6 in which R₉ and R₁₀ each represent a -CH₂OH radical and either R₃ represents a methylene radical, R₄ represents a -CHOH-radical and one of the R₁, R₂, R₅ and R₆ radicals represents a -CH(OR₈) radical and the others each represent a -CHOH- radical or R₁ and R₆ each represent a -CHOH- radical, R₃ represents a methylene radical and R₂, R₄ and R₅ are identical and each represent a -CH(OR₈) radical or R₂ and R₅ each represent a -CHOH- radical, R₃ represents a methylene radical and R₁, R₄ and R₆ are identical and each represent a -CH(OR₈) radical, **characterized in that** a derivative of formula: 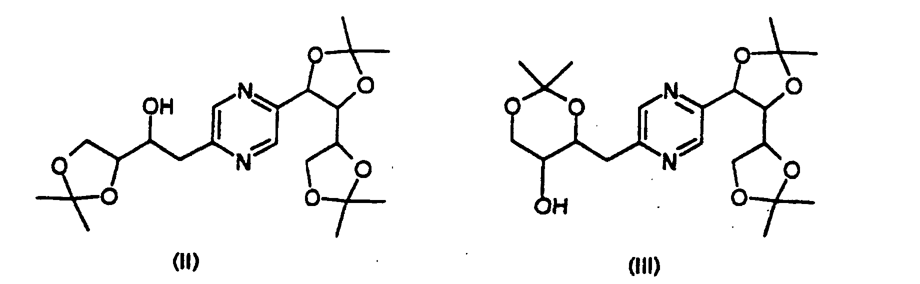 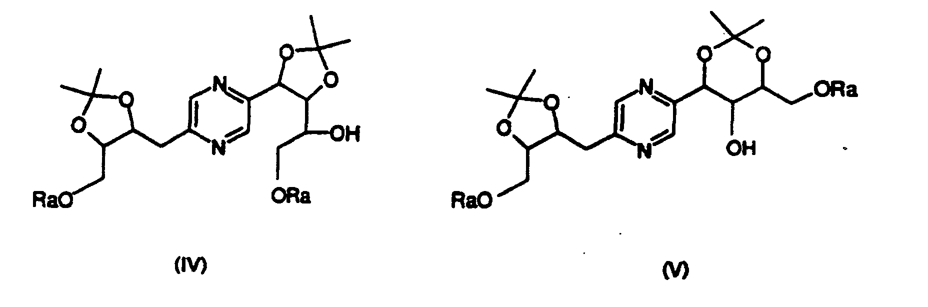 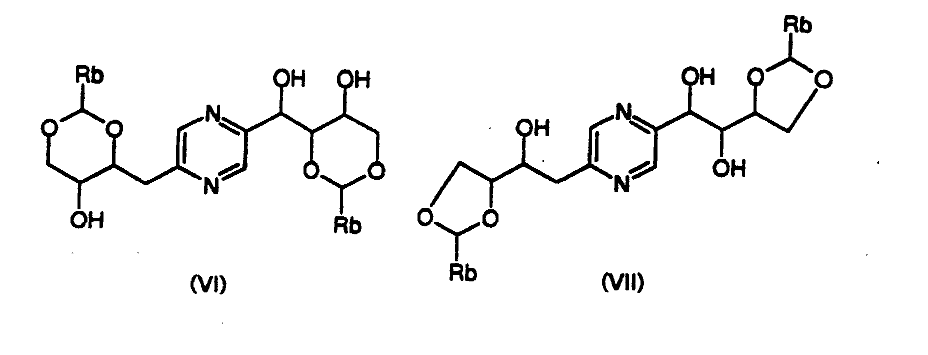 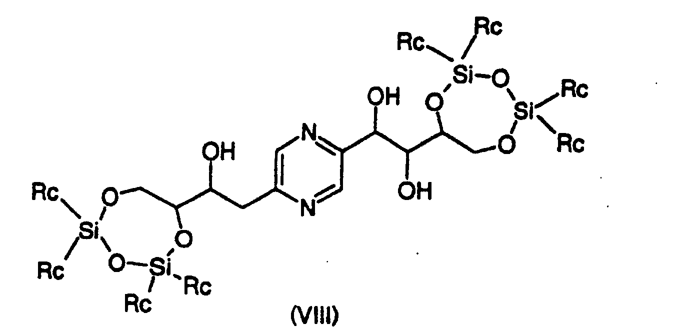 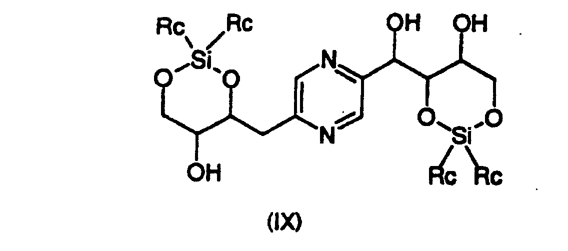 in which Ra represents a trialkylsilyl, alkyldiphenylsilyl or dialkylphenylsilyl radical, Rb represents a phenyl radical optionally substituted by at least one alkoxy radical and Rc represents an alkyl or phenyl radical, the alkyl and alkoxy radicals and portions comprising 1 to 6 carbon atoms in a straight or branched chain, or a stereoisomer of such a derivative is reacted with an HalR₈ derivative, in which R₈ has the same meanings as in Claim 4, the hydroxyls are then deprotected and the product is isolated and optionally converted to a salt.

12. Process for the preparation of the compounds of formula (I) according to Claim 6 in which R₉ and R₁₀ each represent a -CH₂OH radical and R₁, R₄, R₅ and R₆ each represent a -CHOH- radical and -R₂-R₃-represents a -CH=CH- radical, **characterized in that** a derivative of formula: 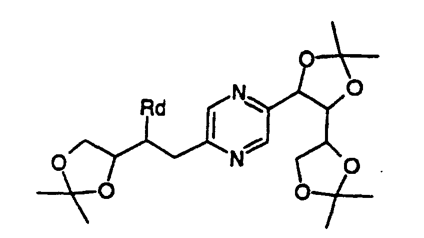 in which Rd represents an -OSO₂-Re radical and Re represents a methyl, trifluoromethyl or 4-methylphenyl radical, or a stereoisomer of such a derivative is dehydrated, the hydroxyls are then deprotected and the product is isolated and optionally converted to a salt.

13. Use of the compounds of general formula: 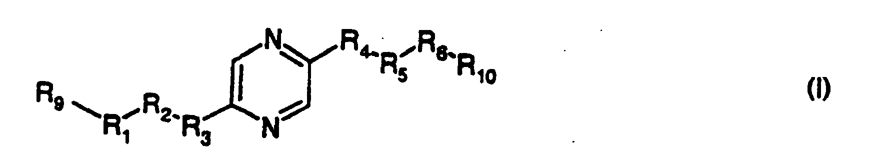 in which
R₉ and R₁₀ are identical and represent a -CH₂OH radical and either
a) R₃ represents a methylene radical, R₄ represents a -CHOH- radical and one of the R₁, R₂, R₅ and R₆ radicals represents a carbonyl, methylene, -CHF-, -CH(NHR₇)- or -CH(OR₈) radical and the others each represent a -CHOH-radical,
b) R₂ and R₅ each represent a -CHOH- radical, R₃ represents a methylene radical and R₁, R₄ and R₆ are identical and each represent a carbonyl, methylene, -CHF-, -CH(NHR₇)- or -CH(OR₈) radical,
c) R₁ and R₆ each represent a -CHOH- radical, R₃ represents a methylene radical and R₂, R₄ and R₅ are identical and each represent a carbonyl, methylene, -CHF-, -CH(NHR₇)- or -CH(OR₈) radical,
d) R₁, R₄, R₅ and R₆ each represent a -CHOH- radical and -R₂-R₃- represents a -CH=CH- radical,
R₇ represents a hydrogen atom or an alkyl, -CO-alk, -CO-Ar or -CO-Het radical,
R₈ represents an alkyl, -alk-COOH or -alk-OH radical, alk represents an alkyl radical,
Ar represents a phenyl radical or a phenyl radical substituted with one or more substituents chosen from a halogen atom or an alkyl, alkoxy, alkoxycarbonyl, amino, monoalkylamino or dialkylamino radical,
Het represents a saturated or unsaturated, mono-, di- or tricyclic heterocycle comprising 1 to 9 carbon atoms and one or more heteroatoms chosen from oxygen, sulphur and nitrogen,
the alkyl and alkoxy radicals and portions comprising 1 to 6 carbon atoms in a straight or branched chain,
or one of its stereoisomers or, for the compound in which -R₂-R₃- represents a -CH=CH- radical, its cis or trans forms, or one of its salts with a pharmaceutically acceptable inorganic or organic acid for the preparation of medicament which is useful in the treatment or prevention of diabetes and complications of diabetes.
